(19) Europäisches Patentamt
European Patent Office
Office européen des brevets

(11) EP 1 976 560 B1

# (12) FASCICULE DE BREVET EUROPEEN

(45) Date de publication et mention de la délivrance du brevet:
**08.01.2014 Bulletin 2014/02**

(21) Numéro de dépôt: **07717813.5**

(22) Date de dépôt: **09.01.2007**

(51) Int Cl.:
***A61K 39/39*** (2006.01) ***A61K 39/245*** (2006.01)
***A61K 39/145*** (2006.01) ***A61K 9/107*** (2006.01)
***A61K 9/00*** (2006.01) ***C07K 14/005*** (2006.01)

(86) Numéro de dépôt international:
**PCT/FR2007/000030**

(87) Numéro de publication internationale:
**WO 2007/080308 (19.07.2007 Gazette 2007/29)**

## (54) EMULSION HUILE DANS EAU THERMOREVERSIBLE

THERMOREVERSIBLE ÖL-IN-WASSER-EMULSION

THERMOREVERSIBLE OIL-IN-WATER EMULSION

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **13.01.2006 FR 0600309**

(43) Date de publication de la demande:
**08.10.2008 Bulletin 2008/41**

(73) Titulaire: **Sanofi Pasteur**
**69367 Lyon Cedex 07 (FR)**

(72) Inventeurs:
- **KLUCKER, Marie-Françoise**
**F-69300 Caluire et Cuire (FR)**
- **HAENSLER, Jean**
**F-69290 Grezieu la Varenne (FR)**
- **PROBECK-QUELLEC, Patricia**
**F-69002 Lyon (FR)**
- **CHAUX, Pascal**
**F-69210 Bully (FR)**

(74) Mandataire: **Hurpin, Christian Marcel**
**Sanofi Pasteur**
**Direction Propriété Intellectuelle**
**2, avenue Pont Pasteur**
**69367 Lyon Cedex 07 (FR)**

(56) Documents cités:
**US-A1- 2004 202 669**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention est relative à une composition immunostimulante sous la forme d'une émulsion Huile dans Eau (H/E) thermoréversible contenant un agoniste du TLR4, dénommé TLA4.

TLR4 (toll-like receptor type 4) est un récepteur exprimé par des cellules présentatrices de l'antigène du système immunitaire ; il intervient dans les mécanismes de défense précoce contre les infections à bactéries gram-. Le lipopolysaccharide des bactéries gram- (LPS) est le ligand naturel du TLR4 ; il active le récepteur, ce qui entraîne une cascade d'évènements biochimiques, en particulier l'activation du facteur de transcription Nf-Kappa B, et la production de cytokines pro-inflammatoires. Le monophosphoryl lipide A provenant de l'hydrolyse du LPS est également un ligand du TLR4 avec l'avantage d'être moins toxique que le LPS.

WO20004/060396 décrit des formulations sous la forme d'émulsions H/E contenant un adjuvant phospholipidique. Les émulsions qui ont une taille sub-micronique sont obtenues au moyen d'un homogénéiseur haute pression (microfluidiseur). Le procédé de fabrication met en oeuvre de hautes énergies mécaniques afin d'obtenir des forces de cisaillement suffisamment importantes pour réduire la taille des gouttes d'huile. Selon cet enseignement, l'émulsion obtenue contient des gouttelettes dont la taille est d'environ 500 nm.

Il est souhaitable de pouvoir disposer d'une formulation alternative à celle proposée dans cette demande de brevet, et surtout qui puisse être obtenue par un procédé plus simple (ne nécessitant pas une technologie de cisaillement particulière), de basse énergie tout en étant reproductible, fiable et utilisable à une large échelle; en outre, la formulation adjuvante doit pouvoir améliorer l'efficacité des vaccins, en augmentant la réponse immune à un antigène, tout en ne présentant pas de signe de toxicité qui nuirait à son administration en toute sécurité.

A cet effet, l'invention a pour objet:

Une émulsion Huile dans eau (H/E) comprenant:

i) un agoniste du TLR4, dénommé TLA4, dont la structure chimique ne comporte pas de noyau sucré,
ii) du squalène,
iii) un solvant aqueux,
iv) un tensioactif hydrophile non ionique qui est un polyoxyethylène alkyl éther,
v) un tensioactif hydrophobe non ionique et,
qui est thermoréversible.

L'agoniste du TLR4 contenu dans l'émulsion, selon l'invention, n'est pas le lipide A, ou un dérivé du lipide A ou une molécule qui mime la structure du lipide A.

Typiquement TLA4 est un composé chimique de formule I, II, III, ou IV :

Composé de formule I

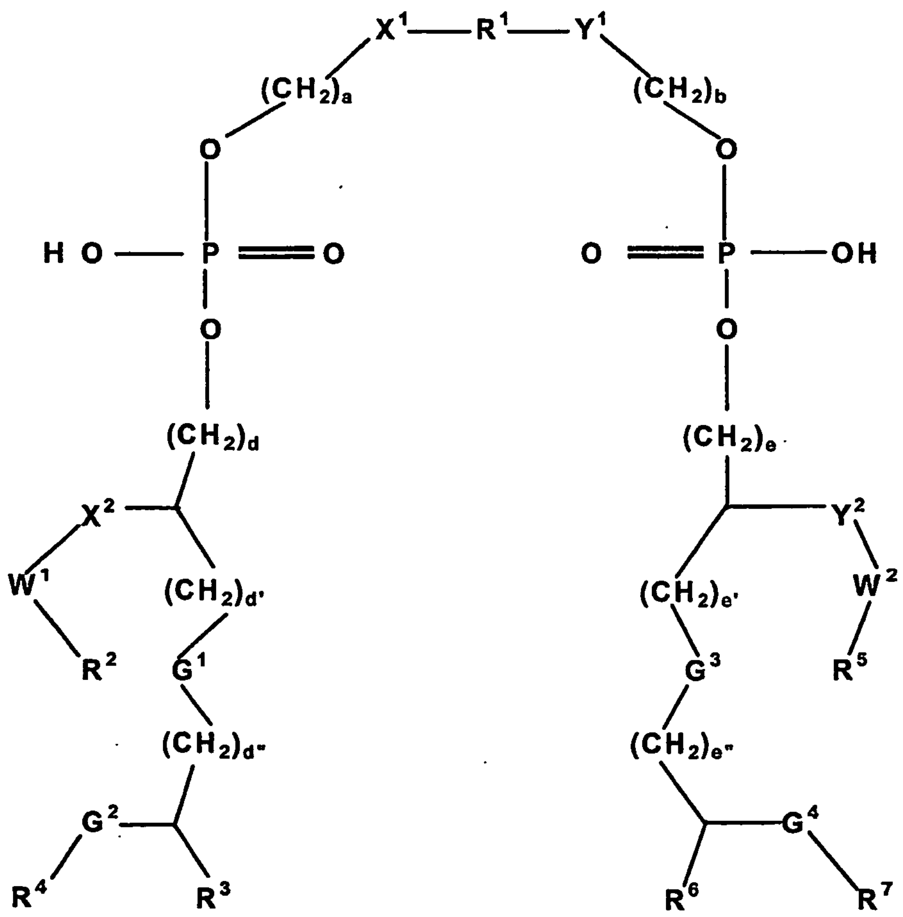

Composé de formule II

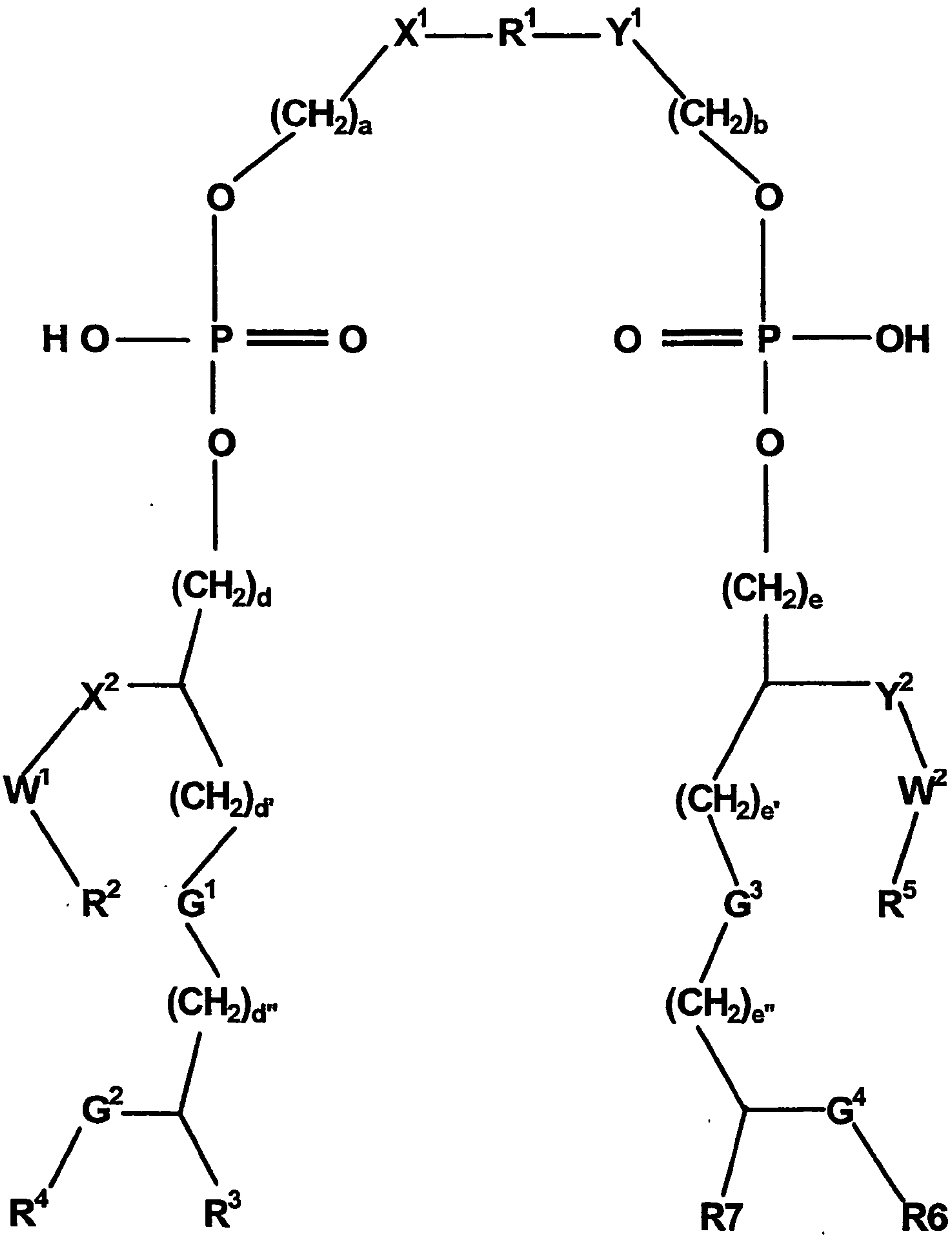

Composé de formule III

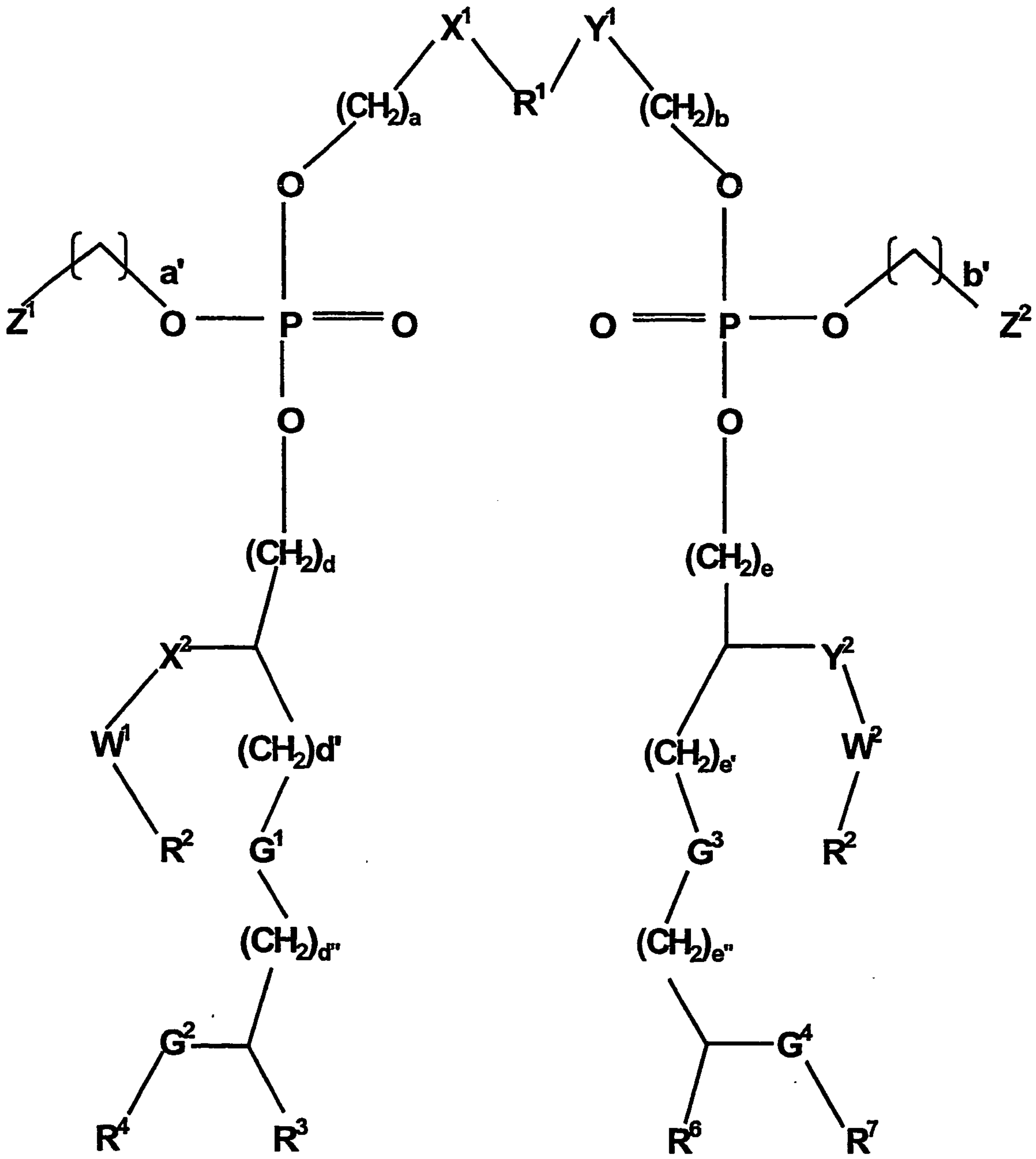

Composé de formule IV

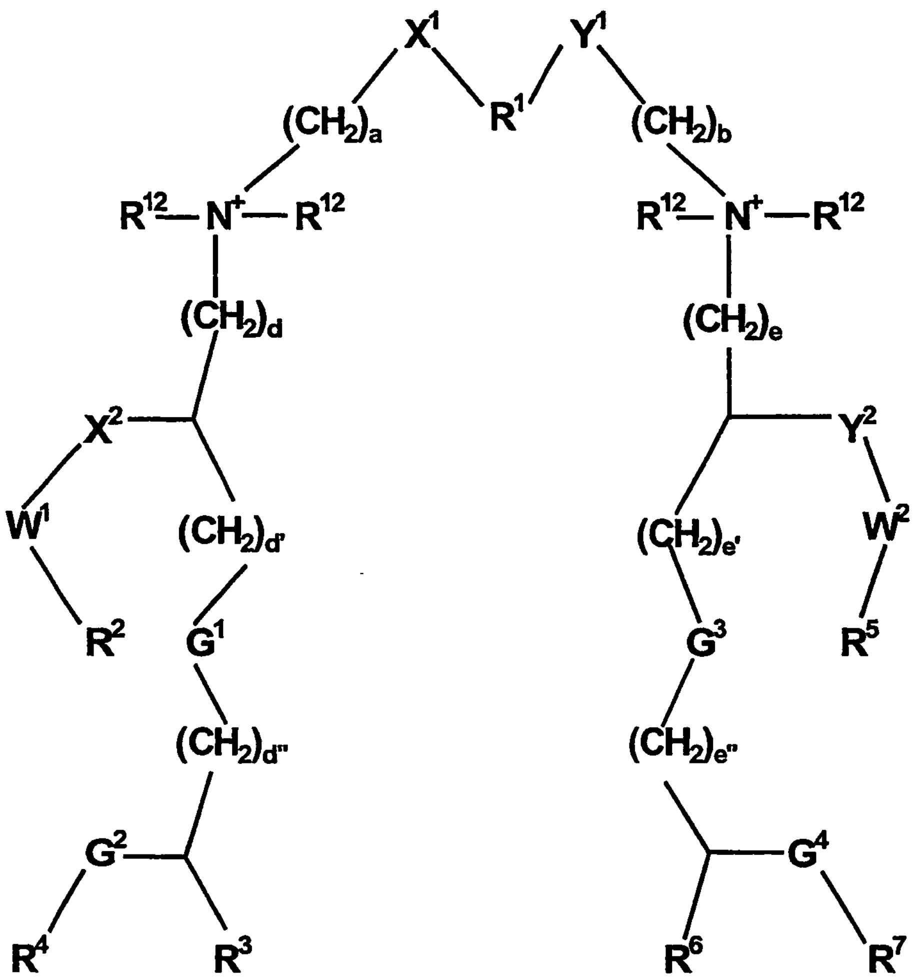

dans laquelle pour chacune des formules I, II, III, ou IV, $R^1$est sélectionné dans le groupe consistant en :

a) C(O);
b) C(O)-alkyle en $C_1$-$C_{14}$-C(O), dans lequel ledit alkyle en $C_1$-$C_{14}$ est facultativement substitué par un hydroxy, un alcoxy en $C_1$-$C_5$, un alkylènedioxy en $C_1$-$C_5$, un (alkyl en $C_1$-$C_5$)amino ou un (alkyle en $C_1$-$C_5$)aryle, dans lequel ladite partie aryle dudit (alkyle en $C_1$-$C_5$)aryle est facultativement substituée par un alcoxy en $C_1$-$C_5$, un (alkyle en $C_1$-$C_5$) amino, un (alcoxy en $C_1$-$C_5$)amino, un (alkyle en $C_1$-$C_5$)aminoalcoxy en $C_1$-$C_5$, -O-(alkyle en $C_1$-$C_5$) aminoalcoxy en $C_1$-$C_5$, -O-(alkyle en $C_1$-$C_5$) amino-C(O)-alkyle en $C_1$-$C_5$-C(O)OH, ou -O-(alkyle en $C_1$-$C_5$)amino-C(O)-alkyle en $C_1$-$C_5$-C(O)-alkyle en $C_1$-$C_5$;
c) un alkyle à chaîne linéaire ou ramifiée en $C_2$-$C_{15}$ facultativement substitué par un hydroxy ou un alcoxy; et
d) -C(O)-arylène en $C_6$-$C_{12}$-C(O)- dans lequel ledit arylène est facultativement substitué par un hydroxy, un halogène, un nitro ou un amino;

a et b sont indépendamment 0, 1, 2, 3 ou 4;

d, d', d", e, e' et e" sont indépendamment 0, 1, 2, 3 ou 4 ;

$X^1$, $X^2$, $Y^1$ et $Y^2$ sont indépendamment sélectionnés dans le groupe consistant en rien, un oxygène, NH et N(C(O)

alkyle en $C_1$-$C_4$), et N(alkyle en $C_1$-$C_4$):

$W^1$ et $W^2$ sont indépendamment sélectionnés dans le groupe consistant en un carbonyle, un méthylène, un sulfone et un sulfoxyde;

$R^2$ et $R^5$ sont indépendamment sélectionnés dans le groupe consistant en :

a) un alkyle à chaîne linéaire ou ramifiée en $C_2$-$C_{20}$ qui est facultativement substitué par un oxo, un hydroxy, ou un alcoxy,
b) un alcényle ou un dialcényle à chaîne linéaire ou ramifiée en $C_2$-$C_{20}$ qui est facultativement substitué par un oxo, un hydroxy, ou un alcoxy;
c) un alcoxy à chaîne linéaire ou ramifiée en $C_2$-$C_{20}$ qui est facultativement substitué par un oxo, un hydroxy, ou un alcoxy;
d) NH-alkyle à chaîne linéaire ou ramifiée en $C_2$-$C_{20}$, dans lequel ledit groupement alkyle est facultativement substitué par un oxo, un hydroxy, ou un alcoxy; et
e)

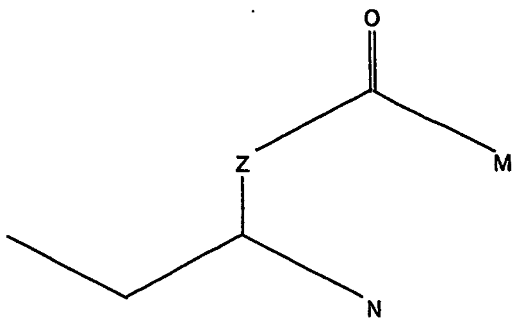

dans laquelle Z est sélectionné dans le groupe consistant en un O et NH, et M et N sont indépendamment sélectionnés dans le groupe consistant en un alkyle, un alcényle, un alcoxy, un acyloxy, un alkylamino et un acylamino à chaîne linéaire ou ramifiée en $C_2$-$C_{20}$;

$R^3$ et $R^6$ sont indépendamment sélectionnés dans le groupe consistant en un alkyle et un alcényle à chaîne linéaire ou ramifiée en $C_2$-$C_{20}$ facultativement substitué par un oxo ou un fluoro;

$R^4$ et $R^7$ sont indépendamment sélectionnés dans le groupe consistant en un C(O)-alkyle ou alcényle à chaîne linéaire ou ramifiée en $C_2$-$C_{20}$, un alkyle à chaîne linéaire ou ramifiée en $C_2$-$C_{20}$, un alcoxy à chaîne linéaire ou ramifiée en $C_2$-$C_{20}$, et un alcényle à chaîne linéaire ou ramifiée en $C_2$-$C_{20}$; dans lequel lesdits groupements alkyle, alcényle, ou alcoxy peuvent être indépendamment et facultativement substitués par un hydroxy, un fluoro ou un alcoxy en $C_1$-$C_5$;

$G_1$, $G_2$, $G_3$ et $G_4$ sont indépendamment sélectionnés dans le groupe consistant en un oxygène, un méthylène, un amino, un thiol, -C(O)NH-, -NHC(O)-, et -N(C(O)alkyle en $C_1$-$C_4$)- ;

ou $G^2R^4$ ou $G^4R^7$ peuvent ensemble être un atome d'hydrogène ou un hydroxyle ;

et dans lequel pour la formule III:

a' et b' sont indépendamment 2, 3, 4, 5, 6, 7 ou 8, de façon préférée 2 ;

$Z^1$ est sélectionné dans le groupe consistant en $-OP(O)(OH)_2$, $-P(O)(OH)_2$, $-OP(O)(OR^8)(OH)$ où $R^8$ est une chaîne alkyke en $C_1$-$C_4$, $-OS(O)_2OH$, $-S(O)_2OH$, $-CO_2H$, $-OB(OH)_2$, -OH, $-CH_3$, $-NH_2$, et $-NR^9_3$ où $R^9$ est une chaîne alkyle en $C_1$-$C_4$;

$Z^2$ est sélectionné dans le groupe consistant en $-OP(O)(OH)_2$, $-P(O)(OH)_2$, $-OP(O)(OR^{10})(OH)$ où $R^{10}$ est une chaîne alkyle en $C_1$-$C_4$, $-OS(O)_2OH$, $-S(O)_2OH$, $-CO_2H$, $-OB(OH)_2$, -OH, $-CH_3$, $-NH_2$, et $-NR^{11}$ où $R^{11}$ est une chaîne alkyle en $C_1$-$C_4$;

et dans lequel pour la formule IV :

R12 est H ou une chaîne alkyle en $C_1$-$C_4$ ;

ou un sel pharmaceutique acceptable du composé de formule I, II, III ou IV.

L'émulsion selon l'invention est thermoréversible, ce qui signifie qu'elle passe de l'état d'émulsion H/E à l'état d'émulsion E/H lorsqu'on la chauffe à une température au moins égale à une « température dite d'inversion de phase ». A l'échelle microscopique, la température d'inversion de phase traduit le passage d'une courbure orientée vers la phase huileuse à une courbure orientée vers la phase aqueuse, cette transition impliquant nécessairement le passage par une phase de courbure moyenne nulle (le système étant alors apparenté soit à une phase lamellaire soit à une microémulsion).

L'émulsion selon l'invention peut être obtenue par un procédé d'inversion de phase par variation de température, ce qui procure un très gros avantage d'un point de vue industriel car il est facilement contrôlable et adapté à des volumes de production importants. Un tel procédé présente les garanties de sécurité et de rentabilité nécessaires à l'industrie pharmaceutique. En outre grâce à ce procédé, il est possible d'obtenir une émulsion monodisperse dont la taille des gouttes est faible, ce qui rend l'émulsion facilement filtrable au moyen de filtres stérilisants dont le seuil de coupure est de 200 nm.

Avantageusement, au moins 90% de la population volumique des gouttelettes d'huile de l'émulsion selon l'invention ont une taille ≤ 200 nm. En général, au moins 50% de la population volumique des gouttelettes d'huile de ces émulsions ont une taille ≤ 110 nm. Selon une caractéristique particulière, au moins 90% de la population volumique des gouttelettes d'huile ont une taille ≤ 180 nm et au moins 50% de la population volumique des gouttelettes ont une taille ≤ 110nm.

Généralement, l'émulsion thermoréversible selon l'invention est homogène. Par émulsion homogène, on entend une émulsion dont la représentation graphique de distribution de taille (granulogramme) des gouttelettes d'huile est unimodale. Typiquement, cette représentation graphique est de type « gaussien ».

La mesure de la taille des gouttelettes peut se faire par différents moyens et notamment par des granulomètres à diffraction LASER, tels que les appareils Beckman Coulter de la gamme LS (notamment le LS230) ou des appareils Malvern de la gamme Mastersizer (notamment le Mastersizer 2000). Le principe de mesure de ces appareils est basé sur l'analyse de l'intensité de la lumière diffusée par les particules en fonction de l'angle (détecteurs de grands, moyens et petits angles) lorsque l'échantillon est éclairé par un faisceau laser. Cette analyse se fait au moyen de modèles mathématiques choisis selon la taille et la nature du matériau utilisé. Dans le cas de la mesure de la taille de particules sub-microniques, il faut appliquer un modèle optique particulier (théorie de Mie) prenant en compte les indices de réfraction de la phase huileuse (ici 1,495 pour le squalène) et de la phase aqueuse (par exemple il est de 1,332 pour l'eau) ; il faut également être capable de détecter les faibles intensités émises par les particules très fines, ce qui nécessite une optimisation de l'analyse avec:

- une cellule supplémentaire de détection pour la mesure aux grands angles de la diffusion différentielle des intensités polarisées (système PIDS chez Coulter qui permet une mesure dès 40 nm).
- un système de détection combinant 2 longueurs d'onde, lumière bleue et rouge chez Malvern. La source de lumière bleue de plus basse longueur d'onde, associée à des détecteurs de diffusion aux larges angles et en rétrodiffusion renforce les performances de l'analyse de la gamme submicronique.

Selon les appareils utilisés, les mesures peuvent légèrement varier en fonction des composants de l'appareil et des logiciels de traitement de données utilisées.

La température d'inversion de phase d'une émulsion thermoréversible selon l'invention est une caractéristique propre à chaque émulsion et varie en fonction de la nature de ses composants et de leurs concentrations relatives. Avantageusement, la composition de l'émulsion selon l'invention est choisie de sorte que l'inversion de phase se produit à une température entre 45°C et 80°C, de façon préférée entre 50 et 65°C. Cette gamme de température est avantageuse car l'émulsion ne risque pas de changer d'état si elle est stockée à une température relativement élevée (≈37°C). De plus, comme dans le procédé de préparation de l'émulsion thermoréversible, le chauffage des composants n'excède pas 80°C, cela contribue au maintien de l'intégrité structurale des composants et en particulier du TLA4. Lorsque la température d'inversion de phase de l'émulsion est élevée, notamment lorsqu'elle est supérieure ou voisine de 80°C, on peut utilement l'abaisser en ajoutant à la composition de l'émulsion un alditol qui est habituellement choisi parmi le sorbitol, le mannitol, le glycérol, le xylitol ou l'érythritol. Lorsque l'alditol est utilisé dans une gamme de concentration allant de 0,1 à 10% (p/p), de préférence dans une gamme de concentration allant de 1 à 10% (p/p) et en particulier dans une gamme de concentration allant de 2 à 7% (p/p), on arrive à abaisser la température d'inversion de phase de l'émulsion d'environ 10°C. On peut également abaisser la température d'inversion de phase de l'émulsion, en remplaçant la phase aqueuse constituée uniquement d'eau par une phase aqueuse saline tamponnée. Habituellement, on utilise un tampon

TRIS, un tampon phosphate comme le PBS, le tampon Dulbecco PBS sans $Ca^{2+}$ ni $Mg^{2+}$ ou un tampon citrate.

Les composés chimiques de formule I, II, III, ou IV sont obtenus par voie de synthèse selon les procédés décrits notamment dans US 2003/0153532 ou dans US 2005/0164988.

En particulier, le TLA4 selon l'invention est un composé chimique de formule I.

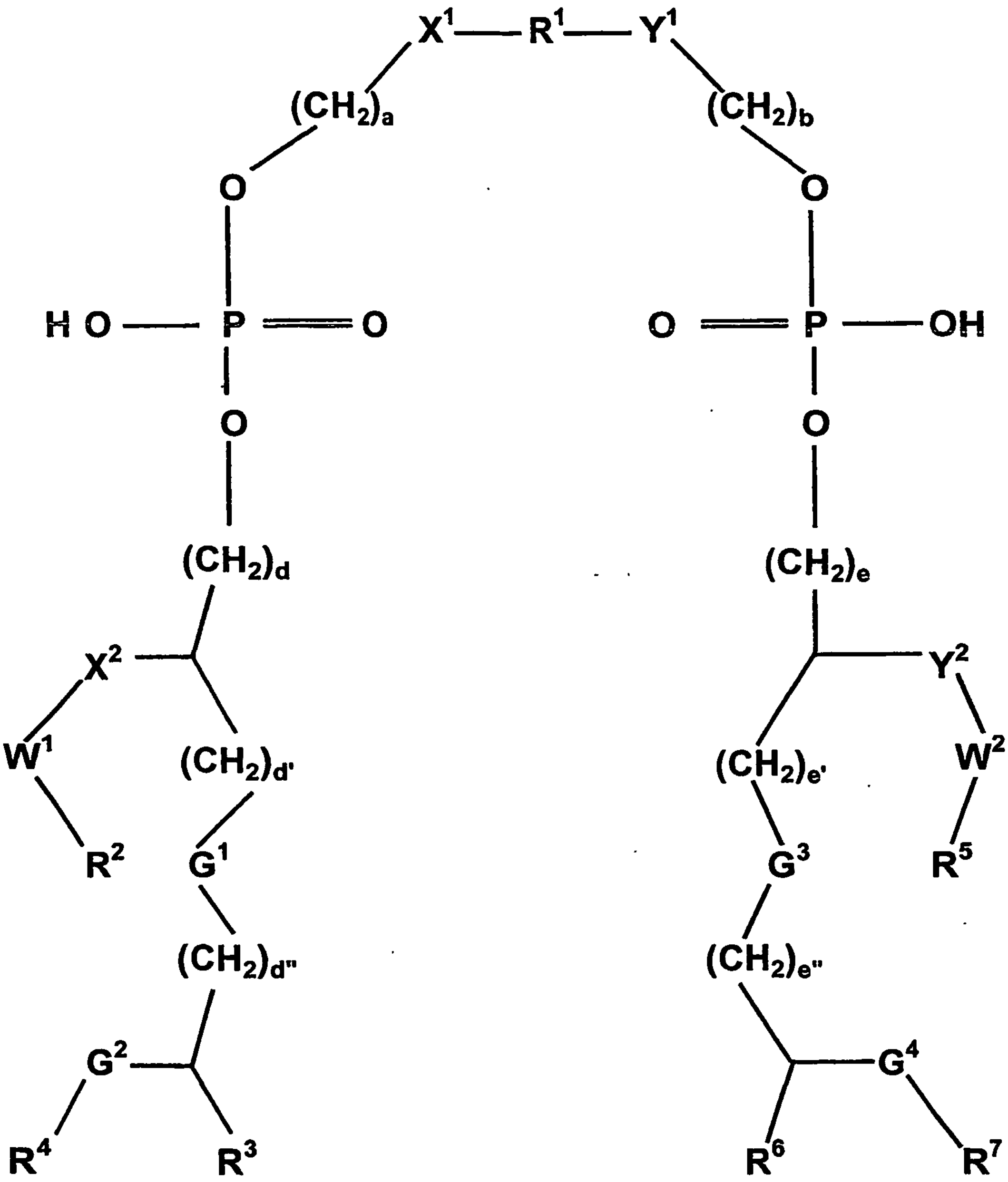

ou un sel pharmaceutiquement acceptable de ce composé.
Préférentiellement,

R1 est C(O) ou C(O)-$(CH2)_n$-C(O), n étant 1, 2, 3 ou 4
a, b, d, d', d", e, e', e" sont indépendamment 1 ou 2,
X1, X2, Y1 et Y2 sont NH,
W1 et W2 sont C(O),
R2 et R5 sont indépendamment sélectionnés dans le groupe consistant en un alkyle à chaîne linéaire en C10-C15 facultativement substitué par un oxo, un NH-alkyle à chaîne linéaire en C10-C15 et,

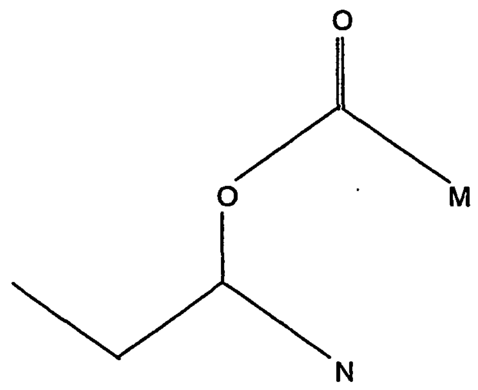

dans lequel M et N sont indépendamment un alkyle ou un alcényle à chaîne linéaire en C2-C20,
R3 et R6 sont des chaînes alkyles linéaires en C5-C10,
R4 et R7 sont sélectionnés dans le groupe consistant en un hydrogène, C(O)-alkyle à chaîne linéaire en C8-C12 et C(O) alcényle à chaîne linéaire en C8-C12,
G1 et G3 sont un oxygène ou -NH(CO)-
G2 et G4 sont un oxygène ;

TLA4 exerce une activité immunostimulante *in vitro* et/ou *in vivo.* L'activité immunostimulante *in vitro* est évaluée notamment :

1) en mesurant l'augmentation de la production de TNFα par les cellules du sang humain total ou,
2) en mesurant l'augmentation de la production de la phosphatase alcaline par une lignée THP-1 transfectée par le gène de la phosphatase alcaline sous la dépendance du promoteur du TNFα ou,
3) en mesurant l'augmentation de la production de cytokines telles que l'IL-10 et l'interféron γ par des splénocytes murins, ou
4) en mesurant l'augmentation de la production du TNFα par la lignée macrophagique murine RAW264 ou,
5) en mesurant l'augmentation de la production de l'IL-6 par l'astrocytome humainU373 ou,
6) en mesurant l'augmentation de l'activation/la maturation de cellules dendritiques dérivées de monocytes humains sur la base de l'expression des marqueurs d'activation tels que CD25, CD80/CD83 par cytométrie de flux.

Tous ces tests de mesure sont bien connus de l'homme de métier et sont notamment décrits dans l'exemple 7 de US 2003/0153532 ou dans Journal of Biological Chemistry, (2001), vol 276/3, page 1873-1880.

L'activité immunostimulante *in vivo* se traduit par une augmentation de la réponse humorale et/ou de la réponse cellulaire spécifique. Pour évaluer la réponse humorale on mesure la production d'anticorps spécifiques dirigés contre un antigène. A titre d'exemple, on peut se référer aux tests qui sont décrits dans l'exemple 8 de US 2003/0153532 pour évaluer cette réponse. Lorsque la production d'anticorps spécifiques (que ce soit sous forme d'immunoglobulines totales ou d'un isotype particulier) observée à la suite de l'injection d'un antigène associé au TLA4 est supérieure à celle que l'on observe consécutivement à l'administration de la même quantité d'antigène seule, TLA4 est considéré comme exerçant une activité immunostimulante *in vivo.* On peut aussi évaluer l'activité immunostimulante du TLA4 en utilisant des tests de mesure de la réponse cellulaire spécifique qui sont bien connus de l'homme du métier, comme par exemple, en mesurant l'activité des lymphocytes T cytotoxiques (CTL) ou la lymphoprolifération.

Préférentiellement, TLA4 est choisit dans le groupe constitué par les composés chimiques identifiés et décrits dans US 2003/0153532 sous les dénominations de ER803022 (sel disodique de l'acide dodécanoïque 1,1'-[(1R,6R, 22R,27R)-1,27-diheptyl-9,19-dihydroxy-9,19-dioxido-14-oxo-6,22 bis [(1-oxotetradecyl)amino]-4,8,10,18,20,24-hexaoxa-13,15-diaza-9,19-diphosphaheptacosane-1,27-diyl] ester), ER803058 (acide dodécanoïque 1,1'-[(1S,6R,24R, 29S)-1,29-diheptyl-9,21-dihydroxy-9,21- dioxido-14,16-dioxo-6,24-bis [(1-oxotetradecyl)amino]-4,8,10,20,22,26-hexaoxa-13,17-diaza-9,21-diphosphanonacosane-1,29-diyl] ester), ER803732 (sel disodique de l'acide dodécanoïque 1,1'-[(1R,6R,22S,27R)-1,27 diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester), ER803789 (acide dodécanoïque 1,1'-[(1R,6R,22S,27R)-1,27-diheptyl9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester), ER804053 (sel disodique de l'acide dodécanoïque 1,1'-[(1R,6S,22S,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester), ER804057 (sel disodique de l'acide dodécanoïque 1,1'-[(1R,6R,22R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester), ER804058 (sel disodique de l'acide 5 dodecenoïque 1,1'-[(1R,6R,24R,29R)-6,24-bis[(1,3-dioxotetradecyl)amino]-1,29-diheptyl-9,21-dihydroxy-9,21-dioxido-14,16-dioxo-4,8,10,20,22,26-hexaoxa-13,17-diaza-9,21-diphosphanonacosane-1,29-diyl] ester), ER804059 (sel disodique de l'acide 5 dodecenoïque 1,1'-[(1R,6S,24S,29R)-6,24-bis[(1,3-dioxotetradecyl)amino]-1,29-diheptyl-9,21-dihydroxy-9,21-dioxido-14,16-dioxo-4,8,10,20,22,26-hexaoxa-13,17-diaza-9,21-diphosphanonacosane-1,29-diyl] ester), ER804442 (sel disodique de l'acide dodécanoïque 1,1'-[(1R,6R.22R,27R)-6,22-bis[[(dodecylamino)carbonyl]amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester), ER804764 (acide dodécanoïque 1,1'-[(1R,6R,24R,29R)-1,29-diheptyl-10,20-dihydroxy-10,20-dioxido-15-oxo-6,24-bis[(1-oxotetradecyl)amino)-4,9,11,19,21,26-hexaoxa-14,16-diaza-10,20-diphosphanonacosane-1,29-diyl] ester), ER111232 (sel disodique de l'acide 5 dodecenoïque 1,1'-[(1R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester), ER112022 (sel disodique de l'acide 5 dodecenoïque 1,1'-[(1R,32R)-6,27-bis[(1,3-dioxotetradecyl)amino]-1,32-diheptyl-9,24-dihydroxy-9,24-dioxido-14,19-dioxo-4,8,10,23,25,29-hexaoxa-13,20-diaza-9,24-diphosphadotriacontane-1,32-diyl] ester), ER112048 (sel disodique de l'acide 5 dodecenoïque 1,1'-[(1R,28R)-1,28-diheptyl-8,21-dihydroxy-5,24-bis[[[(3R)-3-hydroxydecyl]oxy]methyl]-8,21-dioxido-3,13,16,26-tetraoxo-7,9,20,22-tetraoxa-4,12,17,25-tetraaza-8,21-diphosphaoctacosane-1,28-diyl] ester), ER 112065 (sel disodique de l'acide dodécanoïque 1,1'-[(1R,30R)-6,2S-bis[(1,3-dioxotetradecyl)amino]-1,30-diheptyl-9,22-dihydroxy-9,22-dioxido-14,17-dioxo-4,8,10,21,23,27-hexaoxa-13,18-diaza-9,22-diphosphatriacontane-1,30-diyl] ester), ER112066 (sel disodique de l'acide dodécanoïque 1,1'-[(1R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester), ER113651 (sel disodique de l'acide 5 dodecenoïque 1,1'-[(1R,29R)-6,24-bis[(1,3-dioxotetradecyl)amino]-1,29-diheptyl-9,21-dihydroxy-9,21-dioxido-14,16-dioxo-4,8,10,20,22,26-hexaoxa-13,17-diaza-9,21-diphosphanonacosane-1,29-diyl] ester), ER118989 (sel disodique de l'acide 5 dodecenoïque 1,1'-[(1R,29R)-11,29-diheptyl-9,21-dihydroxy-9,21-dioxido-14,16-dioxo-6,24-bis[(1-oxotetradecyl)amino]-4,8,10,20,22,26-hexaoxa-13,17-diaza-9,21-diphosphanonacosane-1,29-diyl] ester), ER119327 (sel disodique de l'acide dodécanoïque 1,1'-[(1R,27R)-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester), ER119328 (sel disodique de l'acide 5 dodecenoïque 1,1'-[(1R,27R)-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester).

Les composés peuvent être sous la forme de diastéréoisomères ou sous une forme racémique (mélange de diastéréoisomères) lorsque la structure chimique comporte plusieurs carbones asymétriques. Par exemple, ER804057 et ER804053 qui ont 4 carbones asymétriques sont des diastéréoisomères d'ER112066 qui est la forme racémique. ER804057 est dans une configuration isomérique de type (R,R,R,R) tandis que ER804053 est dans une configuration de type (R,S,S,R). De la même façon, ER804058 qui est dans une configuration isomérique de type (R,R,R,R) et ER804059 qui est dans une configuration isomérique de type (R,S,S,R) sont des diastéréoisomères de ER113651 qui est la forme racémique. ER803022 qui est dans une configuration de type (R,R,R,R), ER803732 qui est dans une configuration (R,S,S,R) et ER803789 qui est dans une configuration (R,R,S,R) sont également des diastéréoisomères d'une même molécule chimique. On utilise, de façon préférée, les diastéréoisomères qui ont une configuration de type R,R,R,R généralement plus actifs que les autres formes. Parmi ceux ci, ER804057 est particulièrement préféré. Il s'agit de l'acide dodecanoique *(1R,6R,22R,27R)*-1,27-diheptyl-9,19-dihydroxy-9,19-dioxido-14-oxo-6,22-bis[(1,3-dioxotetradecyl)amino]-4,8,10,18,20,24-hexaoxa-13,15-diaza-9,19-diphosphaheptacosane-1,27-diyl ester ; Il est sous forme d'acide libre ou sous forme de sel. Le poids moléculaire de la forme acide libre est de 1579, celui du sel disodique est de 1624. La formule brute du sel disodique est $C_{83}H_{158}N_4Na_2O_{19}P_2$.

Sur le plan structural, l'agoniste du TLR4 selon l'objet de l'invention est une molécule amphiphile. Les molécules amphiphiles, ont un comportement à la fois hydrophile et hydrophobe et ont tendance à précipiter au cours du temps. Elles se dissolvent souvent incomplètement dans les solvants organiques ou aqueux et sont souvent à l'origine de solutions instables ou difficilement reproductibles. Il existe un besoin d'améliorer la formulation de ces molécules. L'émulsion telle que décrite dans l'invention répond à ce besoin en fournissant des émulsions qui sont stables au cours du temps. Une émulsion selon l'invention qui est conservée pendant 6 mois à +4°C conserve les caractéristiques qu'elle avait initialement : la distribution de taille des gouttelettes d'huile ne varie pas d'une façon sensible; l'aspect laiteux, fluide est homogène de l'émulsion est conservé ; et de façon notoire, l'intégrité structurale du TLA4 n'est pas altérée comme le montre l'exemple II. On a même constaté qu'une émulsion selon l'invention pouvait être stockée à une température de -2°C pendant au moins 48 heures sans observer de variation notoire au niveau de la distribution de taille des gouttelettes d'huile. Par ailleurs, l'émulsion selon l'invention diminue le pouvoir pyrogène de certains agonistes du TLR4.

Le ratio entre la quantité de TLA4 et la quantité totale de tensioactifs hydrophile et hydrophobe de l'émulsion est habituellement entre $0{,}01 \times 10^{-2}$ et $5 \times 10^{-2}$, plus particulièrement entre $0{,}1 \times 10^{-2}$ et $2 \times 10^{-2}$. Dans cette gamme de ratio, la quantité de TLA4 est suffisamment faible pour ne pas exercer d'influence sur le pouvoir émulsionnant des tensioactifs

mais est en quantité suffisante pour exercer une activité immunostimulante *in vitro* et/ou *in vivo.*

Le tensioactif hydrophile selon l'invention a une HLB (balance hydrophile/lipophile) ≥ 10 et appartient au groupe chimique des polyoxyéthylène alkyl éthers (PAE), encore appelés éthers polyoxyéthylénés d'alcools gras. Ces tensioactifs non ioniques sont obtenus par condensation chimique entre un alcool gras et l'oxyde d'éthylène. Ils ont une formule chimique générale du type $R-(O-CH_2-CH_2)_n$ -OH dans laquelle le radical R désigne habituellement un reste alkyl saturé ou insaturé et n désigne le nombre d'unités d'oxyde d'éthylène. Selon l'objet de l'invention, R contient entre 1 et 50 atomes de carbone, de façon préférée entre 4 et 20 atomes de carbone et de façon particulièrement préférée entre 10 et 20 atomes de carbone. n est ≥ 2, généralement compris entre 4 et 50. L'émulsion selon l'invention comprend habituellement un seul PAE hydrophile. Un mélange de plusieurs PAE convient également dans la mesure ou l'HLB global est ≥ 10.

Les éthers polyoxyéthylénés d'alcools gras convenant à l'objet de l'invention peuvent être sous une forme liquide ou solide à température ambiante. Parmi les composés solides, on préfère ceux qui se dissolvent directement dans la phase aqueuse ou qui ne nécessitent pas de chauffage important.

Dans la mesure où le nombre d'unités d'oxydes d'éthylènes est suffisant, les éthers polyoxyéthylénés d'alcools laurique, myristique, cétylique, oléique et/ou stéarique sont particulièrement appropriés à l'objet de l'invention. On peut notamment les trouver dans la gamme des produits connus sous les appellations commerciales de Brij ®, Eumulgin ® ou Simulsol ®.

Une émulsion particulièrement préférée selon l'invention contient comme tensioactif hydrophile non ionique un polyoxyéthylène alkyl éther choisi dans le groupe constitué par du polyoxyéthylène (12) cétostearyl éther (ceteareth-12) (commercialisé sous la dénomination d'Eumulgin ® B1), du polyoxyéthylène (20) cétostéaryl éther (ceteareth-20) (Eumulgin ® B2), du poyoxyéthylène (21) stéaryl éther (steareth-21) (Eumulgin ® S21), du polyoxyéthylène (20) cétyl éther (ceteth-20) (Simulsol ® 58 ou Brij ®58), du polyoxyéthylène (10) cétyl éther (ceteth-10) (Brij ®56), du polyoxyéthylène (10) stéaryl éther (steareth-10) (Brij ®76), du polyoxyéthylène (20) stéaryl éther (steareth-20) (Brij ®78), du polyoxyéthylène (10) oleyl éther (oleth-10) (Brij ®96 ou Brij ®97, du polyoxyéthylène (20) oléyl éther (oleth-20) (Brij ®98 ou Brij ®99). Le nombre apposé à chaque nom chimique correspond au nombre d'unités d'oxyde d'éthylène dans la formule chimique.

Un composé particulièrement adapté et préféré en raison de son origine semi synthétique est l'Eumulgin ™ B1 (ceteareth-12) fourni par la société COGNIS.

L'émulsion selon l'invention contient également un tensioactif hydrophobe non ionique dont l'HLB est ≤ 6. L'émulsion comprend habituellement un seul tensioactif hydrophobe non ionique. Un mélange de plusieurs tensioactifs hydrophobes non ioniques convient également dans la mesure où l'HLB global est ≤ 6. Typiquement, il s'agit d'un ester hydrophobe du sorbitane ou d'un ester hydrophobe du mannide. Les esters de sorbitane sont habituellement obtenus par réaction d'estérification entre un acide gras et le sorbitol, le monoanhydre de sorbitol, ou le dianhydre de sorbitol. Les esters de mannide sont habituellement obtenus par réaction d'estérification entre un acide gras et le monoanhydre ou le dianhydre de mannitol. De façon préférée, il s'agit du mannide monooléate (commercialisé par la société SIGMA, ou fourni par la société SEPPIC sous la dénomination de Montanide 80 ™), ou du sorbitane monooléate commercialisé sous la dénomination de Span ®80, de Dehymuls SMO ™ (COGNIS) ou de montane 80 ™ (SEPPIC)).

Grâce à la sélection de ces tensioactifs particuliers parmi tous les tensioactifs proposés dans l'art antérieur pour préparer des émulsions, il a maintenant été trouvé que l'on pouvait très avantageusement produire une émulsion H/E adjuvante en utilisant un procédé d'inversion de phase facile à mettre en oeuvre.

Lorsque les concentrations respectives en tensioactifs hydrophile et hydrophobe sont telles que l'HLB du mélange ($HLB_m$) se situe entre 8,5 et 10, et plus particulièrement entre 8,6 et 9,6, les émulsions selon l'invention sont généralement homogènes et souvent au moins 90% de la population volumique des gouttelettes d'huile ont une taille ≤ 0,2 μm. Ces émulsions sont par ailleurs particulièrement stables. Les quantités de tensioactifs hydrophile et hydrophobe dans l'émulsion de squalène sont ajustées de préférence pour que l'$HLB_m$ se situe entre 8,5 et 10, et plus particulièrement pour que l'$HLB_m$ se situe entre 8,6 et 9,6. Pour déterminer les concentrations respectives en tensioactifs hydrophile et hydrophobe dans la composition de l'émulsion on utilise la formule suivante :

$$HLB_m = HLB_e \times M + HLB_{pae} \times (1-M)$$

dans laquelle,

$HLB_m$ correspond à l'HLB du mélange qui est de préférence entre 8,5 et 10, et plus particulièrement entre 8,6 et 9,6

$HLB_e$ correspond à l'HLB du tensioactif hydrophobe

M correspond au pourcentage en masse du tensioactif hydrophobe dans le mélange constitué par le tensioactif hydrophobe et du PAE

HLB $_{pae}$ correspond à l'HLB du PAE

Le squalène, qui représente la phase huileuse de l'émulsion, a pour formule chimique brute $C_{30}H_{50}$ et comprend 6 double-liaisons. Cette huile est métabolisable et présente les qualités requises pour être utilisée dans un produit pharmaceutique injectable. Elle provient du foie de requin (origine animale) mais peut être extraite également de l'huile d'olive (origine végétale). On a notamment obtenu de bons résultats en utilisant le squalène fourni par la société Fluka qui est d'origine animale. Généralement, la quantité de squalène représente entre 5 et 45% du poids total de l'émulsion.

Le ratio massique entre la quantité de squalène et la quantité totale de tensioactifs dans l'émulsion selon l'invention est habituellement compris entre 2,0 et 4,0, de façon préférée entre 2,5 et 3,5.

Une composition de l'émulsion selon l'invention particulièrement préférée comprend :

- du squalène,
- un tampon phosphate ou un tampon citrate comme solvant aqueux,
- le composé ER 804057 comme agoniste du TLR4,
- le ceteareth-12 (Eumulgin ® B1) comme tensioactif hydrophile,
- du monooléate de sorbitane comme tensioactif hydrophobe.

La quantité de squalène représente entre 5 et 45% du poids total de l'émulsion. La quantité du composé ER804057 représente habituellement entre 0,05% et 2% du poids des deux tensioactifs. De façon préférée, les quantités de ceteareth-12 et de monooléate de sorbitane sont telles que l'HLB du mélange des deux tensioactifs se situe entre 8,5 et 10 et plus particulièrement entre 8,6 et 9,6. Le ratio entre la quantité de squalène et la quantité totale de ceteareth-12 et de monooléate de sorbitane est compris entre 2,0 et 4,0, de façon préférée entre 2,5 et 3,5. En outre, cette composition peut contenir du mannitol dont la quantité représente habituellement entre 0, 1 % et 10% du poids total de l'émulsion.

La phase aqueuse de l'émulsion selon l'invention peut contenir en outre un substrat de lyophilisation contenant un ou plusieurs cryoprotecteurs. Les cryoprotecteurs sont habituellement choisis parmi les sucres comme le saccharose, les poly alcools comme le mannitol ou le sorbitol ou les dérivés de sucres tels que les alkyl poly glycosides comme le décyl-D galactoside uronate de sodium ou le dodécyl β maltoside. On utilise habituellement un substrat de lyophilisation qui contient en mélange du saccharose, du mannitol et du dodécyl β maltoside . L'émulsion selon l'invention peut être alors lyophilisée et conservée sous forme de lyophilisat. Elle conserve toutefois toutes ses caractéristiques car une fois reprise en phase aqueuse elle redevient une émulsion H/E laiteuse, stable et fluide, thermoréversible avec une distribution de taille de gouttelettes d'huile similaire à celle qui préexistait avant lyophilisation.

L'émulsion selon l'invention joue également le rôle d'adjuvant de la réponse immune à un antigène. Par antigène au sens de la présente invention, on entend tout antigène susceptible d'être utilisé dans un vaccin, qu'il s'agisse d'un germe entier vivant, atténué ou tué, d'un extrait de germe ou d'une forme sous unitaire. Lorsqu'il est sous une forme sous unitaire, la nature de l'antigène importe peu: il peut être un peptide, une protéine, une glycoprotéine, un polysaccharide, un glycolipide, un lipopeptide, un acide nucléique. Parmi les antigènes convenant à l'objet de l'invention, on cite les antigènes bactériens provenant de *Clostridium tetani,* de *Clostridium diphteriae,* de *Bordetella pertussis,* d'*Haemophilus influenzae type b,* de *Streptococcus pneumoniae,* de *Neisseria meningitidis,* de *Shigella sp.,* de *Salmonella typhi,* de *Staphylococcus aureus* ou de *Staphylococcus epidermidis,* de *Mycobacterium tuberculosis,* de *Chlamydia trachomatis* ou de *Streptococcus sp,* d'antigènes viraux provenant du virus de l'hépatite A, B, ou C, du virus de la grippe, du virus syncytial respiratoire, du virus West Nile, du virus de la rage, du poliovirus, du virus HIV, du virus de la dengue, de l'encéphalite japonaise, du virus de la fièvre jaune, du cytomegalovirus, ou des herpes virus, d'antigènes parasitaires provenant notamment de *Plasmodium sp.* ou d'antigènes tumoraux. Ces antigènes peuvent être obtenus en utilisant des procédés de recombinaison génétique ou en utilisant des procédés d'extraction bien connus de l'homme du métier. L'émulsion selon l'invention agit sur l'immunité humorale en augmentant la production d'anticorps spécifiques et/ou sur l'immunité cellulaire spécifique en favorisant notamment la prolifération des lymphocytes T, le développement d'une réponse cytolytique spécifique (réponse CTL), et/ou la production de cytokines, chemokines, facteurs de croissance, produites par les lymphocytes activés.

C'est pourquoi l'invention a également pour objet l'utilisation d'une émulsion selon l'invention pour la préparation d'une composition vaccinale. La composition vaccinale obtenue s'avère plus immunogénique, par exemple parce que la composition induit une réponse immune spécifique plus forte qu'elle soit d'ordre humorale et/ou d'ordre cellulaire, ou parce qu'une quantité d'antigène plus faible est nécessaire pour obtenir une réponse immune de même intensité et de durée comparable. La composition vaccinale obtenue à partir d'une émulsion selon l'invention peut être administrée par toutes les voies habituellement utilisées ou préconisées pour les vaccins : voie parentérale, voie intradermique, voie sous cutanée, ou intra musculaire ou la voie mucosale, et se présenter sous diverses formes notamment liquide ou lyophilisée. Elle peut être administrée au moyen d'une seringue ou au moyen d'un injecteur sans aiguille pour injection intramusculaire, sous-cutanée ou intradermique, ou au moyen d'un spray nasal.

La composition vaccinale est généralement sous la forme d'un mélange de l'antigène avec une émulsion selon l'invention. Elle peut se présenter aussi sous la forme d'une formulation extemporanée. Dans ce cas, l'antigène et l'émulsion sont mis en contact juste avant ou au moment de l'administration de la composition vaccinale. Par exemple, l'antigène peut être lyophilisé et repris par l'émulsion juste avant l'administration ou à l'inverse, l'émulsion peut être sous une forme lyophilisée et repris par une solution de l'antigène. La composition vaccinale peut se trouver également dans un dispositif particulier d'injection comme la seringue « by pass » lorsque l'on ne souhaite pas mélanger l'antigène avec l'émulsion.

Lorsque la composition vaccinale est sous la forme d'un mélange obtenu par dilution d'une émulsion selon l'invention avec une solution d'antigène, elle est habituellement sous la forme d'une émulsion H/E dans laquelle la quantité de squalène représente en poids en général entre 0,5 et 5% du poids total de la composition vaccinale. Elle peut être également sous la forme d'une émulsion H/E thermoréversible lorsque la quantité de squalène dans la composition vaccinale atteint ou dépasse 5% (p/p). Lorsque la composition vaccinale est une émulsion H/E thermoréversible, elle peut être, en particulier, sous une forme dans laquelle au moins 90% de la population volumique des gouttelettes d'huile a une taille $\leq$ 0,2 $\mu$m.

D'une façon surprenante, l'émulsion selon l'invention a une plus grande capacité à induire des anticorps neutralisants qu'une émulsion H/E de l'art antérieur obtenue par microfluidisation dont la composition renferme du squalène, du polyoxyéthylène sorbitane monooléate (Tween® 80) et du sorbitane trioleate (Span® 85) (émulsion H/E de l'art antérieur)

Les anticorps neutralisants sont des anticorps fonctionnels dirigés contre un germe infectieux, produits par un individu qui a été immunisé avec un antigène apparenté ou dérivé de ce germe ou qui a été en contact avec ce germe, et qui empêchent l'infection des cellules par ce germe. Ils jouent un rôle très important dans la prévention ou le traitement des infections causées par des germes intracellulaires, en particulier les virus et les parasites unicellulaires notamment plasmodium sp.. Des antigènes provenant de la forme «sporozoïte» de *Plasmodium falciparum,* (tels que la protéine majeure de surface du sporozoïte (circumsporozoite protein), LSA3, ou l'antigène Pfs 16), ainsi que des antigènes provenant de la forme « mérozoïte » de *Plasmodium falciparum* (tels que l'antigène MSP1, MSP2, MSP3, EBA-175, Rhop-1, Rhop-2, Rhop-3, RAP-1, RAP-2, RAP-3, Pf155/RESA ou AMA-1) induisent des anticorps neutralisants. L'utilisation d'une émulsion selon l'invention pour préparer une composition vaccinale contenant un ou plusieurs antigènes issus des sporozoïtes ou des mérozoïtes de *Plasmodium falciparum* est indiquée pour amplifier la réponse immune neutralisante. On peut en particulier utiliser une émulsion selon l'invention pour préparer une composition vaccinale comprenant comme antigène vaccinal la protéine LSA3 de Plasmodium falciparum. Le gène codant pour cette protéine a été identifié par Gardner et al., (Science (1998), 282, 1126-1132) et se trouve sur le chromosome 2 de Plasmodium falciparum (souche 3D7). La séquence entière du gène est longue de 12240 paires de bases et code pour une protéine de 1558 acides aminés. Les séquences nucléotidique et protéique sont décrites dans la banque de données EMBL sous les numéros d'accession AE001424 et uniprot 096275-PLAF7. Comme antigène vaccinal, on peut utiliser la protéine entière telle que décrite, des peptides, ou des fragments de cette protéine, comme ceux décrits dans WO 02/38176. Habituellement, on utilise la protéine entière (qui peut contenir une ou plusieurs mutations ponctuelles pour tenir compte des variations qui existent entre les souches de Plasmodium falciparum) ou un fragment de cette protéine dont la séquence d'acides aminés a une identité d'au moins 80% par rapport à la séquence entière décrite dans uniprot 096275-PLAF7. L'efficacité de certains vaccins antiviraux est dans certains cas évaluée sur la base du taux d'anticorps neutralisants qu'ils induisent. C'est le cas du vaccin contre la grippe dont l'efficacité est reliée au taux d'anticorps inhibant l'hémagglutination (IHA).

L'émulsion selon l'invention sert à préparer une composition vaccinale pour le traitement ou la prévention des maladies infectieuses chez l'homme ou l'animal (oiseaux, cheval) liées au virus de la grippe. Selon la nature du vaccin contre la grippe la composition vaccinale peut se présenter sous différentes formes :

- Lorsque le vaccin grippal contient une ou plusieurs souches de virus entiers inactivés ou « splittés », ou est sous la forme d'un vaccin sous unitaire contenant de l'hémagglutinine purifiée d'une ou plusieurs souches virales, ou sous la forme de virosomes (vaccin Berna), la composition vaccinale, se présente habituellement sous la forme d'un mélange, d'une émulsion H/E ou d'une émulsion H/E thermoréversible.
- Lorsque le vaccin grippal contient une ou plusieurs souches de virus vivants atténués, la composition vaccinale se trouve de façon préférée dans un dispositif, type seringue by pass, de façon à ce que le virus vivant ne soit pas au contact direct de l'émulsion. La suspension virale et l'émulsion selon l'invention se trouve dans deux compartiments séparés de la seringue.

Les vaccins contre la grippe sont fabriqués à partir de virus grippaux cultivés sur oeufs ou sur cellules selon des méthodes bien connues de l'homme du métier et comprennent tous comme composant essentiel l'hémagglutinine d'une ou plusieurs souches virales.

L'invention a donc également pour objet l'utilisation d'une émulsion selon l'invention pour la préparation d'une

composition vaccinale comprenant comme antigène vaccinal une ou plusieurs hémagglutinines du virus grippal. Cette composition vaccinale peut être utilisée pour vacciner :

1) des populations d'individus qui sont séronégatives vis-à-vis du virus grippal : il s'agit d'individus qui n'ont jamais été en contact ou sensibilisés avec le virus grippal ou ses composants immunogéniques, ou d'individus qui n'ont jamais été en contact avec une nouvelle souche de virus grippal à l'origine de pandémie ;
2) des populations d'individus séropositifs vis-à-vis du virus grippal : il s'agit d'individus qui ont déjà été en contact ou sensibilisés avec le virus grippal ou ses composants immunogéniques ;
3) des populations d'individus âgés qui présentent souvent une altération de l'immunité cellulaire et/ou humorale qui s'observe notamment vis-à-vis du virus de la grippe.

L'émulsion selon l'invention sert également à préparer une composition vaccinale pour le traitement ou la prévention des maladies infectieuses à herpes virus (HSV1, HSV2, cytomégalovirus (CMV)). Les antigènes de l'enveloppe virale sont généralement utilisés dans la composition vaccinale. Dans les infections à CMV, les anticorps qui sont dirigés contre les protéines de l'enveloppe virale, principalement la glycoprotéine B (gB) et la glycoprotéine H (gH) et qui neutralisent l'infection virale, jouent un rôle très important dans le développement d'une immunité protectrice. L'utilisation d'une émulsion selon l'invention dans la préparation d'une composition vaccinale contenant une protéine de l'enveloppe du CMV a pour effet d'augmenter la production d'anticorps neutralisants.

L'invention a donc pour objet l'utilisation d'une émulsion selon l'invention pour la préparation d'une composition vaccinale comprenant comme antigène vaccinal un antigènes de l'enveloppe du CMV. Typiquement, l'antigène est la glycoprotéine gB et/ou la glycoprotéine gH. Il peut s'agir également d'un peptide ou d'un polypeptide dérivé de la gB et/ou de la gH comprenant un ou plusieurs épitopes neutralisants.

gB dans sa forme native (gp130), codée par le gène UL 55 du CMV, est une glycoprotéine de 906 ou 907 acides aminés, selon qu'il s'agit de la souche AD169 ou de la souche Towne. Les séquences protéiques de ces deux souches sont décrites dans US 2002/0102562 (figure 2). La forme native de la gB contient une séquence signal, suivie d'un domaine extracellulaire contenant un site de clivage endoprotéolytique entre les résidus Arginine 460 et Sérine 461, d'un domaine transmembranaire et d'un domaine intracellulaire. On a décrit plusieurs domaines antigéniques induisant des anticorps neutralisants. Il s'agit notamment du domaine qui se situe entre les résidus d'acides aminés 461 et 680 de la gp 130, ce domaine se subdivisant en deux domaines discontinus, le domaine compris entre les résidus 461 et 619 et le domaine compris entre les résidus 620 et 680 (US 5,547,834). Il s'agit également du domaine AD-1 localisé entre les résidus d'acides aminés 552 et 635 ou du domaine AD-2 localisé entre les résidus d'acides aminés 50 et 77 (Journal of Général Virology (1999), 80, 2183-2191 ; Journal of Virology (2005), 79, 4066-4079).Par conséquent, un polypeptide qui comprend dans sa séquence d'acides aminés une séquence homologue à l'un des domaines cités convient à l'objet de l'invention. Typiquement, le polypeptide comprend dans sa séquence d'acides aminés, une séquence homologue à celle qui se situe entre les résidus 461 et 680 de la gp130 ou plus spécifiquement à celle qui se situe entre les résidus 552 et 635. Par séquence homologue, on entend toute séquence d'acides aminés dont l'identité est d'au moins 80% avec la séquence d'acides aminés du domaine antigénique considéré situé sur la gp 130 de la souche Towne ou AD169 (décrites dans US 2002/0102562), Typiquement, l'homologie de séquence est basée sur une identité d'au moins 90%, et de façon encore plus particulière l'homologie de séquence est basée sur une identité de séquence de 100%.

Parmi les peptides ou polypeptides dérivés de gB convenant à l'objet de l'invention, on cite notamment la gp 55 telle que décrite dans US 5,547,834. Elle est issue du clivage de la gB au niveau du site de clivage endo-protéolytique ; sa séquence d'acides aminés correspond à celle qui se situe entre les résidus sérine 461 et l'extrémité C-terminale. On peut également utiliser des formes tronquées de la gp55, telle qu'une gp 55 dépourvue de tout ou partie de la séquence transmembranaire et de tout ou partie du domaine C-terminal intra-cellulaire (par exemple un peptide ayant une séquence homologue à la séquence d'acides aminés de la gp130 comprise entre les résidus 461 et 646) ou une gp 55 dépourvue de tout ou partie du domaine C-terminal intra-cellulaire (par exemple un peptide ayant une séquence homologue à la séquence d'acides aminés de la gp130 comprise entre les résidus 461 et 680) qui sont décrites dans US 5,547,834. On peut aussi utiliser une forme mutée de la gB qui porte une ou plusieurs mutations au niveau du site de clivage endo-protéolytique de sorte que celui-ci est rendus inopérant. La(les) mutation(s) est (sont) localisée(s) entre les résidus 457 et 460 de la séquence de la gp130 et plus particulièrement se situe(nt) au niveau de l'arginine 460 et/ou la lysine 459 et/ou l'arginine 457. Un antigène d'enveloppe du CMV convenant particulièrement à l'objet de l'invention est une forme tronquée de la gB dépourvue de tout ou partie du domaine C-terminal et/ou dépourvue de tout ou partie de la séquence transmembranaire et dont le site de clivage est inopérant. Une forme tronquée de la gB particulièrement préférée correspond à celle qui est décrite dans US 6,100,064, nommé gBdTM ; elle porte trois mutations au niveau du site de clivage et une délétion au niveau de la région transmembranaire entre les résidus d'acides aminés Valine 677 et Arginine 752 de sorte que le domaine extracellulaire est directement relié au domaine cytoplasmique.

La protéine gB ou les peptides, polypeptides dérivés de celle ci sont obtenus au moyen de procédés de

recombinaison génétique et purifiés selon des méthodes bien connues de l'homme de l'art. On peut utiliser notamment les procédés décrits dans US 6,100,064 et dans US 2002/0102562 incorporés par référence. Pour augmenter leur immunogénicité on peut secondairement les conjuguer à une protéine porteuse ou les fusionner à d'autres protéines, notamment à des protéines formant des particules comme l'antigène de surface de l'hépatite B (HbS). La protéine gB ou les peptides dérivés de celle-ci peuvent également être exprimés par des virus recombinants, notamment par des adénovirus recombinants ou des poxvirus recombinants. Pour la préparation de ces vecteurs recombinants exprimant la gB ou des peptides dérivées, on utilise les méthodes qui sont décrites notamment dans US 6,162,620, US 5,866,383, US 5,552,143, US 6,183,750, US 5,338,683, WO 9215672 ou dans WO 9639491. La gB peut être également présentée par une souche de CMV qui a été atténuée par passages successifs sur des cultures cellulaires, notamment la souche Towne qui a déjà été testée à des fins vaccinales.

La protéine gH est codée par le gène *UL 75* du CMV. C'est une glycoprotéine de 742 ou 743 acides aminés selon qu'il s'agit de la souche Towne ou de la souche AD169. Les séquences sont décrites dans US 5,474,914 (figure 1) et dans US 6,610,295 (figure 5(a)). La séquence protéique de la gH déduite de sa séquence nucléotidique contient un peptide signal suivi d'un domaine extracellulaire ne possédant pas de site de clivage endoprotéolytique, d'un domaine transmembranaire et d'un domaine cytoplasmique C-terminal. Les épitopes neutralisants se situent dans le domaine extracellulaire, principalement dans la partie N-terminale de ce domaine, plus spécifiquement entre les résidus d'acides aminés 15 et 142 de la séquence protéique de la gH native et de façon encore plus spécifique entre les résidus d'acides aminés 33 et 142. Un épitope majeur neutralisant de la souche AD 169 a été identifié et se situe entre les résidus 33 et 43 de la séquence de la gH et a pour séquence LDPHAFHLLL (Urban M et al. : J. Virol (1992, vol 66/3, p1303-1311)). Par conséquent, un polypeptide qui comprend dans sa séquence d'acides aminés une séquence homologue à la séquence LDPHAFHLLL ou une séquence homologue à celle qui se situe entre les résidus 15 et 142 ou entre les résidus 33 et 142 de la séquence protéique de la gH convient à l'objet de l'invention. Par séquence homologue, on entend une séquence d'acides aminés dont l'identité est d'au moins 80% avec la séquence d'acides aminés qui se situe soit entre les résidus 15 et 142, soit entre les résidus 33 et 142 de la séquence protéique de la gH de la souche AD 169, ou avec la séquence LDPHAFHLLL Plus particulièrement, l'homologie de séquence est basée sur une identité d'au moins 90%, et de façon encore plus particulière l'homologie de séquence est basée sur une identité de séquence de 100%.

Comme peptides ou polypeptides dérivés de la gH convenant à l'objet de l'invention on cite la gH dépourvue de tout ou partie de sa région transmembranaire et/ou dépourvue de tout ou partie de sa région cytoplasmique. Typiquement, cela correspond à une protéine gH qui est délétée d'au moins 5 résidus, de façon préférée d'au moins 10 résidus C-terminaux et de façon encore plus préférée entre 20 et 34 résidus de l'extrémité C-terminale de la séquence d'acides aminés.

La protéine gH, les polypeptides ou les peptides dérivés de celle-ci sont obtenues au moyen de procédés de recombinaison génétique et purifiées selon des méthodes bien connues de l'homme de l'art notamment celles décrites dans US 5,474,914 ou dans US 5,314,800 incorporés par référence. Pour augmenter leur immunogénicité on peut secondairement les conjuguer à une protéine porteuse. Ils peuvent être aussi produits sous la forme de protéines de fusion comme cela est décrit dans J. Virol (1992, vol 66/3, p1303-1311). La protéine gH, les polypeptides ou les peptides dérivés de celle-ci peuvent également être exprimés par des virus recombinants, notamment par des adénovirus recombinants ou des poxvirus recombinants. Pour la préparation de ces vecteurs recombinants exprimant la gH ou des formes dérivées, on utilise les méthodes qui sont décrites notamment, dans US 6,162,620 , US 5,866,383, US 5,552,143, ou dans WO 9639491. La protéine gH peut être également présentée par une souche de CMV qui a été atténuée par passages successifs sur des cultures cellulaires, notamment la souche Towne qui a déjà été testée à des fins vaccinales.

On peut également utiliser comme antigène vaccinal une protéine résultant de la fusion entre la glycoprotéine gB ou la glycoprotéine gH (ou une forme tronquée de celles-ci) et une protéine de membrane de HSV1 ou de HSV2 (ou une forme tronquée de celles-ci). A titre d'exemple, on cite les protéines de fusion décrites dans EP 0759995, en particulier gB 685* et gB 685** qui résultent de la fusion entre une portion de la glycoprotéine gB du CMV et une portion de la glycoprotéine gD de HSV.

Selon la nature de l'antigène du CMV, la composition vaccinale peut se présenter sous différentes formes :

- Lorsque l'antigène est une protéine ou un peptide, la composition vaccinale peut être sous la forme d'un mélange, d'une émulsion H/E ou d'une émulsion H/E thermoréversible. Elle peut être aussi sous la forme d'une préparation extemporanée que l'on réalise juste avant administration. La composition vaccinale peut également se trouver à l'intérieur d'un dispositif, tel qu'une seringue « bypass » qui sépare physiquement l'antigène de l'émulsion.
- Lorsque l'antigène du CMV est sous la forme d'un virus recombinant exprimant la gB, la gH ou un peptide dérivé de la gB ou de la gH, ou lorsqu'il est sous la forme d'une souche atténuée d'un CMV, l'antigène et l'émulsion selon l'invention ne sont pas habituellement directement en contact dans la composition vaccinale. L'antigène et l'émulsion peuvent se trouver à l'intérieur d'un dispositif qui les sépare physiquement, tel qu'une seringue « bypass », mais ils sont administrés au même moment sur le même site d'administration.

L'émulsion selon l'invention oriente également la réponse cellulaire T CD4+ spécifique vers un profil de type Th1 en favorisant la production de cytokines de type Th1 (IL2, IFN-γ,....) et/ou en diminuant la production de cytokines de type Th2 (IL4, IL5, IL10,...) en réponse à un antigène présenté dans un contexte MHC classe II. Cet effet est évalué en mesurant le taux d' IFN-γ et d'IL5 produits après re-stimulation *in vitro* avec un antigène apparenté à celui qui a servi à l'immunisation *in vivo* et en déterminant le ratio IFN-γ /IL5. Plus le ratio est élevé et plus la réponse CD4+ est de type Th1. On peut également évaluer indirectement le profil de la réponse cellulaire T CD4+ en mesurant le ratio entre le taux d'Ig2a spécifiques/ IgGl spécifiques obtenu après immunisation des souris avec une composition vaccinale selon l'invention.

L'émulsion selon l'invention peut donc être utilisée pour corriger un déséquilibre de la réponse cellulaire T CD4+ qui s'observe dans certaines populations d'individus qui présentent un déficit immunitaire ou une altération du système immunitaire. Il s'agit notamment des personnes âgées qui présentent un déficit en production d'IFN-γ et/ou en IL2 consécutivement à une stimulation *in vitro* avec un antigènes provenant de germes intracellulaires, notamment avec un antigène de la grippe ( Ouyang et al (Mechanisms of ageing and development), 2000, vol 121, 131-137).
L'invention a donc pour objet l'utilisation d'une émulsion selon l'invention pour la préparation d'une composition vaccinale destinée à une population d'individus qui présentent un déséquilibre au niveau de la réponse cellulaire T CD4+.

L'invention a également pour objet un procédé de préparation d'une émulsion H/E selon l'invention comprenant une étape où une émulsion inverse E/H est obtenue par élévation de température et une étape où l'émulsion inverse E/H est transformée en émulsion H/E par abaissement de température. Cette transformation se produit lorsque l'émulsion E/H obtenue est abaissée à une température inférieure à la température d'inversion de phase de cette émulsion.

Selon un mode de réalisation du procédé, l'émulsion E/H est obtenue en mélangeant dans une première étape une phase aqueuse comprenant un solvant aqueux, un polyoxyéthylène alkyl éther et un agoniste du TLR4 avec une phase huileuse comprenant du squalène et un tensioactif hydrophobe non ionique pour obtenir une émulsion H/E et en chauffant dans une deuxième étape l'émulsion H/E à une température qui est au moins la température d'inversion de phase de l'émulsion.
On incorpore la phase aqueuse comprenant la solution aqueuse (habituellement une solution tamponnée), l'agoniste du TLR4 (s'il n'est pas dans la phase huileuse) et le tensioactif hydrophile non ionique, dans la phase huileuse comprenant le squalène, et le tensioactif non ionique hydrophobe, ou bien l'inverse : on incorpore la phase huileuse dans la phase aqueuse. Cette incorporation se fait sous agitation mécanique. On obtient une émulsion H/E grossière, non calibrée et instable (pré-émulsion). On chauffe cette pré-émulsion sous agitation mécanique jusqu'à obtention d'une inversion de phase, c'est-à-dire l'obtention d'une émulsion E/H. La transition ou inversion de phase peut être suivie par conductimétrie. La température à laquelle se produit le changement de courbure traduisant le passage d'un type d'émulsion à un autre est la température d'inversion de phase. En réalité, cette température est plutôt un intervalle de température qu'une valeur ponctuelle très précise ; en effet, on peut considérer que cette température est susceptible d'une variation d'un ou deux degrés, ceci afin que la totalité de l'émulsion subisse le phénomène d'inversion de phase. Lorsque l'émulsion est sous la forme d'une émulsion E/H on observe une chute brutale de la conductivité. On arrête le chauffage et on refroidit le mélange. Le refroidissement peut être effectué de façon passive, en laissant simplement la température revenir spontanément à la température ambiante ou de manière plus active, en effectuant par exemple une trempe de l'émulsion dans un bain de glace. Lors de la descente en température, l'émulsion E/H va à nouveau s'inverser à la température d'inversion de phase pour redonner une émulsion H/E. L'émulsion peut être stockée en l'état en attendant d'être diluée par une solution comprenant l'antigène vaccinal. Elle est thermoréversible, ce qui signifie que si elle est portée à nouveau à une température au moins égale à la température d'inversion de phase, elle va redevenir une émulsion E/H. La température d'inversion de phase est habituellement entre 45 et 80 °C, et de façon typique entre 50 et 65°C. On soumet ainsi les composants de l'émulsion, notamment l'agoniste du TLR4 à un chauffage modéré ce qui évite une évaporation de la phase aqueuse ou une dégradation chimique des composants.

Selon un autre mode de réalisation, l'émulsion E/H est obtenue en chauffant séparément une phase aqueuse comprenant un solvant aqueux, un polyoxyéthylène alkyl éther et un agoniste du TLR4 et une phase huileuse comprenant du squalène et un tensioactif hydrophobe non ionique à une température qui est au moins égale à la température d'inversion de phase de l'émulsion puis en mélangeant la phase aqueuse avec la phase huileuse tout en maintenant la température du mélange à une température qui est au moins égale à la température d'inversion de phase.
Dans ce cas on chauffe séparément les phases aqueuse et huileuse à une température légèrement supérieure à la température d'inversion de phase, avant de les mélanger pour donner une émulsion inverse E/H qui sera ensuite refroidie jusqu'à obtention de l'émulsion submicronique H/E. Ces opérations peuvent être réalisées dans des réservoirs séparés pour une préparation en lots.
Il est également possible d'utiliser un procédé de fabrication en ligne. Le procédé consiste à mélanger à chaud les deux phases aqueuse et huileuse préparées séparément, au travers d'un mélangeur statique thermostaté, suivi d'un refroidissement en ligne au travers d'un échangeur thermique réfrigéré connecté en sortie du mélangeur statique, puis de la récupération finale de l'émulsion selon l'invention dans un récipient approprié (flacon ou réacteur). On a utilisé avec succès un mélangeur statique constitué d'une succession d'éléments de mélange composés de lames croisées et

inclinées par rapport à l'axe du tube dans lequel ils sont introduits. L'énergie nécessaire au mélange est fournie par les pompes qui véhiculent les fluides et le mélange est réalisé sans pièce mobile, au travers des éléments de mélange par la séparation, le déplacement et la réunion successive des constituants du mélange.

Le procédé de fabrication en ligne est mis en oeuvre selon la manière suivante : on prépare séparément, comme précédemment la phase aqueuse et la phase huileuse dans deux flacons ou réacteurs. Les deux phases sont chauffées sous agitation à une température légèrement supérieure à la température d'inversion de phase. Les deux phases sont alors introduites dans un mélangeur statique thermostaté au moyen de 2 pompes, dont les débits sont régulés de manière à obtenir la composition de l'émulsion selon l'invention. L'émulsion inverse E/H est obtenue durant le passage des deux phases dans le mélangeur statique. L'émulsion inverse est ensuite refroidie par passage en ligne au travers d'un échangeur thermique réfrigéré connecté en sortie du mélangeur statique. L'émulsion E/H va alors s'inverser au travers de l'échangeur thermique réfrigéré pour donner lieu à une émulsion H/E, qui sera réceptionnée dans un flacon ou réacteur et dont les caractéristiques sont identiques à celles de l'émulsion obtenue par un procédé en lots.

Il existe des alternatives aux modes de réalisation du procédé qui viennent d'être décrits ; lorsque l'agoniste du TLR4 a un comportement plus hydrophobe qu'hydrophile, on l'introduit dans la phase huileuse plutôt que dans la phase aqueuse. L'agoniste du TLR4 peut également être introduit une fois que le mélange de la phase huileuse et la phase aqueuse a été réalisé, ou lorsque l'émulsion a déjà été chauffée et qu'elle se trouve sous une forme d'émulsion E/H. La phase aqueuse peut contenir en outre un alditol. Enfin le procédé de préparation l'émulsion selon l'invention peut comprendre plusieurs cycles de thermoinversion successifs.

L'invention a également pour objet un procédé de préparation d'une composition vaccinale, dans lequel on mélange au moins un antigène vaccinal avec une émulsion H/E contenant un agoniste du TLR4 dont la structure chimique ne comporte pas de noyau sucré, caractérisé en ce que l'émulsion H/E contenant l'agoniste du TLR4 a été préparée selon un procédé d'inversion de phase comprenant une étape où l'on obtient une émulsion sous la forme d'émulsion inverse E/H par augmentation de la température et une étape où l'on transforme l'émulsion E/H en émulsion H/E par abaissement de la température.

Un mode de réalisation simple consiste à mélanger une solution aqueuse d'un antigène vaccinal dans une émulsion thermoréversible H/E obtenue selon l'un des modes de réalisation qui viennent d'être décrits. La composition vaccinale obtenue est sous la forme d'une émulsion H/E. ou sous la forme d'une émulsion H/E thermoréversible lorsque la quantité de squalène représente en poids au moins 5% du poids total de la composition vaccinale. De façon alternative, on peut mélanger l'antigène à la phase aqueuse ou à la phase huileuse avant de préparer l'émulsion. Une telle façon de procéder implique bien sûr qu'il s'agisse d'antigènes qui soient compatibles avec le procédé de thermoinversion. Les solutions d'antigènes peuvent en outre contenir des sels minéraux et un ou plusieurs tampons, ainsi que tout autre composé habituellement utilisé dans les vaccins tels que des stabilisants, des conservateurs, ou éventuellement aussi d'autres adjuvants. A titre indicatif, la concentration en antigène dans les solutions aqueuses est généralement comprise entre 1 $\mu$g/ml et 1 mg/ml.

Le procédé selon l'invention peut également inclure une étape de lyophilisation. On prépare tout d'abord une émulsion concentrée liquide comme cela vient d'être décrit mais en choisissant de préférence comme solution aqueuse de l'eau plutôt qu'une solution tamponnée. On dilue ensuite cette émulsion dans un substrat de lyophilisation comprenant un alditol, un sucre et un alkylpolyglycoside. Un substrat de lyophilisation habituellement employé comprend du mannitol, du saccharose et du dodécylmaltoside. L'émulsion diluée est alors répartie en échantillons (par exemple 0,5ml) et soumise à un cycle de lyophilisation qui peut s'effectuer de la manière suivante :

- chargement des échantillons à +4°C,
- environ 2 heures de congélation à une température de consigne de - 45°C,
- 14 à 19 heures de dessiccation primaire à une température de consigne de 0°C,
- 3 heures 30 de dessiccation secondaire à une température de consigne de + 25°C. Le lyophilisat obtenu est généralement conservé à une température voisine de +4°C avant d'être mélangé à un ou plusieurs antigènes vaccinaux. Une composition vaccinale selon l'invention peut être ainsi préparée en reprenant l'émulsion lyophilisée au moyen d'une solution aqueuse d'antigènes puis conservée en l'état (*i.e.* à l'état liquide), ou être soumise à un nouveau cycle de lyophilisation afin d'être conservée sous forme de lyophilisat, si la nature des antigènes le permet.
  De manière alternative, il est possible de diluer directement l'émulsion concentrée avec une solution aqueuse comprenant à la fois les antigènes vaccinaux ainsi que l'alditol, le sucre et l'alkylpolyglycoside, et de soumettre ensuite la composition obtenue à la lyophilisation. Une telle façon de procéder implique bien sûr qu'il s'agisse d'antigènes qui soient compatibles avec un procédé de lyophilisation.

Les exemples qui suivent illustrent de façon non limitative différents modes de réalisation de l'invention

Exemple I : Préparation d'une émulsion H/E thermoréversible concentrée à 32,4% de squalène (p/p)

On a préparé une solution de mannitol à 18% en tampon phosphate (p/p) sous agitation mécanique à 40°C. A 0,454g de cette solution on a rajouté 0,093g d'Eumulgin ™ B1 que l'on a homogénéisé sous agitation mécanique à 40°C pendant 5 min.
On a préparé une suspension mère à 1000μg/ml du composé chimique ER804057 dans un tampon TRIS 50mM. On a ajouté 390μl de la suspension mère de ER804057 au mélange Eumulgin ™ B1/mannitol.
Dans un autre récipient on a mélangé 0,073g de Dehymuls ™ SMO et 0,484g de squalène que l'on a homogénéisé par agitation magnétique pendant 5 minutes à 30°C.
On a incorporé ensuite sous agitation à environ 30°C le contenu de la phase aqueuse contenant ER804057 à la phase huileuse contenant le mélange Dehymuls ™ SMO /squalène.
On a chauffé l'émulsion brute obtenue, sous agitation mécanique, jusqu'à ce que la température atteigne 60°C. Cette température correspond à la température d'inversion de phase de cette composition. L'émulsion est alors sous une forme d'émulsion inverse (émulsion E/H)
On a cessé ensuite le chauffage mais on a maintenu l'agitation jusqu'à ce que la température revienne à la température ambiante du laboratoire (≈20°C). L'émulsion redevient sous la forme d'une émulsion H/E.
On a obtenu ainsi une émulsion H/E thermoréversible, homogène dont plus de 90% de la population volumique des gouttelettes d'huile ont une taille ≤200nm et dont la composition en pourcentage massique est la suivante :

32,4% de squalène,
6,2% de ceteareth-12 (Emulgin B1),
4,9% de monooléate de sorbitane (dehymuls SMO),
5,5% de mannitol
0.026% de ER804057

La quantité de squalène dans cette émulsion adjuvante représente donc 32,4% du poids total de l'émulsion.

Dans une autre variante on a réalisé dans un bécher, un mélange contenant 50,5 g d'un tampon phosphate, 6g de mannitol, 6,18g d'Eumulgin ™ B1 et 0,026g d'ER804057. Ce mélange a été maintenu sous agitation à environ 40°C ; Dans un autre récipient, on a préparé la phase huileuse en mélangeant sous agitation magnétique 32,5g de squalène avec 4,8g de deshymuls SMO jusqu'à dissolution complète du deshymuls SMO. Lorsque les phases homogènes ont été obtenues, l'incorporation de la phase aqueuse à la phase huileuse, les étapes de montée en température suivie de l'étape de descente en température ont été réalisées comme précédemment. On a obtenu une émulsion H/E thermoréversible, homogène dont plus de 90% de la population volumique des gouttelettes d'huile ont une taille ≤200nm et dont la composition en pourcentage massique est la suivante :

32,5% de squalène,
6,2% de ceteareth-12 (Emulgin B1),
4,8% de monooléate de sorbitane (dehymuls SMO),
6% de mannitol
0.026% de ER804057
50,5% de PBS

Dans une autre variante du procédé, on a utilisé, à la place du tampon PBS, un tampon citrate pH 6,04 préparé en mélangeant 0,83mM d'acide citrique monohydraté avec 9,14 mM de citrate de sodium.

Cette émulsion concentrée en squalène a été utilisée en tant qu'émulsion « mère », à partir de laquelle on a dérivé des émulsions H/E diluées thermoréversibles par dilution dans un tampon phosphate, dans un tampon Tris, ou dans un tampon citrate puis que l'on a stérilisées par filtration (voir exemple II). Ces émulsions H/E diluées thermoréversibles sont ensuite mélangées avec un ou plusieurs antigènes vaccinaux (voir exemples III, IV, et V).

Exemple II : Etude de la stabilité d'une émulsion H/E thermoréversible diluée à 5% de squalène (p/p)

On a dilué l'émulsion concentrée de l'exemple 1 dans un tampon phosphate 9,6 mM (pH=7,4) pour obtenir une émulsion diluée dont la quantité de squalène représente 5% du poids total de l'émulsion. La composition de l'émulsion diluée, dénommée PIT-ER804057 à 5%, était la suivante :

Squalène : 50mg/ml
Ceteareth-12 (Emulgin B1): 9,5 mg/ml
Monooléate de sorbitane (dehymuls SMO): 7,4 mg/ml

Mannitol : 9 mg/ml
ER804057 : 40 µg/ml

On a évalué la stabilité de cette émulsion thermoréversible après une conservation de 6 mois à une température de +4°C en contrôlant la teneur en ER804057 dans l'émulsion et la distribution de taille de l'émulsion. Pour doser ER804057 on a procédé à une extraction sélective de ER804057 de l'émulsion suivie d'une analyse par chromatographie liquide haute performance (HPLC) couplée à un détecteur à barrette de diodes (détection UV). On a déterminé la teneur en ER804057 de l'émulsion à contrôler à partir d'une gamme d'étalonnage renfermant entre 5 et 25 µg/ml de ER804057. Pour pallier les variations des rendements d'extraction, on a introduit dans chaque échantillon à doser (y compris dans les échantillons de la gamme d'étalonnage) une quantité constante d'un standard interne dont la structure chimique est très proche de celle de ER804057. Il s'agit de la molécule chimique dénommée ER803022.
La gamme d'étalonnage a été réalisée à partir d'une émulsion thermoréversible qui a la même composition et préparée de la même façon que l'émulsion PIT-ER804057 à 5% (cf exemple II) hormis le fait qu'elle ne contenait pas de ER804057 (émulsion PIT à 5%), à laquelle on a rajouté une quantité variable de ER804057 prélevée d'une solution stock de ER804057 à 0,1mg/ml d'un mélange contenant 2 volumes de chloroforme pour 1 volume de méthanol (mélange CM 2 :1), une quantité fixe d'un standard interne (10µg) prélevée d'une solution stock d'un standard interne à 0,1mg/ml d'un mélange CM 2:1, que l'on a dilué à convenance dans de l'eau pour préparation injectable (EPPI)

L'échantillon de PIT-ER804057 à 5% à doser, a été préparé en prélevant une aliquote de l'émulsion PIT-ER804057 à 5% auquel on a rajouté 10µg de standard interne et que l'on a dilué dans de l'EPPI.

L'extraction de ER804057 à partir des échantillons de la gamme d'étalonnage ou des échantillons de PIT-ER804057 à 5% a été réalisée de la façon suivante: On a solubilisé l'échantillon par du CM 2 :1. Le système biphasique obtenu est composé d'une phase chloroformique contenant majoritairement ER804057 et d'une phase aqueuse contenant les autres composés de l'émulsion. On a récupéré la phase chloroformique que l'on a évaporé à chaud sous un courant d'azote. L'extrait sec obtenu, a été repris et solubilisé à nouveau dans le mélange CM 2 :1. On a déposé le mélange sur une cartouche échangeuse d'anions préalablement équilibrée dans le mélange CM 2 :1. Elle a retenu sélectivement ER804057 et le standard interne qui sont chargés négativement tandis que les autres composants de l'émulsion, non chargés, ont été éliminés. On a élué ER804057 et le standard interne au moyen d'un mélange contenant 2 volumes de chloroforme, 3 volumes de méthanol, pour 1 volume de NaCl 1M. L'éluat a ensuite été séché à chaud sous un courant d'azote. On a réalisé enfin une dernière extraction en eau et CM 2 :1 pour éliminer les sels résiduels et récupérer ER804057 ainsi que le standard interne dans la phase chloroformique qui a finalement été évaporée à chaud sous un courant d'azote. L'extrait sec issu de chaque échantillon a été conservé à -20°C avant d'être analysé par HPLC.

L'extrait sec de chaque échantillon a été repris par 50µl de CM 4 :1, puis dilué au 1/2 dans le méthanol, puis au 1/10$^{e}$ dans le mélange acétonitrile 30%-EPPI. On a injecté 20µl de la dilution dans un appareil à chromatographie liquide (HPLC Merck Hitachi, Lachrom serie 7000) comprenant une colonne Waters XTerra™ RP8 préalablement équilibrée dans une phase mobile constituée d'un mélange à 80% de la phase A (eau PPI/éthanol 50/50 contenant 2% de $H_3PO_4$) et 20% de la phase B (ethanol contenant 2% de $H_3PO_4$).On a élué ER804057 et le standard interne à l'aide d'un gradient d'ethanol à 2% de $H_3PO_4$. En sortie d'HPLC, l'éluat est arrivé au niveau du détecteur à barrette de diodes et les molécules ont été détectées à la longueur d'onde de 215 nm. Sur le chromatogramme obtenu les surfaces des 2 pics (analyte et référent) ont été intégrées et corrélées: Pour corriger les variations liées à la préparation de l'échantillon, la courbe d'étalonnage a été établie entre le ratio des surfaces des pics correspondant au couple ER804057 (molécule quantifiée) et ER803022 (standard interne) et le ratio des concentrations correspondant à ER 804057 et ER803022 (standard interne). Une fois la courbe établie, on a déterminé la quantité de ER804057 présente dans l'émulsion PIT-ER804057 à 5% par mesure du ratio des surfaces des pics ER804057/standard interne et comparaison à la courbe étalon.

| | 1 mois à +4°C | 3 mois à +4°C | 6 mois à +4°C |
|---|---|---|---|
| ER 804057<br>(concentration théorique 40µg/ml) | 38 µg/ml | 37 µg/ml | 42 µg/ml |

Les résultats mentionnés dans le tableau ci dessus montrent que ER804057 conserve son intégrité structurale et que sa concentration dans l'émulsion PIT-ER804057 à 5% n'a pas sensiblement varié après avoir conservé l'émulsion pendant 6 mois à + 4°C.

Les analyses de distribution de taille ont été réalisées après dilution de l'émulsion au 1/100$^{ème}$ avec le Mastersizer 2000, en utilisant les paramètres suivants : IR particule=1.495 ; IR milieu=1.332 ; valeur d'absorption=0 ; limite d'obscuration basse= 4% ; limite d'obscuration haute=7%; modèle d'analyse « général purpose ». Pour chaque analyse de distribution de taille, on a évalué les paramètres suivants : d10, d50, et d90 qui représentent respectivement les

valeurs des diamètres moyens de particule en dessous desquels se trouvent respectivement 10%, 50% et 90% de la population volumique des gouttelettes d'huile.

| | T= 0 | T= 3 mois | T= 6 mois |
|---|---|---|---|
| d10 | 71 nm | 70 nm | 70 nm |
| d50 | 103 nm | 100 nm | 101 nm |
| d90 | 155 nm | 152 nm | 153 nm |

Ces résultats montrent que la distribution de taille de l'émulsion est stable à +4°C sur une période de temps d'au moins 6 mois.

<u>Exemple III: Composition vaccinale contre les infections à cytomégalovirus préparée à partir d'une émulsion H/E selon l'invention</u>

On a préparé des compositions vaccinales comprenant à titre d'antigène vaccinal une protéine recombinante qui dérive de la glycoprotéine gB du CMV. Cette protéine recombinante a été produite par une lignée CHO recombinante transfectée par un plasmide dénommé pPRgB27clv4 qui contient un gène modifié de la gB. Pour faciliter la production de cette protéine recombinante par la lignée CHO le gène de la gB dont la séquence est décrite dans US 5,834,307 a été modifié au préalable en supprimant la partie du gène qui code pour la région transmembranaire de la protéine gB correspondant à la séquence d'acides aminés comprise entre la Valine 677 et l'Arginine 752 et en introduisant 3 mutations ponctuelles au niveau du site de clivage. La protéine produite par la lignée CHO, dénommée gBdTM correspond à une protéine gB tronquée dépourvue de site de clivage et de région transmembranaire.

La construction du plasmide pPRgB27clv4 et la production de la protéine gB tronquée (gBdTM) par la lignée CHO recombinante sont décrites dans US 6,100,064. La protéine gBdTM produite dans le milieu de culture est ensuite purifiée par chromatographie d'affinité en utilisant l'anticorps monoclonal 15D8 décrit par Rasmussen L et al. (J. Virol. (1985) 55 : 274-280). La protéine purifiée a été stockée sous la forme d'une solution stock à 0,975 mg/ml de gBdTM en tampon phosphate.

On a préparé des compositions immunostimulantes de gBdTM formulées avec différentes compositions d'émulsions H/E ou avec une suspension d'hydroxyde d'aluminium.

La composition n°1 renfermait 2 µg de gBdTM en tampon citrate à pH 6 sous 50 µl (groupe gB).

La composition n°2 renfermait 2 µg de gBdTM, 1,075 mg de squalène, 0,133 mg de Trioléate de sorbitane (Montane™ VG 85) et 0,125 mg de Tween™80 en tampon citrate à pH 6 sous 50 µl (groupe gB+émulsion H/E). Cette composition a été obtenue en mélangeant volume à volume une solution de gB avec une émulsion H/E de l'art antérieur que l'on a obtenue par microfluidisation.

La composition n°3 renfermait 2 µg de gBdTM et 60µg d'hydroxyde d'aluminium en tampon phosphate sous 50µl (groupe gB+AL)

La composition n°4 renfermait 2 µg de gB, 1,25 mg de squalène, 0,187 mg de Dehymuls™ SMO, 0,237 mg d'Eumulgin™ B1 et 0,225 mg de mannitol en tampon PBS à pH 7,4 sous 50µl. Cette composition a été obtenue en mélangeant volume à volume une solution de gB avec une émulsion H/E thermoréversible à 5% de squalène (Groupe gB+PIT). L'émulsion H/E thermoréversible servant à la préparation de cette composition a été obtenue par dilution d'une émulsion H/E thermoréversible concentrée à 32,4% de squalène (p/p) qui a été préparée en utilisant le même procédé que celui de l'exemple 1 hormis le fait que la phase aqueuse ne contenait pas de ER804057.

La composition n°5 renfermait 2µg de gBdTM, 1µg de ER804057, dans un tampon citrate pH 6 sous 50µl (groupe gB+ER804057).

La composition n°6 renfermait 2 µg de gBdTM, 1,25 mg de squalène, 0,145 mg de Montane™ VG 85, 0,147 mg de Tween™80, 1 µg de ER804057 en tampon citrate à pH 6 sous 50 µl (groupe gB+émulsion H/E+ER804057). Cette composition a été obtenue en mélangeant volume à volume une solution de gB avec une émulsion H/E de l'art antérieur obtenue par microfluidisation à laquelle on a ajouté ER804057.

La composition n°7 renfermait 2 µg de gBdTM, 1 µg de ER804057, 60 µg d'hydroxyde d'aluminium dans un tampon phosphate sous 50 µl (groupe gB+A1+ER804057).

La composition n°8 renfermait 2 µg de gB, 1,25 mg de squalène, 0,189 mg de Dehymuls™ SMO, 0,240 mg d'Eumulgin™ B1 et 0,211 mg de mannitol et 1 µg de ER804057 en tampon PBS à pH 7,4 sous 50 µl. Cette composition a été obtenue en mélangeant volume à volume une solution de gB avec une émulsion H/E thermoréversible PIT-ER804057 à 5% de squalène obtenue par dilution de l'émulsion mère de l'exemple 1 (Groupe gB+PIT/ER804057).

On a immunisé 8 groupes de 10 souris non consanguines OF1 femelles, âgées de 8 semaines par voie sous-cutanée, aux jours J0 et J21, avec les compositions indiquées ci-dessus (chaque groupe de souris a reçu 2 injections de la même composition).
On a prélevé à J20 et J34 au sinus retro-orbital des échantillons sanguins qui ont été utilisés pour déterminer les concentrations en anticorps en IgGl et IgG2a spécifiques de gBdTM. Ces dosages ont été réalisés par ELISA en sensibilisant pendant une nuit à +4°C les puits de microplaques de 96-puits Dynex avec 100 ng (100 μl) de gBdTM en solution de tampon carbonate 0,05 M à pH 9,6. Pour la détermination des anticorps neutralisants on a utilisé le protocole décrit par Gonczol E. et al. dans J. Virological Methods, 14: 37-41 (1986).
On a utilisé des cellules MRC5 cultivées dans un milieu MEM contenant 10% de sérum de veau foetal, entre les passages 28 à 38, pour les analyses de microneutralisation. La souche CMV Towne (Wistar Institute, Philadelphie, US) purifiée et propagée sur cellules MRC5, ayant un titre d'environ 2 x $10^6$ PFU/ml, a servi de souche d'infection. On a utilisé également une source de complément obtenu à partir des sérums de souris de l'institut Virion Ltd (Suisse). Un mélange de sérums humains ayant un titre au 1:128 a été utilisé comme contrôle positif, et a été inclus dans chaque test de microneutralisation.
On a inactivé les sérums à tester par chauffage à 56°C pendant 30 minutes. On a ajouté à une aliquote de 15 μl de chaque sérum inactivé, 105 μl de milieu de culture (MEM + 10% de sérum de veau foetal) dans des plaques de culture de 96 puits à fond plat (dilution au 1/8). Puis on a réalisé des dilutions successives de raison 2. Les sérums de contrôle ont été testés de la même façon. 60 μl de suspension de virus contenant 3000 PFU et 5 μl de complément de souris ont été ajoutés dans chaque puits. Après une incubation de 1 heure à 37°C sous $CO_2$, on a ajouté 3-4x$10^4$ cellules MRC5 dans un volume de 150 μl de milieu de culture dans chacun des puits. Les microcultures ont été cultivées pendant 4 jours. L'activité cytopathique du virus était de 100% dans les puits qui ne contenaient pas de sérums. Par contre, on a observé une inhibition de l'activité cytopathique du virus dans les puits qui renfermaient des sérums neutralisants. Le titre en anticorps neutralisants d'un sérum correspond à l'inverse de sa dilution qui inhibe à plus de 90% l'activité cytopathique du virus.

Les résultats qui ont été obtenus pour chaque groupe de souris sont représentés dans les tableaux ci après :

| Groupe de souris | IgG1 à J20 | IgG2a à J20 | IgG1 à J34 | IgG2a à J34 | ratio à J34 IgG1/IgG2a |
|---|---|---|---|---|---|
| Groupe gB | 2,47* | 2,09 | 3,80 | 2,94 | 137 |
| Groupe gB+émulsion H/E | 4,06 | 2,98 | 5,49 | 4,18 | 143 |
| Groupe gB+AL | 3,06 | 1,85 | 4,90 | 3,33 | 357 |
| Groupe gB+PIT | 4,61 | 3,91 | 5,61 | 4,85 | 14 |
| Groupe gB+ER804057 | 3,09 | 3,12 | 4,43 | 4,16 | 6 |
| Groupe gB+PIT/ER804057 | 4,78 | 4,58 | 5,83 | 5,74 | 3 |
| * titre moyen des dilutions de sérums (exprimées en $log_{10}$) | | | | | |

Ces résultats montrent que l'émulsion PIT/ER804057 a un pouvoir immunostimulant plus important que les autres adjuvants puisque les taux d'IgGl et d'IgG2a spécifiques obtenus dans le groupe de souris « gB+PIT/ER804057 » sont significativement plus élevés que ceux obtenus dans les groupes de souris « gB+AL » ou « gB+émulsion H/E ». Le pouvoir immunostimulant de l'émulsion selon l'invention n'est pas dû uniquement à l'émulsion thermoréversible (émulsion PIT) ou à l'agoniste du TLA4 mais à la combinaison des deux produits. Les taux d'IgGl et IgG2a spécifiques observés dans les groupes «gB+PIT» et « gB+ER804057 » sont en effet significativement plus faibles que ceux qui sont observés dans le groupe « gB+PIT/ER804057 ».

Tableau récapitulatif de la production d'anticorps neutralisants

| Groupe de souris | Titre moyen en anticorps neutralisants |
|---|---|
| Groupe gB | 16** |
| Groupe gB+émulsion H/E | 32 |
| Groupe gB+AL | 32 |
| Groupe gB+PIT | 48 |
| Groupe gB+ER804057 | 16 |
| Groupe gB+émulsion H/E+ER804057 | 32 |

(suite)

| Groupe de souris | Titre moyen en anticorps neutralisants |
|---|---|
| Groupe gB+AL+ER804057 | 32 |
| Groupe gB+PIT/ER804057 | 128 |
| ** : inverse de la moyenne des dilutions sériques inhibant à plus de 90% l'effet cytopathique du virus. | |

Ces résultats montrent que la composition immunostimulante résultant du mélange d'un antigène d'enveloppe du CMV avec une émulsion H/E thermoréversible renfermant un agoniste du TLR4 telle que décrite dans l'invention est celle qui induit le plus fort taux d'anticorps neutralisants chez la souris. L'émulsion PIT/ER804057 a une plus grande capacité à stimuler la production d'anticorps neutralisants que les autres compositions adjuvantes testées. L'émulsion PIT/ER804057 s'avère plus performante (pour sa capacité à stimuler la production d'anticorps neutralisants) qu'une émulsion H/E de l'art antérieur à base de squalène, renfermant les mêmes composants que l'émulsion MF59, considérée jusqu'à présent comme l'adjuvant de référence pour l'adjuvantation des protéines du CMV. On constate aussi que l'ajout d'un agoniste du TLR4 à l'émulsion H/E de l'art antérieur n'augmente pas la performance de cette émulsion (le titre en anticorps neutralisants reste le même) alors que la performance d'une émulsion thermoréversible (PIT) s'accroît lorsqu'elle contient un agoniste du TLR4 (le titre en anticorps neutralisants augmente).

Exemple IV: Composition vaccinale contre la grippe préparée à partir d'une émulsion H/E selon l'invention

On a préparé des compositions immunostimulantes à partir d'une composition vaccinale antigrippale comprenant les 3 souches vaccinales de la campagne 2004 (la souche A /New Caledonia (H1N1), la souche A/Wyoming (H3N2), et la souche B/Jiangsu que l'on formule avec différentes compositions d'émulsions H/E ou avec une suspension d'hydroxyde d'aluminium.
La composition n°1 renfermait 0,3 μg d'hémagglutinine (HA) de chacune des souches virales en tampon PBS sous 30 μl. (groupe 0,3 μg HA)
La composition n°2 renfermait 6,3 μg d'hémagglutinine (HA) de chacune des souches virales en tampon PBS sous 30 μl. (groupe 6,3 μg HA)
La composition n°3 renfermait 0,3 μg d'hémagglutinine (HA) de chacune des souches virales 0,65 mg de squalène, 0,075 mg de trioléate de sorbitane (Span™ 85) et 0,075 mg de Tween™80 en tampon PBS sous 30 μl (groupe 0,3 μg HA+émulsion H/E). Cette composition a été obtenue en mélangeant la composition vaccinale antigrippale avec une émulsion H/E de l'art antérieur obtenue par microfluidisation.
La composition n°4 renfermait 0,3 μg d'hémagglutinine (HA) de chacune des souches virales, 0,75 mg de squalène, 0,11 mg de Dehymuls™ SMO, 0,143 mg d'Eumulgin™ B1 et 0,138 mg de mannitol et 0,6 μg de ER804057 en tampon PBS à pH 7,4 sous 30 μl (Groupe 0,3 μg HA+PIT/ER804057). Cette composition a été obtenue en mélangeant la composition vaccinale antigrippale avec l'émulsion thermoinversible telle que décrite dans l'exemple 1 et que l'on a préalablement dilué en tampon PBS.

On a immunisé 4 groupes de 8 souris BALB/c femelles, âgées de 8 semaines, à qui on a administré à J0 par voie intradermique (face interne de l'oreille) une dose de 30 μl de l'une des compositions immunostimulantes indiquées ci-dessus.
On a prélevé à J21 au sinus retro-orbital des échantillons sanguins qui ont été utilisés pour déterminer les taux d'anticorps neutralisants spécifiques de chaque souche virale (anticorps inhibant l'hémagglutination (IHA)) obtenus dans chaque groupe de souris immunisées. Le principe de ce dosage est basé sur la capacité des virus grippaux à agglutiner les globules rouges tandis qu'un sérum qui contient des anticorps neutralisants dirigés spécifiquement contre la HA du virus inhibe l'activité « hémagglutinante » du virus. On a éliminé dans un premier temps les inhibiteurs non spécifiques contenus dans les sérums en les traitant avec une enzyme RDE (Receptor destroying enzyme) fournie par Sigma puis en les mettant en contact avec une solution de globules rouges de poulet à 10%. On a obtenu un surnageant débarrassé d'inhibiteurs non spécifiques et qui correspondait à un sérum dilué au 1/10$^{e}$. On a réalisé ensuite des dilutions successives de raison 2 du surnageant en tampon phosphate puis on a déposé 50μl de chacune des dilutions dans les puits d'une microplaque en V. On a ajouté dans chaque puits 50μl d'une suspension virale provenant d'un liquide allantoïque clarifié et titrant 4 unités hémagglutinantes (4HAU). On a laissé incuber pendant 1 heure à la température du laboratoire avant d'ajouter 50μl d'une solution de globules rouges de poulet ou de dinde dans chacun des puits. On a laissé reposer pendant 1 heure à +4°C avant d'effectuer la lecture du test. La présence d'une inhibition d'hémagglutination se traduisait par la présence d'un point rouge au fond du micropuits tandis que la présence d'une hémagglutination se traduisait par la présence d'un halo rosé dans le micropuits. Le titre en anticorps IHA est représenté par l'inverse de la dernière dilution

où l'on n'observe pas d'hémagglutination dans le micropuits.

Les résultats qui ont été obtenus sont regroupés dans le tableau ci dessous :

| Groupe de souris | IHA contre A /New Caledonia (H1N1) | IHA contre A/Wyoming (H3N2) | IHA contre B/Jiangsu |
|---|---|---|---|
| 0,3 µg HA | 26*** | 174 | 8 |
| 6,3 µg HA | 247 | 907 | 73 |
| 0,3 µg HA+émulsion H/E | 135 | 987 | 57 |
| 0,3 µg HA+PIT+ER804057 | 290 | 1522 | 98 |
| *** moyenne des titres IHA obtenus sur les 8 sérums de chaque groupe de souris. | | | |

Ces résultats montrent que la composition vaccinale obtenue en mélangeant un vaccin antigrippal avec une émulsion H/E thermoréversible renfermant un agoniste du TLR4 est celle qui induit le plus fort taux d'anticorps neutralisants chez la souris quelle que soit la souche vaccinale testée comparativement aux autres compositions vaccinales. L'émulsion PIT/ER804057 s'avère même légèrement plus performante (pour sa capacité à stimuler la production d'anticorps neutralisants) qu'une émulsion H/E de l'art antérieur dont la composition est similaire à MF59. L'intérêt de cette émulsion réside aussi en ce que l'on peut réduire fortement les quantités d'antigène puisque les résultats obtenus avec une dose de 0,3 µg d'hémagglutinine mélangée avec une émulsion PIT/ER804057 sont meilleurs que ceux que l'on obtient avec une dose 20 fois plus forte d'hémagglutinine.

Dans un autre test, on a suivi l'évolution du taux des anticorps inhibant l'hémagglutination au cours du temps dans des groupes de souris immunisées avec différentes compositions immunostimulantes préparées à partir du même vaccin de la campagne 2004.

La composition n°1 renfermait 0,3 µg d'hémagglutinine (HA) de chacune des souches virales en tampon PBS sous 30 µl. (groupe 0,3 µg HA).

La composition n°2 renfermait 6,3 µg d'hémagglutinine (HA) de chacune des souches virales en tampon PBS sous 30 µl. (groupe 6,3 µg HA).

La composition n°3 renfermait 0,3 µg d'hémagglutinine (HA) de chacune des souches virales, 0,6 µg de ER804057 dans un tampon aqueux sous 30 µl (groupe 0,3 µg HA+ER804057).

La composition n°4 renfermait 0,3 µg d'hémagglutinine (HA) de chacune des souches virales, 0,30 mg de squalène, 0,044 mg de Dehymuls™ SMO, 0,057 mg d'Eumulgin™ B1, 0,055 mg de mannitol en tampon PBS à pH 7,4 sous 30 µl (Groupe 0,3 µg HA+PIT à 1%)

La composition n°5 renfermait 0,3 µg d'hémagglutinine (HA) de chacune des souches virales, 0,30 mg de squalène, 0,044 mg de Dehymuls™ SMO, 0,057 mg d'Eumulgin™ B1, 0,055 mg de mannitol et 0,6 µg de ER804057 en tampon PBS à pH 7,4 sous 30 µl (Groupe 0,3µg HA+PIT à 1%/ER804057).

On a immunisé 5 groupes de 5 souris BALB/c femelles, âgées de 8 semaines, à qui on a administré à J0 par voie intradermique (face interne de l'oreille) une dose de 30 µl de l'une des compositions immunostimulantes indiquées ci-dessus.

On a prélevé au sinus rétro-orbital des échantillons sanguins à J23, J51 et J79 qui ont été utilisés pour déterminer les taux d'anticorps neutralisants spécifiques de la souche H1N1 (anticorps inhibant l'hémagglutination (IHA)) obtenus dans chaque groupe de souris immunisées. Les résultats qui ont été obtenus sont regroupés dans le tableau ci-dessous :

| Groupe de souris | J23 | J51 | J79 |
|---|---|---|---|
| groupe 0,3µg HA | 35*** | 53 | 80 |
| groupe 6,3 µg HA | 235 | 243 | 279 |
| groupe 0,3µg HA+ER804057 | 65 | 211 | 243 |
| groupe 0,3µg HA+PIT à 1% | 226 | 557 | 735 |
| Groupe 0,3µg HA+PIT à 1%/ER804057 | 226 | 970 | 844 |
| *** moyenne des titres IHA obtenus sur les 5 sérums de chaque groupe de souris. | | | |

La performance de l'émulsion PIT-ER804057 pour sa capacité à produire des anticorps inhibant l'hémagglutination du virus grippal (anticorps protecteurs) est le résultat de l'action combinée de l'émulsion et de l'agoniste du TLR4; les performances de l'émulsion PIT seule ou de ER804057 seule sont plus faibles.

Exemple V : Composition vaccinale contre la grippe préparée à partir d'une émulsion selon l'invention testée dans une population de souris jeunes ou âgées déjà sensibilisées au virus de la grippe.

On a testé l'efficacité de l'émulsion de l'invention dans le cas d'un vaccin contre la grippe qui serait administré à des personnes déjà sensibilisées au virus de la grippe, soit parce que ces personnes ont déjà été en contact avec le virus grippal, soit parce qu'elles ont déjà été vaccinées avec un vaccin anti-grippal.

Pour faire ce type d'évaluation, on peut, d'après Potter et al (Vaccine, 2003, 21 :940-945), utiliser comme modèle animal non naïf vis-à-vis de la grippe des souris préalablement immunisées par voie intramusculaire (IM) avec un vaccin trivalent.

5 groupes de 10 souris C57B1/6 de 8-10 semaines ont reçu par voie IM une dose d'un vaccin trivalent contenant d'1,5μg d'HA de chacune des souches A/ New Caledonia/20/99 (H1N1), A/New York/55/04 (H3N2) et B/Malaysia/ 2506/04.

A J28, à l'exception d'un groupe (groupe PBS) qui a reçu une injection d'un tampon PBS, tous les autres groupes de souris ont reçu par voie intradermique, sous un volume de 30μl, différentes compositions vaccinales contenant un vaccin trivalent différent de celui qui a servi à la primo immunisation (A/ New Caledonia/20/99 (H1N1), A/Wellington/ 01/04 (H3N2) et B/Jiangsu/10/03).

Un groupe a reçu une composition renfermant 0,3 μg d'HA de chacune des souches en tampon PBS (groupe 0,3 μg HA).

Un autre groupe a reçu une composition renfermant 6,3 μg d'HA de chacune des souches en tampon PBS (groupe 6,3μg HA).

Un autre groupe a reçu une composition renfermant 0,3 μg d'HA de chacune des souches en tampon PBS dans une émulsion H/E à 1% de squalène contenant 0,3 mg de squalène, 0,044 mgde Dehymuls ™·SMO, 0,057 mg d'Eumulgin ™ B1 et 0,055 mg de mannitol en tampon PBS. Cette composition qui contenait 1% de squalène a été préparée en mélangeant le vaccin antigrippal avec une émulsion H/E obtenue par dilution d'une solution mère thermoréversible concentrée préparée selon le même procédé que celui décrit dans l'exemple 1 hormis le fait que la phase aqueuse ne contenait pas de ER804057 (groupe 0,3 μg HA +PIT 1 %).

Enfin, le dernier groupe a reçu une composition renfermant 0,3μg d'HA de chacune des souches en tampon PBS dans une émulsion H/E à 1% de squalène contenant 0,3 mg de squalène, 0,044mgde Dehymuls ™·SMO, 0,057 mg d'Eumulgin ™ B1 et 0,055 mg de mannitol en tampon PBS et 0,6μg d'ER804057. Cette composition qui contenait 1% de squalène et 0,6μg d'ER804057 a été préparée en mélangeant le vaccin antigrippal avec une émulsion H/E obtenue par dilution d'une solution mère thermoréversible concentrée préparée selon le même procédé que celui décrit dans l'exemple 1 (groupe 0,3μg HA+PIT 1%/ER 804057)

A J50, les souris ont été euthanasiées pour recueillir un échantillon sanguin et réaliser un prélèvement de rate. Sur chaque prélèvement de sang on a réalisé un dosage des IHA vis-à-vis des souches A/ New Caledonia/20/99 (H1N1), A/Wellington/01/04 (H3N2) et B/Jiangsu/10/03. Les résultats qui ont été obtenus sont regroupés dans le tableau ci-dessous :

| Groupe de souris | IHA contre A/New Caledonia (H1N1) | IHA contre A/Wellington (H3N2) | IHA contre B/Jiangsu |
|---|---|---|---|
| Groupe PBS | 52* | 48 | 13 |
| Groupe 0,3μg HA | 95 | 226 | 57 |
| Groupe 6,3μg HA | 190 | 640 | 226 |
| groupe 0,3 μg HA +PIT 1% | 431 | 640 | 290 |
| groupe 0,3μg HA+PIT 1%/ER 804057 | 698 | 2348 | 640 |
| * représente la valeur moyenne des titres IHA obtenus sur les 10 sérums de chaque groupe de souris | | | |

Ces résultats montrent que le taux d'IHA moyen dans le groupe de souris immunisées avec la composition vaccinale « HA +PIT 1%/ER804057 » faiblement dosée en squalène (1%) et en agoniste du TLR4 (elle contient 0,6μg d'ER804057), est significativement plus élevé que les titres obtenus après immunisation des souris avec le vaccin antigrippal non adjuvé à dose équivalente en HA (groupe 0,3μg HA) ou à dose 20 fois plus forte en HA (groupe 6,3μg

HA). Ces résultats montrent l'intérêt de l'émulsion PIT 1%/ER 804057, même au sein d'une population déjà sensibilisée au virus de la grippe, puisqu'on peut réduire d'un facteur 20 la quantité d'antigène grippal de chaque souche, tout en obtenant des taux d'anticorps protecteurs plus importants vis-à-vis des 3 souches de virus.

Sur chaque prélèvement de rate on a analysé la réponse immune cellulaire spécifique en utilisant la technique ELISPOT et la technique CBA(Cytometric Bead Array) pour le dosage de l'interferon γ et de l'IL5 produits par les splénocytes après stimulation spécifique.

En ce qui concerne la technique ELISPOT, on a déposé $2x10^5$ splénocytes sous 200μl d'un milieu de culture (RPMI 1640, 10% de sérum de veau foetal, glutamine 2mM, β Mercaptoéthanol 50mM) dans les puits de microplaques de nitocellulose préalablement sensibilisées avec un anticorps de rat anti INFγ de souris (Pharmingen ref : 551216) ou avec un anticorps de rat anti IL5 de souris (Pharmingen erf : 554393). Les splénocytes ont été incubés pendant 1 nuit à 37°C en présence d'IL-2 murine (Bohringer) (10U/ml) et de différents antigènes grippaux à une concentration de 1 μg/ml. Pour l'analyse de la réponse cellulaire CD8+, on a utilisé un peptide NP de la grippe ( TYQRTRALV) reconnu dans le contexte H-2kd. Pour l'analyse de la réponse cellulaire CD4+ l'antigène grippal est représenté par le vaccin trivalent inactivé et splitté contenant les souches A/ New Caledonia/20/99 (H1N1), A/Wellington/01/04 (H3N2) et B/ Jiangsu/10/03. Les microplaques ont ensuite été lavées et les splénocytes qui ont sécrétés de l'IFNγ ou de l'IL5 ont été détectés au moyen d'un anticorps de rat biotinylé anti INFγ de souris (Pharmingen ref: 554410) ou d'un anticorps de rat biotinylé anti IL5 de souris (Pharmingen ref : 554393) et au moyen de la streptavidine conjuguée à la peroxydase (Southern Biotechnology-ref 7100-05) ; Après révélation à l'aide du 3 Amino-9 éthyl-carbazole les spots correspondant aux splénocytes qui sécrétent de l'INFγ ou de l'IL5 ont été comptabilisés au moyen d'un lecteur ELISPOT automatique. Les résultats ont été exprimés en nombre de cellules sécrétant de l'INFγ ou de l'IL5 pour $10^6$ splénocytes. Le seuil positif de détection est de 20 spots pour $10^6$ splénocytes.

En ce qui concerne la technique CBA, on a déposé dans les puits de micoplaques de culture $4x10^5$ splénocytes sous 200μl d'un milieu de culture (RPMI 1640, 10% de sérum de veau foetal, glutamine 2mM, β Mercaptoéthanol 50mM). Les splénocytes ont été incubés pendant 5 jours à 37°C en présence du vaccin trivalent (à 1 μg/ml) ou en l'absence d'agent stimulant pour évaluer la production non spécifique de cytokine (témoin milieu). Le contenu en INFγ ou en IL5 des surnageants de culture a ensuite été dosé par cytométrie de flux en utilisant le kit CBA mouse Th1/Th2 (Becton Dickinson - ref :551287). Le seuil positif de détection était de 2,5 pg/ml pour l'INFγ et de 5 pg/ml pour l'IL5. Pour chaque prélèvement de rate, la concentration spécifique en INFγ ou en IL5 a été calculée en soustrayant du résultat le taux d'INFγ ou d'IL5 qui est produit de façon non spécifique.

Les résultats des analyses de la réponse cellulaire sont regroupés dans le tableau ci-dessous :

| Groupe de souris | Nombre de splénocytes sécrétant de L'IFNγ | Nombre de splénocytes sécrétant de L'IL5 | Taux d'IFNγ dans le surnageant de culture | Taux d'IL5 dans le surnageant de culture | Ratio IFNγ/IL5 |
|---|---|---|---|---|---|
| PBS | 19* | 23* | 148** | 355** | 2,1*** |
| 0,3μg HA | 17 | 66 | 295 | 400 | 1,3 |
| 6,3μg HA | 30 | 13 | 780 | 523 | |
| 0,3 μg HA +PIT 1% | 31 | 74 | 691 | 1301 | 0,6 |
| 0,3μg HA+PIT 1%/ER 804057 | 66 | 22 | 1499 | 640 | 4,1 |

* représente la valeur moyenne du nombre de splénocytes, secrétant de l'IL5 ou de l'IFNγ par $10^6$ splénocytes après stimulation avec le vaccin trivalent; La valeur moyenne est calculée sur la base des résultats ELISPOT obtenus sur les 10 prélèvements de rate/ groupe de souris

** représente le taux moyen (pg/ml) en IL5 ou en IFNγ calculé sur la base des résultats obtenus sur les 10 prélèvements de rate/groupe de souris en utilisant la technique CBA.

*** le ratio représente la moyenne arithmétique des ratios IFNγ/IL5 dans chaque groupe. On a déterminé le ratio IFNγ/IL5 pour chaque prélèvement sur la base des valeurs des concentrations spécifiques en INFγ et en IL5 obtenues selon la méthode CBA après mise en culture des splénocytes puis on a fait la moyenne arithmétique des 10 ratios obtenus pour chaque groupe de souris.

Ces résultats montrent que l'émulsion PIT 1%/ER 804057 oriente fortement la réponse cellulaire CD4+ vers la production d'INFγ consécutivement à une restimulation spécifique des cellules avec le vaccin de la grippe. C'est dans

le groupe de souris qui a reçu cette émulsion qu'on observe la réponse Th1 1a plus prononcée (ratio IFNγ/IL5 le plus élevé). La réponse Th1 est en effet plus forte que celle qui est observée dans le groupe de souris qui a reçu un vaccin grippal 20 fois plus dosé (groupe 6,3 μg). Cette émulsion est donc recommandée dans les populations d'individus qui ont une réponse Th1 déficitaire consécutivement à une vaccination antigrippale, notamment chez les personnes âgées.

On a reproduit le même protocole expérimental en utilisant des souris C57B16 mâles, âgées de 17 mois puis on a mesuré les taux d'IHA dirigés contre A/Wellington (H3N2). Les résultats qui ont été obtenus sont regroupés dans le tableau ci-dessous :

| Groupe de souris | IHA contre A/Wellington (H3N2) |
|---|---|
| Groupe 6,3 μg HA | 104* |
| groupe 0,3 μg HA +PIT 1 % | 247 |
| groupe 0,3 μg HA+PIT 1%/ER 804057 | 476 |
| * représente la valeur moyenne des titres IHA obtenus sur les 10 sérums de chaque groupe de souris | |

Ces résultats montrent l'intérêt d'une composition vaccinale qui a été obtenue en mélangeant un vaccin antigrippal dans une émulsion H/E faiblement dosée en squalène (1%) et en agoniste du TLR4 (ER804057 à 0,6μg) pour vacciner une population d'individus âgés déjà sensibilisés au virus de la grippe puisqu'on peut réduire d'un facteur 20 la quantité d'antigène grippal de chaque souche, tout en ayant des taux d'anticorps protecteurs plus importants vis-à-vis des 3 souches de virus.

## Revendications

**1.** Une émulsion Huile dans Eau (H/E) comprenant :

i. un agoniste du TLR4, dénommé TLA4, dont la structure chimique est un composé chimique de formule I, II, III ou IV:

Composé de formule I

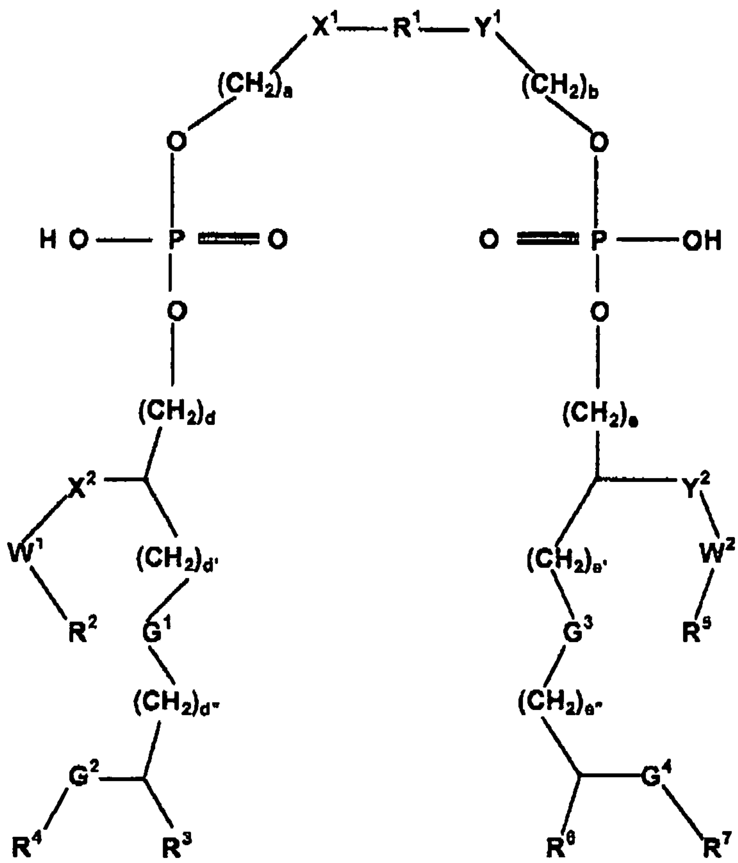

Composé de formule II

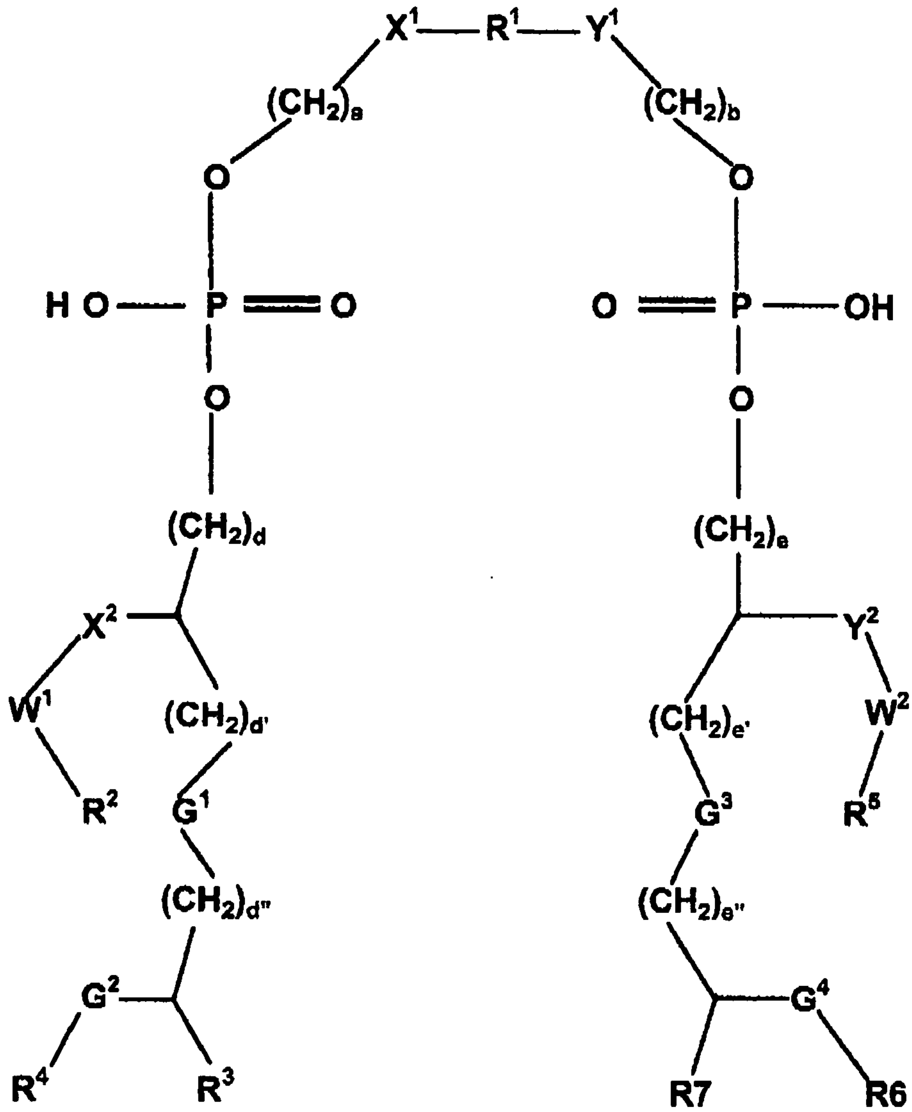

Composé de formule III

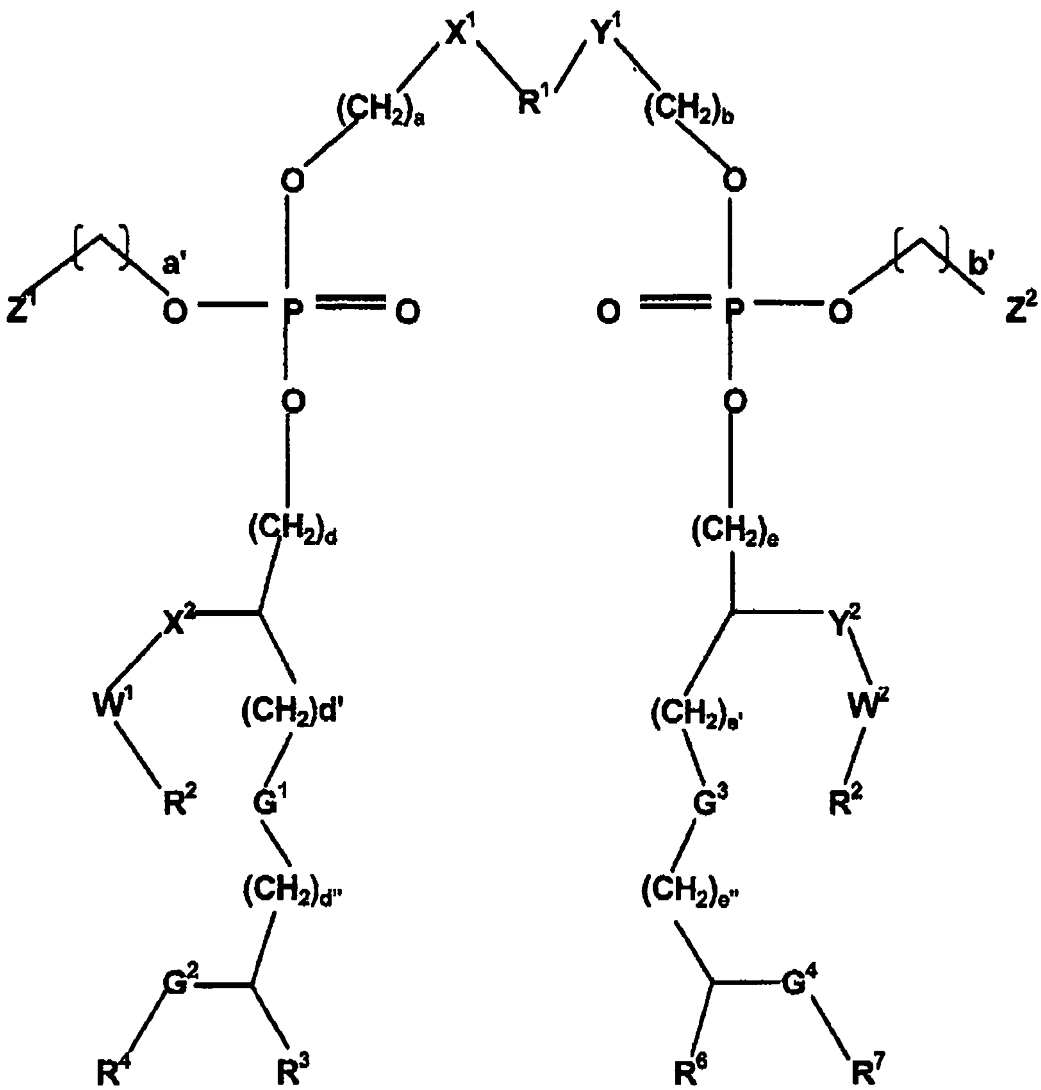

Composé de formule IV

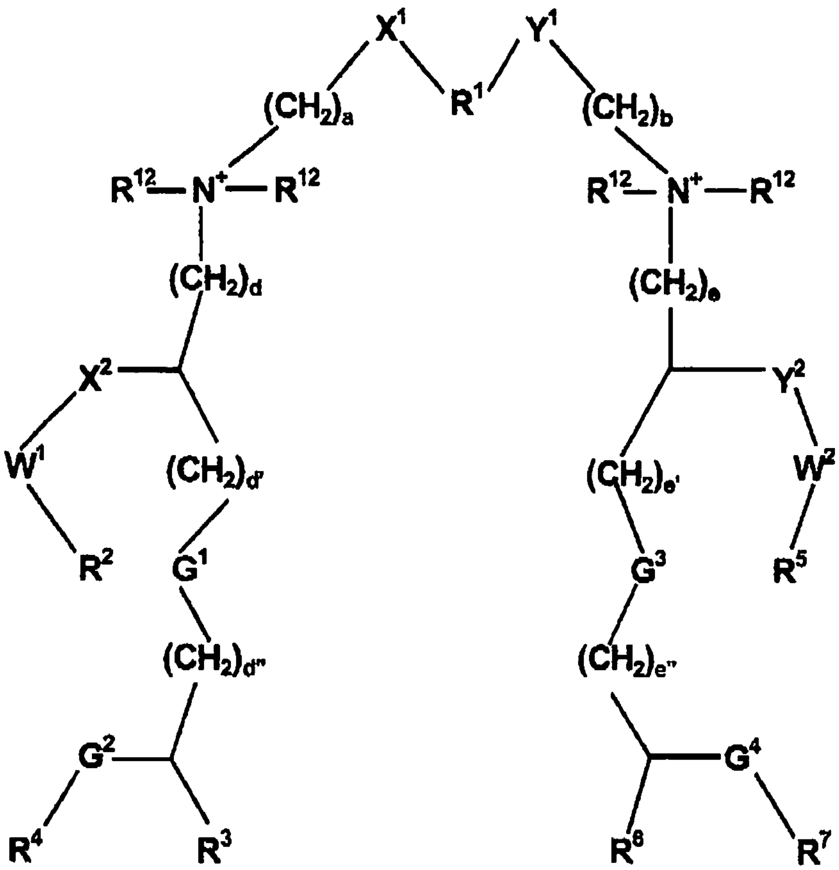

dans laquelle pour chacune des formules I, II, III, ou IV, $R^1$est sélectionné dans le groupe consistant en :

a) C(O);
b) C(O)-alkyle en $C_1$-$C_{14}$-C(O), dans lequel ledit alkyle en $C_1$-$C_{14}$ est facultativement substitué par un hydroxy, un alcoxy en $C_1$-$C_5$, un alkylènedioxy en $C_1$-$C_5$, un (alkyl en $C_1$-$C_5$)amino, ou un (alkyle en $C_1$-$C_5$)aryle, dans lequel ladite partie aryle dudit (alkyle en $C_1$-$C_5$)aryle est facultativement substituée par un alcoxy en $C_1$-$C_5$, un (alkyle en $C_1$-$C_5$) amino, un (alcoxy en $C_1$-$C_5$)amino, un (alkyle en $C_1$-$C_5$) aminoalcoxy en $C_1$-$C_5$, -O-(alkyle en $C_1$-$C_5$) aminoalcoxy en $C_1$-$C_5$, -O-(alkyle en $C_1$-$C_5$) amino-C(O) - alkyle en $C_1$-$C_5$-C(O)OH, ou -O-(alkyle en $C_1$-$C_5$)amino-C(O)-alkyle en $C_1$-$C_5$-C(O)-alkyle en $C_1$-$C_5$-;
c) un alkyle à chaîne linéaire ou ramifiée en $C_2$-$C_{15}$ facultativement substitué par un hydroxy ou un alcoxy; et
d) -C(O)-arylène en $C_6$-$C_{12}$-C(O)- dans lequel ledit arylène est facultativement substitué par un hydroxy, un halogène, un nitro ou un amino;

a et b sont indépendamment 0, 1, 2, 3 ou 4;
d, d', d", e, e' et e" sont indépendamment 0, 1, 2, 3 ou 4 ;
$X^1$, $X^2$, $Y^1$ et $Y^2$ sont indépendamment sélectionnés dans le groupe consistant en rien, un oxygène, NH, N (C(O)alkyle en $C_1$-$C_4$), et N(alkyle en $C_1$-$C_4$) ;
$W^1$ et $W^2$ sont indépendamment sélectionnés dans le groupe consistant en un carbonyle, un méthylène, un sulfone et un sulfoxyde;
$R^2$ et $R^5$ sont indépendamment sélectionnés dans le groupe consistant en :

a) un alkyle à chaîne linéaire ou ramifiée en $C_2$-$C_{20}$ qui est facultativement substitué par un oxo, un hydroxy, ou un alcoxy,
b) un alcényle ou un dialcényle à chaîne linéaire ou ramifiée en $C_2$-$C_{20}$ qui est facultativement substitué par un oxo, un hydroxy, ou un alcoxy;
c) un alcoxy à chaîne linéaire ou ramifiée en $C_2$-$C_{20}$ qui est facultativement substitué par un oxo, un hydroxy, ou un alcoxy;
d) NH-alkyle à chaîne linéaire ou ramifiée en $C_2$-$C_{20}$, dans lequel ledit groupement alkyle est facultativement substitué par un oxo, un hydroxy, ou un alcoxy; et
e)

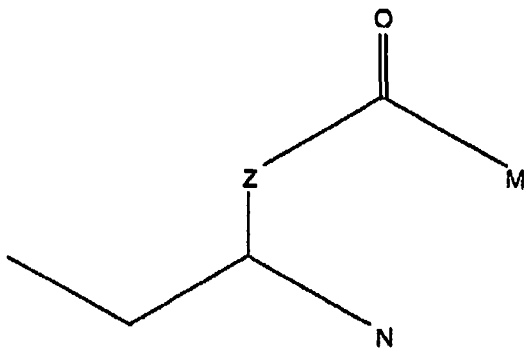

dans laquelle Z est sélectionné dans le groupe consistant en un O et NH, et M et N sont indépendamment sélectionnés dans le groupe consistant en un alkyle, un alcényle, un alcoxy, un acyloxy, un alkylamino et un acylamino à chaîne linéaire ou ramifiée en $C_2$-$C_{20}$;
$R^3$ et $R^6$ sont indépendamment sélectionnés dans le groupe consistant en un alkyle et un alcényle à chaîne linéaire ou ramifiée en $C_2$-$C_{20}$ facultativement substitué par un oxo ou un fluoro;
$R^4$ et $R^7$ sont indépendamment sélectionnés dans le groupe consistant en un C(O)-alkyle ou alcényle à chaîne linéaire ou ramifiée en $C_2$-$C_{20}$, un alkyle à chaîne linéaire ou ramifiée en $C_2$-$C_{20}$, un alcoxy à chaîne linéaire ou ramifiée en $C_2$-$C_{20}$, et un alcényle à chaîne linéaire ou ramifiée en $C_2$-$C_{20}$; dans lequel lesdits groupements alkyle, alcényle, ou alcoxy peuvent être indépendamment et facultativement substitués par un hydroxy, un fluoro ou un alcoxy en $C_1$-$C_5$;
$G_1$, $G_2$, $G_3$ et $G_4$ sont indépendamment sélectionnés dans le groupe consistant en un oxygène, un méthylène, un amino, un thiol, -C(O)NH-, -NHC(O)-, et -N(C(O)alkyle en $C_1$-$C_4$)- ;

ou $G_2R^4$ ou $G_4R^7$ peuvent ensemble être un atome d'hydrogène ou un hydroxyle ;
et dans lequel pour la formule III:

a' et b' sont indépendamment 2, 3, 4, 5, 6, 7, ou 8, de façon préférée 2 ;
$Z^1$ est sélectionné dans le groupe consistant en $-OP(O)(OH)_2$, $-P(O)(OH)_2$, $-OP(O)(OR^8)(OH)$ où $R^8$ est sélectionné dans le groupe consistant en une chaîne alkyke en $C_1$-$C_4$, $-OS(O)_2OH$, $-S(O)_2OH$, $-CO_2H$, $-OB(OH)_2$, -OH, $-CH_3$, $-NH_2$, et $-NR^9{}_3$ où $R^9$ est une chaîne alkyle en $C_1$-$C_4$;
$Z^2$ est sélectionné dans le groupe consistant en $-OP(O)(OH)_2$, $-P(O)(OH)_2$, - $OP(O)(OR^{10})(OH)$ où $R^{10}$ est sélectionné dans le groupe consistant en une chaîne alkyle en $C_1$-$C_4$, $-OS(O)_2OH$, $-S(O)_2OH$, $-CO_2H$, $-OB(OH)_2$, -OH, $-CH_3$, $-NH_2$, et $-NR^{11}$ où $R^{11}$ est une chaîne alkyle en $C_1$-$C_4$ ;

et dans lequel pour la formule IV :

R12 est H ou une chaîne alkyle en $C_1$-$C_4$ ;

ou un sel pharmaceutiquement acceptable du composé de formule chimique I, II, III, ou IV ;

ii. du squalène,
iii. un solvant aqueux,
iv. un tensioactif hydrophile non ionique qui est un polyoxyethylène alkyl éther,
v. un tensioactif hydrophobe non ionique et,

qui est thermoréversible.

2. Une émulsion selon la revendication 1, dans laquelle au moins 90% de la population volumique des gouttelettes d'huile ont une taille ≤ 200 nm.

3. Une émulsion selon la revendication 1 ou 2, dans laquelle au moins 50% de la population volumique des gouttelettes d'huile ont une taille ≤ 110 nm.

4. Une émulsion selon l'une des revendications 1 à 3, dans laquelle l'inversion de phase se produit à une température comprise entre 45°C et 80°C, et de façon préférée entre 50°C et 65°C.

5. Une émulsion selon l'une des revendications 1 à 4, comprenant en outre au moins un alditol.

6. Une émulsion selon l'une des revendications 1 à 5, dans laquelle la phase aqueuse est une solution saline tamponnée.

7. Une émulsion selon l'une des revendications 1 à 6, dans laquelle l'agoniste du TLR4 est un composé chimique de formule I:

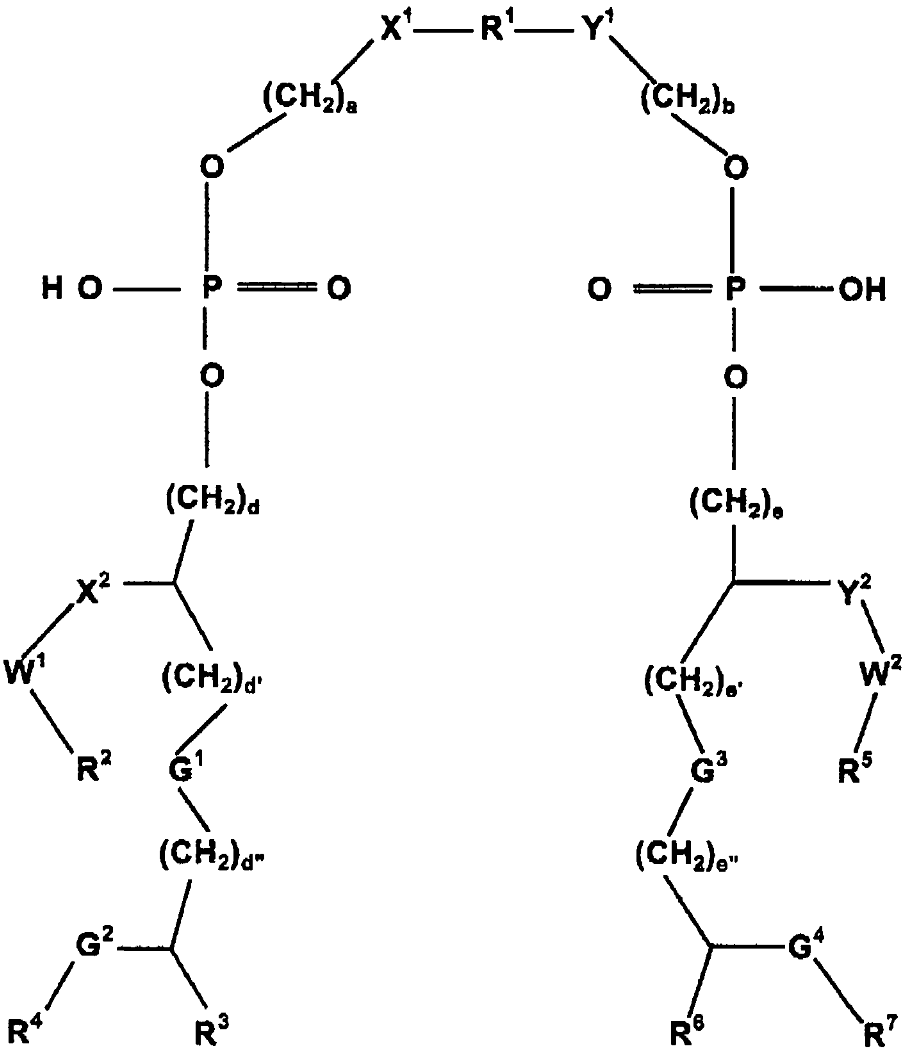

ou un sel pharmaceutiquement acceptable de ce composé.

8. Une émulsion selon la revendication 7, dans laquelle :

R1 est C(O) ou C(O)-(CH2)$_n$-C(O), n étant 1, 2, 3 ou 4,

a, b, d, d', d", e, e', e" sont indépendamment 1 ou 2,
X1, X2, Y1 et Y2 sont NH,
W1 et W2 sont C(O),
R2 et R5 sont indépendamment sélectionnés dans le groupe consistant en un alkyle à chaîne linéaire en C10-C15 facultativement substitué par un oxo, un NH-alkyle à chaîne linéaire en C10-C15 et,

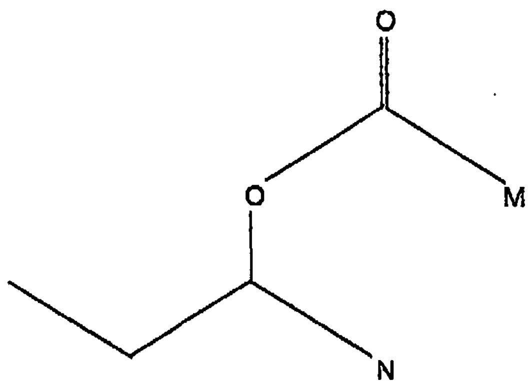

dans lequel M et N sont indépendamment un alkyle ou un alcényle à chaîne linéaire en C2-C20,
R3 et R6 sont des chaînes alkyles linéaires en C5-C10,
R4 et R7 sont sélectionnés dans le groupe consistant en un hydrogène, C(O)-alkyle à chaîne linéaire en C8-C12 et C(O) alcényle à chaîne linéaire en C8-C12,
G1 et G3 sont un oxygène ou -NH(CO)-
G2 et G4 sont un oxygène.

**9.** Une émulsion selon la revendication 7 ou 8, dans laquelle le composé chimique est choisi dans le groupe des composés chimiques constitué par le sel disodique de l'acide dodécanoïque 1,1'-[(1R,6R,22R,27R)-1,27-diheptyl-9,19-dihydroxy-9,19-dioxido-14-oxo-6,22 bis [(1-oxotetradecyl)amino]-4,8,10,18,20,24-hexaoxa-13,15-diaza-9,19-diphosphaheptacosane-1,27-diyl] ester, l'acide dodécanoïque 1,1'-[(1S,6R,24R,29S)-1,29-diheptyl-9,21-dihydroxy-9,21- dioxido-14,16-dioxo-6,24-bis [(1-oxotetradecyl)amino]-4,8,10,20,22,26-hexaoxa-13,17-diaza-9,21-diphosphanonacosane-1,29-diyl] ester, le sel disodique de l'acide dodécanoïque 1,1'-[(1R,6R,22S,27R)-1,27 diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester, l'acide dodécanoïque 1,1'-[(1R,6R,22S,27R)-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester, le sel disodique de l'acide dodécanoïque 1,1'-[(1R,6S,22S,27R)-6,22-bis[(1,3-dioxotetradecyl)amino]-1,27-diheptyl-9,19-dihydroxy-9,19-dioxido-14-oxa-4,8,10,18,20,24-hexaoxa-13,15-diaza-9,19-diphosphaheptacosane-1,27-diyl] ester, le sel disodique de l'acide dodécanoïque 1,1'-[(1R,6R,22R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, le sel disodique de l'acide 5 dodecenoïque 1,1'-[(1R,6R,24R,29R)-6,24-bis[(1,3-dioxotetradecyl)amino]-1,29-diheptyl-9,21-dihydroxy-9,21-dioxido-14,16-dioxo-4,8,10,20,22,26-hexaoxa-13,17-diaza-9,21-diphosphanonacosane-1,29-diyl] ester, le sel disodique de l'acide 5 dodecenoïque 1,1'-[(1R,6S,24S,29R)-6,24-bis[(1,3-dioxotetradecyl)amino]-1,29-diheptyl-9,21-dihydroxy-9,21-dioxido-14,16-dioxo-4,8,10,20,22,26-hexaoxa-13,17-diaza-9,21-diphosphanonacosane-1,29-diyl] ester, le sel disodique de l'acide dodécanoïque 1,1'-[(1R,6R,22R,27R)-6,22-bis[[(dodecylamino)carbonyl]amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, l'acide dodécanoïque 1,1'-[(1R,6R,24R,29R)-1,29-diheptyl-10,20-dihydroxy-10,20-dioxido-15-oxo-6,24-bis[(1-oxotetradecyl)amino]-4,9,11,19,21,26-hexaoxa-14,16-diaza-10,20-diphosphanonacosane-1,29-diyl] ester, le sel disodique de l'acide 5 dodecenoïque 1,1 '-[(1R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, le sel disodique de l'acide 5 dodecenoïque 1,1'-[(1R,32R)-6,27-bis[(1,3-dioxotetradecyl)amino]-1,32-diheptyl-9,24-dihydroxy-9,24-dioxido-14,19-dioxo-4,8,10,23,25,29-hexaoxa-13,20-diaza-9,24-diphosphadotriacontane-1,32-diyl] ester, le sel disodique de l'acide 5 dodecenoïque 1,1'-[(1R,28R)-1,28-diheptyl-8,21-dihydroxy-5,24-bis[[[(3R)-3-hydroxydecyl]oxy]methyl]-8,21-dioxido-3,13,16,26-tetraoxo-7,9,20,22-tetraoxa-4,12,17,25-tetraaza-8,21-diphosphaoctacosane-1,28-diyl] ester, le sel disodique de l'acide dodécanoïque 1,1'-[(1R,30R)-6,25-bis[(1,3-dioxotetradecyl)amino]-1,30-diheptyl-9,22-dihydroxy-9.22-dioxido-14,17-dioxo-4,8,10,21,23,27-hexaoxa-13,18-diaza-9,22-diphosphatriacontane-1,30-diyl] ester, le sel disodique de l'acide dodécanoïque 1,1'-[(1R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, le sel disodique de l'acide 5 dodecenoïque 1,1'-[(1R,29R)-6,24-bis[(1,3-dioxotetradecyl)amino]-1,29-diheptyl-9,21-dihydroxy-9,21-dioxido-14,16-dioxo-4,8,10,20,22,26-hexaoxa-13,17-diaza-9,21-diphosphanonacosane-1,29-diyl] ester, le sel disodique de l'acide 5 dodecenoïque 1, 1'-[(1R,29R)-1,29-diheptyl-9,21-dihydroxy-9,21-dioxido-14,16-dioxo-6,24-bis[(1-oxotetradecyl)amino]-4,8,10,20,22,26-hexaoxa-13,17-diaza-9,21-diphosphanonacosane-1,29-diyl] ester, le sel disodique de l'acide dodécanoïque 1,1'-[(1R,27R)-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester, et le sel disodique de l'acide 5 dodecenoïque 1,1'-[(1R, 27R)-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester.

**10.** Une émulsion selon l'une des revendications 1 à 9, dans laquelle le ratio entre la quantité d'agoniste du TLR4 et la quantité totale de tensioactifs hydrophile et hydrophobe non ioniques est entre $0,01x10^{-2}$ et $5x10^{-2}$, de préférence entre $0.05x10^{-2}$ et $2x10^{-2}$.

**11.** Une émulsion selon l'une des revendications 1 à 10, dans laquelle le polyoxyethylène alkyl éther est choisi dans le groupe constitué par du polyoxyéthylène (12) cétostearyl éther (ceteareth-12), du polyoxyéthylène (20) cétostéaryl éther (ceteareth-20), du poyoxyéthylène (21) stéaryl éther (steareth-21), du polyoxyéthylène (20) cétyl éther (ceteth-20), du polyoxyéthylène (10) cétyl éther (ceteth-10), du polyoxyéthylène (10) stéaryl éther (steareth-10), du polyoxyéthylène (20) stéaryl éther (steareth-20), du polyoxyéthylène (10) oleyl éther (oleth-10), du polyoxyéthylène (20) oléyl éther (oleth-20).

**12.** Une émulsion selon l'une des revendications 1 à 11, dans laquelle le tensioactif hydrophobe est un ester du sorbitane ou un ester du mannide.

**13.** Une émulsion selon l'une des revendications 1 à 12, dans laquelle les quantités de tensioactifs hydrophile et hydrophobe sont telles que l'HLB global des tensioactifs est entre 8,5 et 10.

**14.** Une émulsion selon l'une des revendications 1 à 13, dans laquelle la quantité de squalène représente entre 5% et 45% du poids total de l'émulsion.

**15.** Une émulsion selon l'une des revendications 1 à 14, dans laquelle le ratio entre la quantité de squalène et la quantité de tensioactifs est entre 2,0 et 4,0, de préférence entre 2,5 et 3,5.

**16.** Une émulsion selon l'une des revendications 1 à 15, dans laquelle l'agoniste du TLR4 est le sel disodique de l'acide dodécanoïque 1,1'-[(1R,6R,22R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, le tensioactif hydrophile non ionique est le polyoxyéthylène (12) cetostéaryl éther (ceteareth-12), le tensioactif hydrophobe non ionique est le sorbitane monooléate et le solvant aqueux un tampon phosphate ou un tampon citrate.

**17.** Une émulsion selon la revendication 16, dans laquelle :

a. la quantité de squalène représente entre 5% et 45% du poids total de l'émulsion (poids/poids),
b. le ratio entre la quantité de squalène et la quantité totale de polyoxyéthylène (12) cetostéaryl éther (ceteareth-12) et de sorbitane monooléate est entre 2,0 et 4,0,
c. les quantités de ceteareth-12 et de sorbitane monoléate sont telles que l'HLB se situe entre 8,5 et 10 et
d. le ratio entre la quantité du sel disodique de l'acide dodécanoïque 1,1'-[(1 R,6R,22R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester et la quantité totale de polyoxyéthylène (12) cetostéaryl éther (ceteareth-12) et de sorbitane monooléate est entre $0,01x10^{-2}$ et $2x10^{-2}$.

**18.** Une émulsion selon la revendication 17, comprenant en outre du mannitol dont la quantité représente entre 0,1 et 10% du poids total de l'émulsion.

**19.** Une émulsion selon l'une des revendications 1 à 18, comprenant en outre un substrat de lyophilisation.

**20.** Utilisation d'une émulsion selon l'une des revendications 1 à 19, pour la préparation d'une composition vaccinale.

**21.** Utilisation d'une émulsion selon l'une des revendications 1 à 19, pour la préparation d'une composition vaccinale destinée à une population d'individus qui présentent un déséquilibre au niveau de la réponse cellulaire T CD4+, notamment les personnes âgées.

**22.** Utilisation d'une émulsion selon l'une des revendications 1 à 19, pour la préparation d'une composition vaccinale comprenant comme antigène vaccinal au moins une hémagglutinine du virus grippal.

**23.** Utilisation d'une émulsion selon l'une des revendications 1 à 19, pour la préparation d'une composition vaccinale comprenant comme antigène vaccinal au moins un antigène d'enveloppe du cytomegalovirus (CMV).

**24.** Utilisation d'une émulsion selon la revendication 23, dans laquelle l'antigène d'enveloppe du CMV est la protéine gB du CMV ou un dérivé de celle ci qui comprend au moins un épitope neutralisant.

**25.** Utilisation d'une émulsion selon la revendication 23 ou 24, dans laquelle l'antigène d'enveloppe du CMV est la protéine gH ou un dérivé de celle-ci.

**26.** Utilisation d'une émulsion selon la revendication 24 ou 25, dans laquelle l'antigène d'enveloppe du CMV est la protéine gB délétée du domaine transmembranaire et dont le site de clivage est inopérant.

**27.** Un procédé de préparation d'une émulsion H/E selon l'une des revendications 1 à 19, comprenant une étape où une émulsion inverse Eau dans Huile (E/H) est obtenue par élévation de température et une étape où cette émulsion inverse Eau dans Huile (E/H) est transformée en une émulsion H/E par abaissement de température.

**28.** Un procédé selon la revendication 27, dans lequel l'émulsion E/H est obtenue en mélangeant dans une première étape une phase aqueuse comprenant un solvant aqueux, un polyoxyéthylène alkyl éther et un agoniste du TLR4 avec une phase huileuse comprenant du squalène et un tensioactif hydrophobe non ionique pour obtenir une émulsion H/E et en chauffant dans une seconde étape l'émulsion H/E à une température qui est au moins la température d'inversion de phase de l'émulsion.

**29.** Un procédé selon la revendication 27, dans lequel l'émulsion E/H est obtenue en chauffant séparément une phase aqueuse comprenant un solvant aqueux, un polyoxyéthylène alkyl éther et un agoniste du TLR4 et une phase huileuse comprenant du squalène et un tensioactif hydrophobe non ionique à une température qui est au moins la température d'inversion de phase de l'émulsion puis en mélangeant la phase aqueuse avec la phase huileuse tout en maintenant la température du mélange à une température qui est au moins la température d'inversion de phase.

**30.** Un procédé selon la revendication 28 ou 29, dans lequel l'agoniste du TLR4 est dans la phase huileuse au lieu d'être dans la phase aqueuse.

**31.** Un procédé selon l'une des revendications 28 à 30, dans lequel la phase aqueuse comprend également un alditol.

**32.** Un procédé selon l'une des revendications 28 à 31, dans lequel la température d'inversion de phase est comprise entre 45°C et 80°C, et de façon préférée entre 50°C et 65°C.

**33.** Un procédé de préparation d'une composition vaccinale, dans lequel on mélange au moins un antigène vaccinale avec une émulsion H/E comprenant ;

a) un agoniste du TLR4, tel que défini dans la revendication 1,
b) du squalène,
c) un solvant aqueux,
d) un tensioactif hydrophile non ionique qui est un polyoxyethylène alkyl éther, et
e) un tensioactifhydrophobe non ionique,
**caractérisé en ce que** l'émulsion H/E est préparée selon un procédé d'inversion de phase comprenant une étape où l'on obtient une émulsion sous la forme d'émulsion inverse E/H par augmentation de la température et une étape où l'on transforme l'émulsion E/H en émulsion H/E par abaissement de la température.

**34.** Un procédé selon l'une des revendications 27 à 33, comprenant en outre une étape de lyophilisation.

**35.** Une composition vaccinale sous la forme d'une émulsion H/E thermoréversible comprenant un antigène vaccinale,

un agoniste du TLR4 tel que défini selon la revendication 1, du squalène, un solvant aqueux, un tensioactif hydrophile non ionique qui est un polyoxyéthylène alkyl éther et un tensioactif hydrophobe non ionique.

## Patentansprüche

**1.** Öl-in-Wasser(O/W)-Emulsion, die Folgendes umfasst:

i. einen TLR4-Agonisten mit der Bezeichnung TLA4, dessen chemische Struktur eine chemische Verbindung der Formel I, II, III oder IV ist:

Verbindung der Formel I

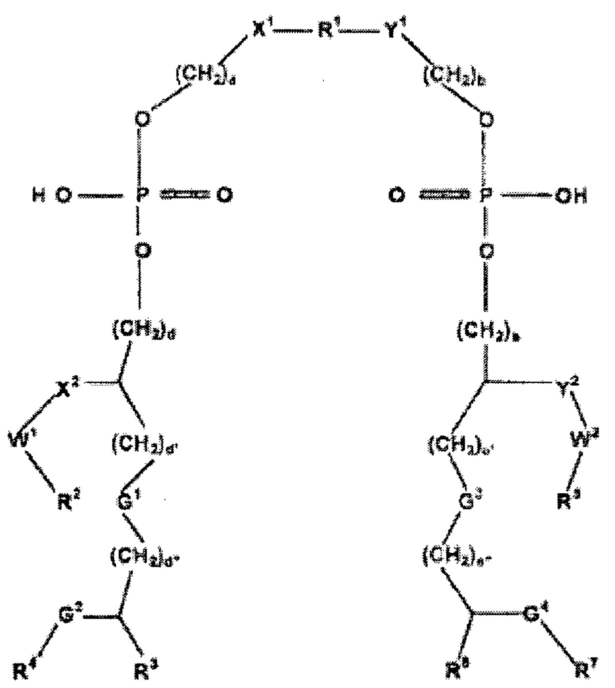

Verbindung der Formel II

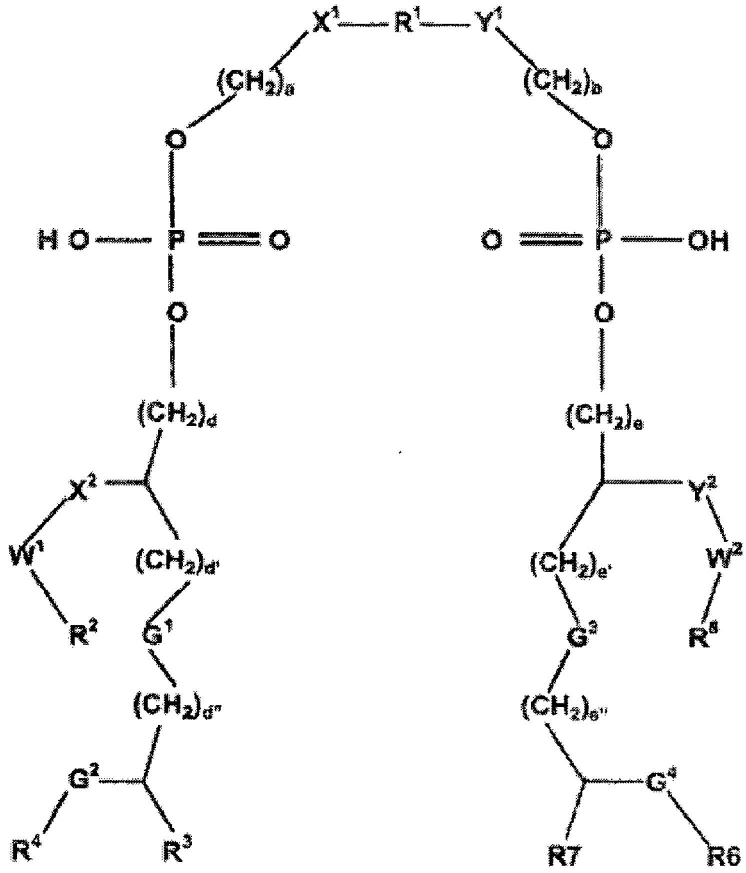

Verbindung der Formel III

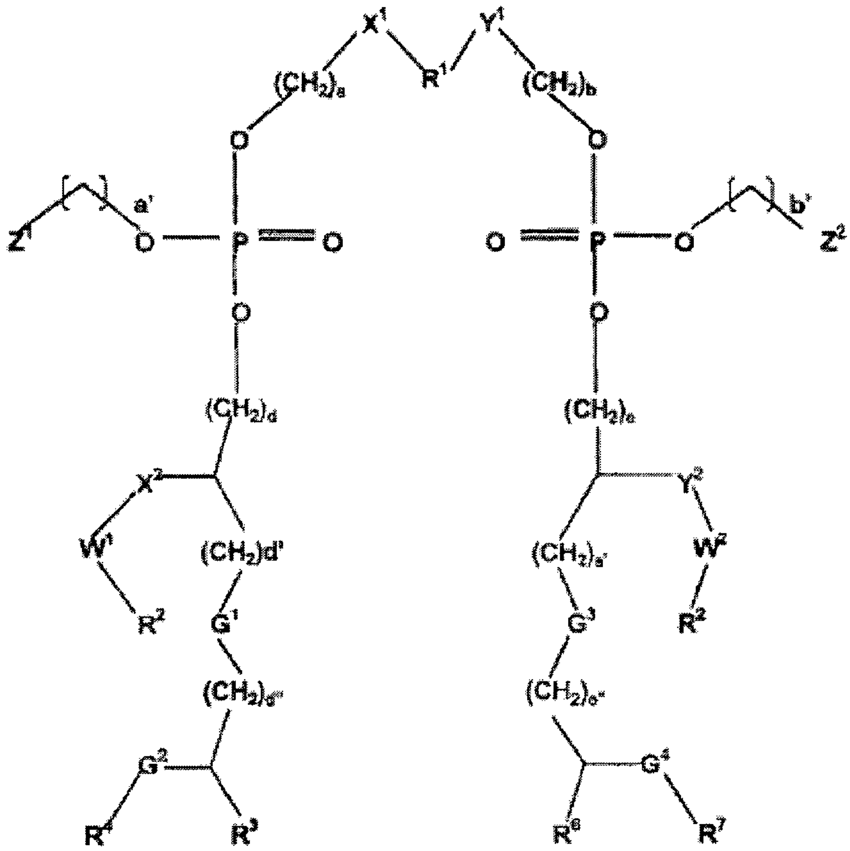

Verbindung der Formel IV

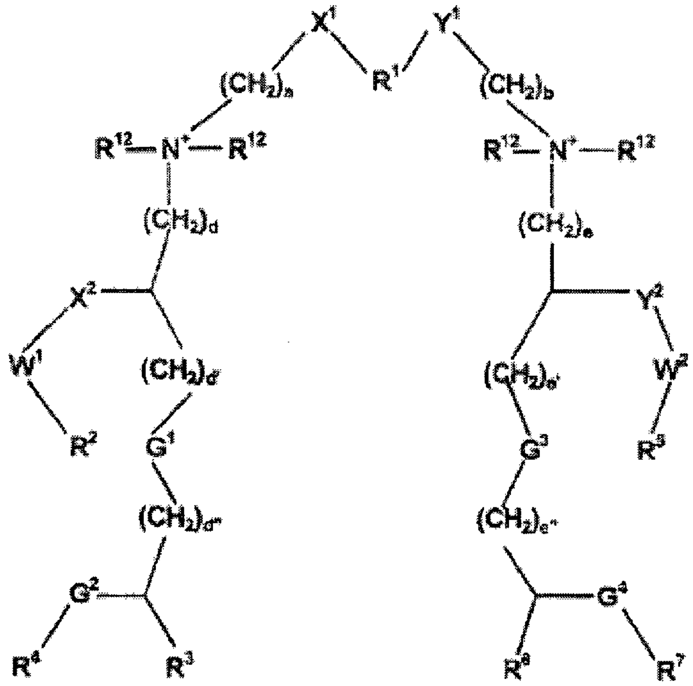

in der bei jeder der Formeln I, II, III bzw. IV $R^1$ aus der Gruppe bestehend aus folgenden ausgewählt ist:

a) C(O) ;
b) C(O)-$C_1$-$C_{14}$-Alkyl-C(O), wobei der $C_1$-$C_{14}$-Alkyl gegebenenfalls durch ein Hydroxy, $C_1$-$C_5$-Alkoxy, $C_1$-$C_5$-Alkylendioxy, ($C_1$-$C_5$-Alkyl)amino oder ($C_1$-$C_5$-Alkyl)aryl substituiert ist, wobei der Arylrest des ($C_1$-$C_5$-Alkyl)aryls gegebenenfalls durch $C_1$-$C_5$-Alkoxy, ($C_1$-$C_5$-Alkyl) amino, ($C_1$-$C_5$-Alkoxy) amino, ($C_1$-$C_5$-Alkyl)-amino-$C_1$-$C_5$-alkoxy, -O- ($C_1$-$C_5$-Alkyl)-amino-$C_1$-$C_5$-alkoxy, -O- ($C_1$-$C_5$-Alkyl)-amino-C (O)-$C_1$-$C_5$-alkyl-C(O)OH oder -O-($C_1$-$C_5$-Alkyl)-amino-C(O)-$C_1$-$C_5$-alkyl-C(O)-$C_1$-$C_5$-alkyl substituiert ist;
c) einem gerad- oder verzweigtkettigen $C_2$-$C_{15}$-Alkyl, das gegebenenfalls durch ein Hydroxy oder Alkoxy substituiert ist; und
d) -C(O)-$C_6$-$C_{12}$-Arylen-C(O)-, worin das Arylen gegebenenfalls durch ein Hydroxy, Halogen, Nitro oder Amino substituiert ist;

a und b sind unabhängig 0, 1, 2, 3 oder 4;
d, d', d'', e, e' und e'' sind unabhängig 0, 1, 2, 3 oder 4;
$X^1$, $X^2$, $Y^1$ und $Y^2$ sind unabhängig aus der Gruppe bestehend aus Null, Sauerstoff, NH, N(C(O)-$C_1$-$C_4$-Alkyl) und N($C_1$-$C_4$-Alkyl) ausgewählt;
$W^1$ und $W^2$ sind unabhängig aus der Gruppe bestehend aus einem Carbonyl, Methylen, Sulfon und Sulfoxid ausgewählt;
$R^2$ und $R^5$ sind unabhängig aus der Gruppe ausgewählt, die aus dem Folgenden besteht:

a) einem gerad- oder verzweigtkettigen $C_2$-$C_{20}$-Alkyl, das gegebenenfalls durch ein Oxo, Hydroxy oder Alkoxy substituiert ist,
b) einem gerad- oder verzweigtkettigen $C_2$-$C_{20}$-Alkenyl oder -Dialkenyl, das gegebenenfalls durch ein Oxo, Hydroxy oder Alkoxy substituiert ist;
c) einem gerad- oder verzweigtkettigen $C_2$-$C_{20}$-Alkoxy, das gegebenenfalls durch ein Oxo, Hydroxy oder Alkoxy substituiert ist;
d) gerad- oder verzweigtkettigem NH-$C_2$-$C_{20}$-Alkyl, in dem die Alkylgruppe gegebenenfalls durch ein Oxo, Hydroxy oder Alkoxy substituiert ist; und

e)

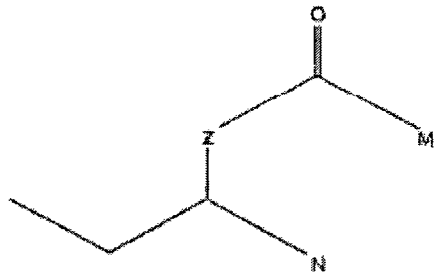

worin Z aus der Gruppe bestehend aus einem O und NH ausgewählt ist und M und N unabhängig aus der Gruppe bestehend aus einem gerad- oder verzweigtkettigen $C_2$-$C_{20}$-Alkyl, -Alkenyl, -Alkoxy, -Acyloxy, -Alkylamino und -Acylamino ausgewählt sind;
$R^3$ und $R^6$ sind unabhängig aus der Gruppe bestehend aus einem gerad- oder verzweigtkettigen $C_2$-$C_{20}$-Alkyl und -Alkenyl, das gegebenenfalls durch ein Oxo oder Fluor substituiert ist, ausgewählt;
$R^4$ und $R^7$ sind unabhängig aus der Gruppe bestehend aus einem gerad- oder verzweigtkettigen $C_2$-$C_{20}$-C(O)-Alkyl oder -Alkenyl, gerad- oder verzeigtkettigen $C_2$-$C_{20}$-Alkyl, gerad- oder verzeigtkettigen $C_2$-$C_{20}$-Alkoxy und gerad- oder verzeigtkettigen $C_2$-$C_{20}$-Alkenyl ausgewählt, wobei die Alkyl-, Alkenyl- oder Alkoxygruppen unabhängig und wahlweise durch ein Hydroxy, Fluor oder $C_1$-$C_5$-Alkoxy substituiert sein können;
$G_1$, $G_2$, $G_3$ und $G_4$ sind unabhängig aus der Gruppe bestehend aus einem Sauerstoff, Methylen, Amino, Thiol, -C(O)NH-, -NHC(O)- und -N(C(O)-$C_1$-$C_4$-Alkyl)-ausgewählt;
oder $G_2R^4$ oder $G_4R^7$ können gemeinsam ein Wasserstoffatom oder Hydroxyl sein;

und worin für die Formel III gilt:

a' und b' sind unabhängig 2, 3, 4, 5, 6, 7 oder 8, vorzugsweise 2;
$Z^1$ ist aus der Gruppe bestehend aus $-OP(O)(OH)_2$, $-P(O)(OH)_2$, -OP(O) ($OR^8$) (OH) ausgewählt, worin $R^8$ aus der Gruppe bestehend aus einer $C_1$-$C_4$-Alkylkette, $-OS(O)_2OH$, $-S(O)_2OH$, $-CO_2H$, $-OB(OH)_2$, -OH, $-CH_3$, $-NH_2$ und $-NR^9{}_3$ ausgewählt, worin $R^9$ eine $C_1$-$C_4$-Alkylkette ist;
$Z^2$ ist aus der Gruppe bestehend aus $-OP(O)(OH)_2$, $-P(O)(OH)_2$, -OP(O) ($OR^{10}$) (OH) ausgewählt, worin $R^{10}$ aus der Gruppe bestehend aus einer $C_1$-$C_4$-Alkylkette, $-OS(O)_2OH$, $-S(O)_2OH$, $-CO_2H$, -OB $(OH)_2$, -OH, $-CH_3$, $-NH_2$ und $-NR^{11}$ ausgewählt, worin $R^{11}$ eine $C_1$-$C_4$-Alkylkette ist;

und worin für die Formel IV:

R12 H oder eine $C_1$-$C_4$-Alkylkette ist;

oder ein pharmazeutisch unbedenkliches Salz der Verbindung der chemischen Formel I, II, III oder IV;

ii. Squalen,
iii. ein wässriges Lösungsmittel,
iv. ein hydrophiles nichtionisches Tensid, bei dem es sich um einen Polyoxyethylenalkylether handelt,
v. ein hydrophobes nichtionisches Tensid, die thermoreversibel ist.

**2.** Emulsion nach Anspruch 1, in der mindestens 90% der Öltröpfchenpopulation, bezogen auf das Volumen, eine Größe von ≤ 200 nm aufweisen.

**3.** Emulsion nach Anspruch 1 oder 2, in der mindestens 50% der Öltröpfchenpopulation, bezogen auf das Volumen, eine Größe von ≤ 100 nm aufweisen.

**4.** Emulsion nach einem der Ansprüche 1 bis 3, in der die Phaseninversion bei einer Temperatur zwischen 45°C und 80°C, vorzugsweise zwischen 50°C und 65°C, erfolgt.

**5.** Emulsion nach einem der Ansprüche 1 bis 4, die weiterhin mindestens ein Alditol umfasst.

**6.** Emulsion nach einem der Ansprüche 1 bis 5, in der es sich bei der wässrigen Phase um eine gepufferte Kochsalzlösung handelt.

**7.** Emulsion nach einem der Ansprüche 1 bis 6, in der es sich bei dem TLR4-Agonisten um eine chemische Verbindung der Formel I:

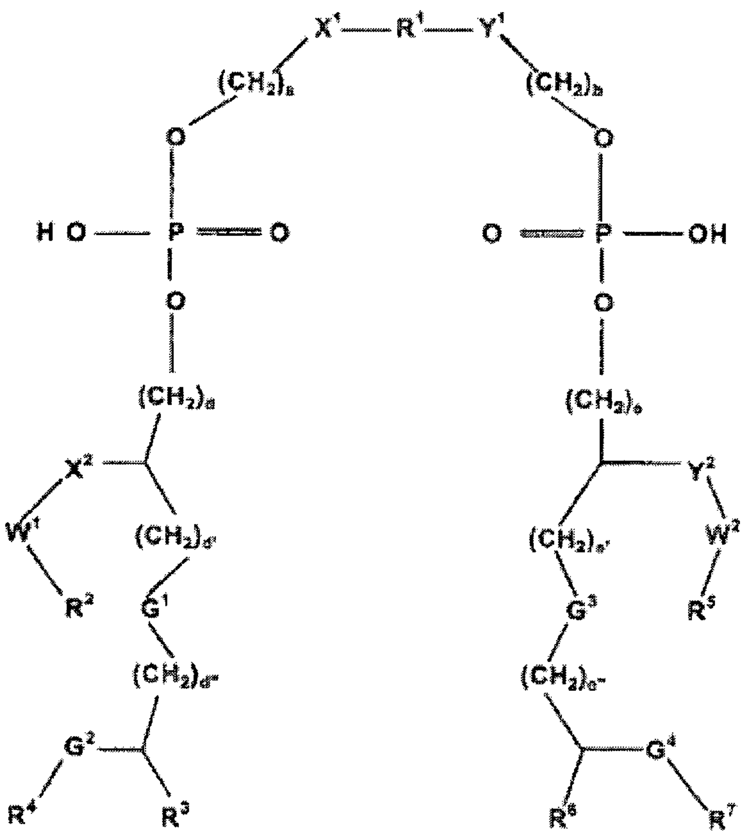

oder ein pharmazeutisch unbedenkliches Salz dieser Verbindung handelt.

**8.** Emulsion nach Anspruch 7, in der:

R1 C(O) oder C(O)-$(CH2)_n$-C(O) bedeutet, wobei n 1, 2, 3 oder 4 bedeutet,
a, b, d, d', d'', e, e', e'' unabhängig 1 oder 2 bedeuten,
X1, X2, Y1 und Y2 NH bedeuten,
W1 und W2 C(O) bedeuten,
R2 und R5 unabhängig aus der Gruppe bestehend aus einem gegebenenfalls durch ein Oxo substituiertes geradkettiges C10-C15-Alkyl, einem geradkettigen NH-C10-C15-Alkyl und

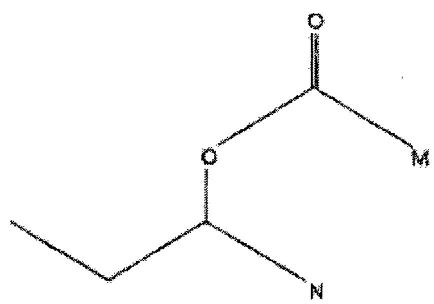

ausgewählt sind,

worin M und N unabhängig ein geradkettiges C2-C20-Alkyl oder -Alkenyl bedeuten,
R3 und R6 geradkettige C5-C10 Alkylketten bedeuten,
R4 und R7 aus der Gruppe bestehend aus einem Wasserstoff, geradkettigem C(O)-C8-C12-Alkyl und geradkettigem C(O)-C8-C12-Alkenyl ausgewählt sind,
G1 und G3 einen Sauerstoff oder -NH(CO)- bedeuten,
G2 und G4 einen Sauerstoff bedeuten.

**9.** Emulsion nach Anspruch 7 oder 8, in der die chemische Verbindung aus der Gruppe der chemischen Verbindungen ausgewählt ist, die aus dem Folgenden besteht: Dinatriumsalz des Dodecansäure-1,1'-[(1R,6R,22R,27R)-1,27-diheptyl-9,19-dihydroxy-9,19-dioxido-14-oxo-6,22-bis[(1-oxotetradecyl)-amino]-4,8,10,18,20,24-hexaoxa-13,15-diaza-9,19-diphosphaheptacosan-1,27-diyl]esters, Dodecansäure-1,1'-[(1S,6R,24R,29S)-1,29-diheptyl-9,21-dihydroxy-9,21-dioxido-14,16-dioxo-6,24-bis[(1-oxotetradecyl)amino]-4,8,10,20,22,26-hexaoxa-13,17-diaza-9,21-diphosphanonacosan-1,29-diyl]ester, Dinatriumsalz des Dodecansäure-1,1'-[(1R,6R,22S,27R)-1,27-diheptyl-9,19-dihydroxy-9,19-dioxido-14-oxo-6,22-bis[(1-oxotetradecyl)-amino]-4,8,10,18,20,24-hexaoxa-13,15-diaza-9,19-diphosphaheptacosan-1,27-diyl]esters, Dodecansäure-1,1'-[(1R,6R,22S,27R)-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosan-1,27-diyl]esters, Dinatriumsalz des Dodecansäure-1,1'-[(1R,6R,22S,27R)-6,22-bis[(1,3-dioxotetradecyl)-amino]-1,27-diheptyl-9,19-dihydroxy-9,19-dioxido-14-oxo-4,8,10,18,20,24-hexaoxa-13,15-diaza-9,19-diphosphaheptacosan-1,27-diyl]esters, Dinatriumsalz des Dodecansäure-1,1'-[(1R,6R,22R,27R)-6,22-bis[(1,3-dioxotetradecyl)-amino]-1,27-diheptyl-9,19-dihydroxy-9,19-dioxido-14-oxo-4,8,10,18,20,24-hexaoxa-13,15-diaza-9,19-diphosphaheptacosan-1,27-diyl]esters, Dinatriumsalz des Dodec-5-ensäure-1,1'-[(1R,6R,24R,29R)-6,24-bis[(1,3-dioxotetradecyl)-amino]-1,29-diheptyl-9,21-dihydroxy-9,21-dioxido-14,16-dioxo-4,8,10,20,22,26-hexaoxa-13,17-diaza-9,21-diphosphanonacosan-1,29-diyl]esters, Dinatriumsalz des Dodec-5-ensäure-1,1'-[(1R,6S,24S,29R)-6,24-bis[(1,3-dioxotetradecyl)-amino]-1,29-diheptyl-9,21-dihydroxy-9,21-dioxido-14,16-dioxo-4,8,10,20,22,26-hexaoxa-13,17-diaza-9,21-diphosphanonacosan-1,29-diyl]esters, Dinatriumsalz des Dodecansäure-1,1'-[(1R,6R,22R,27R)-6,22-bis[[(dodecyl-amino)carbonyl]amino]-1,27-diheptyl-9,19-dihydroxy-9,19-dioxido-14-oxo-4,8,10,18,20,24-hexaoxa-13,15-diaza-9,19-diphosphaheptacosan-1,27-diyl]esters, Dodecansäure-1,1'-[(1R,6R,24R,29R)-1,29-diheptyl-10,20-dihydroxy-10,20-dioxido-15-oxo-6,24-bis[(1-oxotetradecyl)amino]-4,9,11,19,21,26-hexaoxa-14,16-diaza-10,20-diphosphanonacosan-1,29-diyl]ester, Dinatriumsalz des Dodec-5-ensäure-1,1'-[(1R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosan-1,27-diyl]esters, Dinatriumsalz des Dodec-5-ensäure-1,1'-[(1R,32R)-6,27-bis[(1,3-dioxotetradecyl)-amino]-1,32-diheptyl-9,24-dihydroxy-9,24-dioxido-14,19-dioxo-4,8,10,23,25,29-hexaoxa-13,20-diaza-9,24-diphosphadotriacontan-1,32-diyl]esters, Dinatriumsalz des Dodec-5-ensäure-1,1'-[(1R,28R)-1,28-diheptyl-8,21-dihydroxy-5,24-bis[[[(3R)-3-hydroxydecyl]oxy]methyl]-8,21-dioxido-3,13,16,26-tetraoxo-7,9,20,22-tetraoxa-4,12,17,25-tetraaza-8,21-diphosphaoctacosan-1,28-diyl]esters, Dinatriumsalz des Dodecansäure-1,1'-[(1R,30R)-6,25-bis[(1,3-dioxotetradecyl)amino]-1,30-diheptyl-9,22-dihydroxy-9,22-dioxido-14,17-dioxo-4,8,10,21,23,27-hexaoxa-13,18-diaza-9,22-diphosphatriacontan-1,30-diyl]esters, Dinatriumsalz des Dodecansäure-1,1'-[(1R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosan-1,27-diyl]esters, Dinatriumsalz des Dodec-5-ensäure-1,1'-[(1R,29R)-6,24-bis[(1,3-dioxotetradecyl)amino]-1,29-diheptyl-9,21-dihydroxy-9,21-dioxido-14,16-dioxo-4,8,10,20,22,26-hexaoxa-13,17-diaza-9,21-diphosphanonacosan-1,29-diyl]esters, Dinatriumsalz des Dodec-5-ensäure-1,1'-[(1R,29R)-1,29-diheptyl-9,21-dihydroxy-9,21-dioxido-14,16-dioxo-6,24-bis[(1-oxotetradecyl)amino]-4,8,10,20,22,26-hexaoxa-13,17-diaza-9,21-diphosphanonacosan-1,29-diyl]esters, Dinatriumsalz des Dodecansäure-1,1'-[(1R,27R)-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosan-1,27-diyl]esters und Dinatriumsalz des Dodec-5-ensäure-1,1'-[(1R,27R)-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosan-1,27-diyl] esters.

**10.** Emulsion nach einem der Ansprüche 1 bis 9, wobei das Verhältnis zwischen Menge an TLR4-Agonist zu der Gesamtmenge an hydrophilen und hydrophoben nichtionischen Tensiden zwischen $0{,}01x10^{-2}$ und $5x10^{-2}$, vorzugsweise zwischen $0{,}05x10^{-2}$ und $2x10^{-2}$, beträgt.

**11.** Emulsion nach einem der Ansprüche 1 bis 10, wobei der Polyoxyethylenalkylether aus der Gruppe bestehend aus Polyoxyethylen(12)cetostearylether (Ceteareth-12), Polyoxyethylen(20)cetostearylether (Ceteareth-20), Polyoxyethylen(21)stearylether (Steareth-21), Polyoxyethylen(20)cetylether (Ceteth-20), Polyoxyethylen(10)cetylether (Ceteth-10), Polyoxyethylen(10)stearylether (Steareth-10), Polyoxyethylen(20)stearylether (Steareth-20), Polyoxyethylen(10)oleylether (Oleth-10), Polyoxyethylen(20)oleylether (Oleth-20) ausgewählt ist.

**12.** Emulsion nach einem der Ansprüche 1 bis 11, wobei es sich bei dem hydrophoben Tensid um einen Sorbitanester oder einen Mannidester handelt.

**13.** Emulsion nach einem der Ansprüche 1 bis 12, wobei die Mengen an hydrophoben und hydrophilen Tensiden derart sind, dass der Gesamt-HLB-Wert der Tenside zwischen 8,5 und 10 betragen.

**14.** Emulsion nach einem der Ansprüche 1 bis 13, wobei die Menge an Squalen zwischen 5% und 45% des Gesamtgewichts der Emulsion ausmacht.

**15.** Emulsion nach einem der Ansprüche 1 bis 14, wobei das Verhältnis zwischen der Menge an Squalen und der Menge an Tensiden zwischen 2,0 und 4,0, vorzugsweise zwischen 2,5 und 3,5, beträgt.

**16.** Emulsion nach einem der Ansprüche 1 bis 15, wobei es sich bei dem TLR4-Agonisten um das Dinatriumsalz des Dodecansäure-1,1'-[(1R,6R,22R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosan-1,27-diyl]esters, bei dem hydrophilen nichtionischen Tensid um Polyoxyethylen(12)cetostearylether (Ceteareth-12), bei dem hydrophoben nichtionischen Tensid um Sorbitanmonooleat und bei dem wässrigen Lösungsmittel um einen Phosphatpuffer oder einen Citratpuffer handelt.

**17.** Emulsion nach Anspruch 16, wobei:

a. die Squalenmenge zwischen 5% und 45% des Gesamtgewichts der Emulsion (w/w) ausmacht,
b. das Verhältnis zwischen der Squalenmenge und der Gesamtmenge an Polyoxyethylen(12)-cetostearylether (Ceteareth-12) und an Sorbitanmonooleat zwischen 2,0 und 4,0 beträgt,
c. die Mengen an Ceteareth-12 und Sorbitanmonooleat derart sind, dass der HLB-Wert zwischen 8,5 und 10 liegt, und
d. das Verhältnis zwischen der Menge an Dinatriumsalz des Dodecansäure-1,1'-[(1R,6R,22R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosan-1,27-diyl]esters und der Gesamtmenge an Polyoxyethylen(12)cetostearylether (Ceteareth-12) und Sorbitanmonooleat zwischen $0{,}01 \times 10^{-2}$ und $2 \times 10^{-2}$ beträgt.

**18.** Emulsion nach Anspruch 17, die weiterhin Mannit umfasst, dessen Menge zwischen 0,1 und 10% des Gesamtgewichts der Emulsion ausmacht.

**19.** Emulsion nach einem der Ansprüche 1 bis 18, die weiterhin ein Lyophilisationssubstrat umfasst.

**20.** Verwendung einer Emulsion nach einem der Ansprüche 1 bis 19 zur Herstellung einer Impfstoffzusammensetzung.

**21.** Verwendung einer Emulsion nach einem der Ansprüche 1 bis 19 zur Herstellung einer Impfstoffzusammensetzung, die für eine Population von Individuen bestimmt ist, bei denen ein Ungleichgewicht bei der T-CD4+-Zellreaktion herrscht, insbesondere ältere Menschen.

**22.** Verwendung einer Emulsion nach einem der Ansprüche 1 bis 19 zur Herstellung einer Impfstoffzusammensetzung, die als Impfstoffantigen mindestens ein Hämagglutinin des Grippevirus umfasst.

**23.** Verwendung einer Emulsion nach einem der Ansprüche 1 bis 19 zur Herstellung einer Impfstoffzusammensetzung, die als Impfstoffantigen mindestens ein Antigen der Hülle des Cytomegalievirus (CMV) umfasst.

**24.** Verwendung einer Emulsion nach Anspruch 23, wobei es sich bei dem Antigen der Hülle des CMV um das Protein gB des CMV oder um ein Derivat davon, das mindestens ein neutralisierendes Epitop umfasst, handelt.

**25.** Verwendung einer Emulsion nach Anspruch 23 oder 24, in der es sich bei dem Antigen der Hülle des CMV um das Protein gH oder ein Derivat davon handelt.

**26.** Verwendung einer Emulsion nach Anspruch 24 oder 25, in der es sich bei dem Antigen der Hülle des CMV um das Protein gB, dessen Transmembrandomäne deletiert worden ist und dessen Spaltstelle nicht funktionell ist, handelt.

**27.** Verfahren zur Herstellung einer O/W-Emulsion nach einem der Ansprüche 1 bis 19, umfassend einen Schritt, in

dem eine inverse Wasser-in-Öl (W/O)-Emulsion dadurch erhalten wird, dass man die Temperatur erhöht, sowie einen Schritt, in dem diese inverse Wasser-in-Öl (W/O)-Emulsion durch Erniedrigen der Temperatur in eine O/W-Emulsion umgewandelt wird.

**28.** Verfahren nach Anspruch 27, bei dem die W/O-Emulsion dadurch erhalten wird, dass man in einem ersten Schritt eine wässrige Phase, die ein wässriges Lösungsmittel, einen Polyoxyethylen-alkylether und einen TLR4-Agonisten umfasst, mit einer öligen Phase, die Squalen und ein hydrophobes nichtionisches Tensid umfasst, vermischt, wodurch man zu einer O/W-Emulsion gelangt, und in einem zweiten Schritt die O/W-Emulsion auf eine Temperatur erhitzt, bei der es sich zumindest um die Temperatur der Phaseninversion der Emulsion handelt.

**29.** Verfahren nach Anspruch 27, bei dem die W/O-Emulsion dadurch erhalten wird, dass man eine wässrige Phase, die ein wässriges Lösungsmittel, einen Polyoxyethylenalkylether und einen TLR4-Agonisten umfasst, und eine ölige Phase, die Squalen und ein hydrophobes nichtionisches Tensid umfasst, getrennt auf eine Temperatur erhitzt, bei der es sich zumindest um die Temperatur der Phaseninversion der Emulsion handelt, und anschließend die wässrige Phase mit der öligen Phase vermischt, wobei die Mischtemperatur bei einer Temperatur gehalten wird, bei der es sich zumindest um die Temperatur der Phaseninversion handelt.

**30.** Verfahren nach Anspruch 28 oder 29, bei dem der TLR4-Agonist in der öligen Phase statt in der wässrigen Phase vorliegt.

**31.** Verfahren nach einem der Ansprüche 28 bis 30, bei dem die wässrige Phase auch ein Alditol umfasst.

**32.** Verfahren nach einem der Ansprüche 28 bis 31, bei dem die Temperatur der Phaseninversion zwischen 45°C und 80°C, vorzugsweise zwischen 50°C und 65°C, liegt.

**33.** Verfahren zur Herstellung einer Impfstoffzusammensetzung, bei dem man mindestens ein Impfstoffantigen mit einer O/W-Emulsion, die Folgendes umfasst:

a) einen TLR4-Agonisten wie in Anspruch 1 definiert,
b) Squalen,
c) ein wässriges Lösungsmittel,
d) ein hydrophiles nichtionisches Tensid, bei dem es sich um einen Polyoxyethylenalkylether handelt und
e) ein hydrophobes nichtionisches Tensid, vermischt, **dadurch gekennzeichnet, dass** die O/W-Emulsion nach einem Phaseninversionsverfahren hergestellt wird, das einen Schritt umfasst, in dem man durch Erhöhen der Temperatur eine Emulsion in Form einer inversen W/O-Emulsion erhält und einen Schritt, in dem man die W/O-Emulsion durch Erniedrigen der Temperatur in eine O/W-Emulsion umwandelt.

**34.** Verfahren nach einem der Ansprüche 27 bis 33, die weiterhin einen Lyophilisationsschritt umfasst.

**35.** Impfstoffzusammensetzung in Form einer thermoreversiblen O/W-Emulsion, umfassend ein Impfstoffantigen, einen TLR4-Agonisten wie in Anspruch 1 definiert, Squalen, ein wässriges Lösungsmittel, ein hydrophiles nichtionisches Tensid, bei dem es sich um einen Polyoxyethylenalkylether handelt, und ein hydrophobes nichtionisches Tensid.

## Claims

**1.** Oil-in-Water (O/W) emulsion comprising:

i. a TLR4 agonist, called TLA4, the chemical structure of which is a chemical compound of formula I, II, III or IV:

Compound of formula I

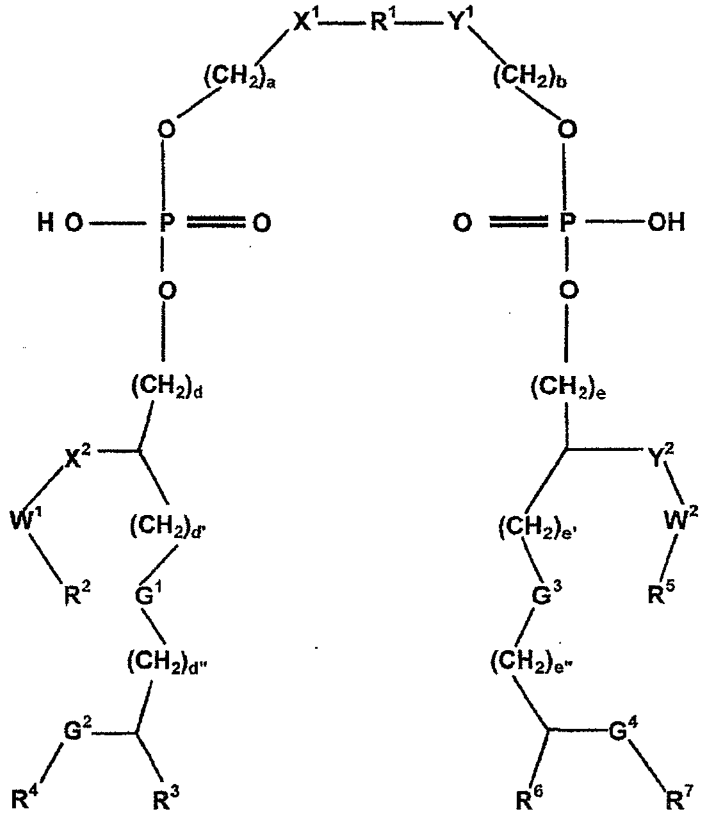

Compound of formula II

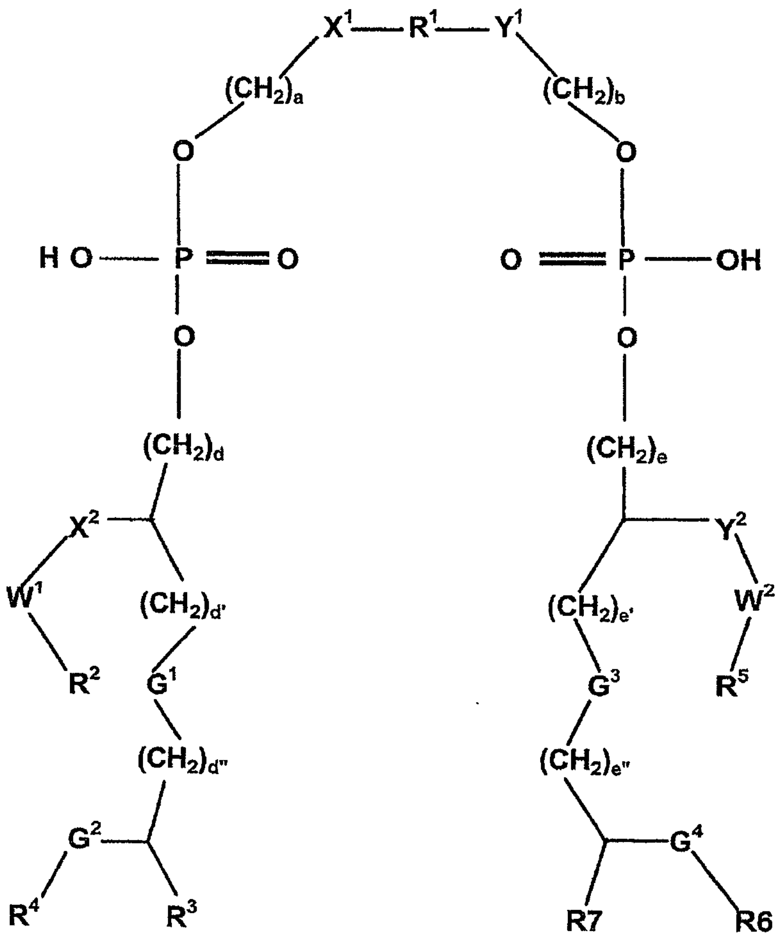

Compound of formula III

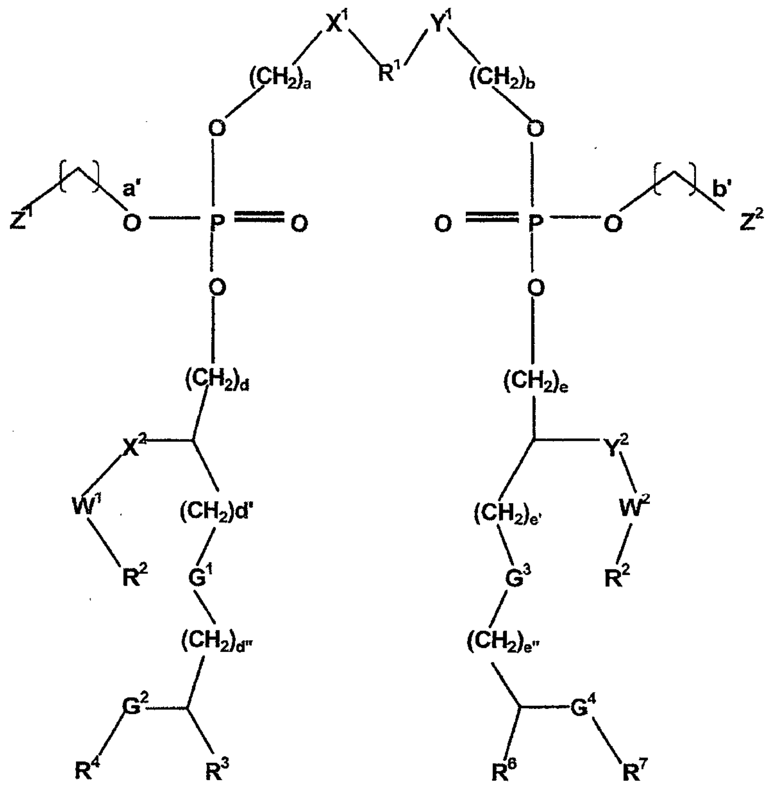

Compound of formula IV

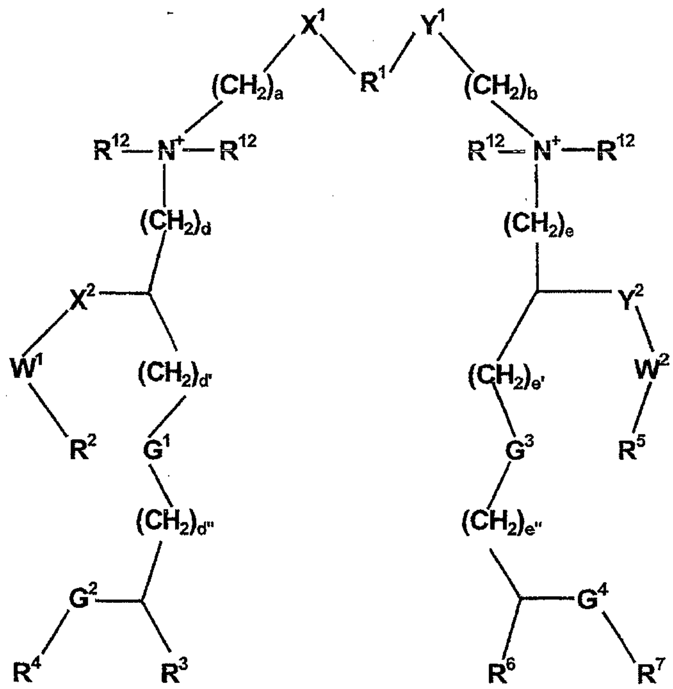

in which, for each of formula I, II, III or IV, $R^1$ is selected from the group consisting of:

a) C(O);
b) C(O)-($C_1$-$C_{14}$ alkyl)-C(O), in which said $C_1$-$C_{14}$ alkyl is optionally substituted with a hydroxyl, a $C_1$-$C_5$ alkoxy, a $C_1$-$C_5$ alkylenedioxy, a ($C_1$-$C_5$ alkyl)amino or a ($C_1$-$C_5$ alkyl)aryl, in which said aryl moiety of said ($C_1$-$C_5$ alkyl)aryl is optionally substituted with a $C_1$-$C_5$ alkoxy, a ($C_1$-$C_5$ alkyl) amino, a ($C_1$-$C_5$ alkoxy) amino, a ($C_1$-$C_5$ alkyl)amino($C_1$-$C_5$ alkoxy), -O-($C_1$-$C_5$ alkyl) amino ($C_1$-$C_5$ alkoxy), -O-($C_1$-$C_5$ alkyl) amino-C(O)-($C_1$-$C_5$ alkyl) - C(O)OH, or -O-($C_1$-$C_5$ alkyl)amino-C(O)-($C_1$-$C_5$ alkyl)-C(O)-($C_1$-$C_5$) alkyl;
c) an alkyl comprising a $C_2$-$C_{15}$ linear or branched chain, optionally substituted with a hydroxyl or an alkoxy; and
d) -C(O)-($C_6$-$C_{12}$ arylene) -C(O)- in which said arylene is optionally substituted with a hydroxyl, a halogen, a nitro or an amino;

a and b are independently 0, 1, 2, 3 or 4;
d, d', d'', e, e' and e'' are independently 0, 1, 2, 3 or 4;
$X^1$, $X^2$, $Y^1$ and $Y^2$ are independently selected from the group consisting of null, an oxygen, NH, N (C(O) ($C_1$-$C_4$ alkyl)), and N($C_1$-$C_4$ alkyl) ;
$W^1$ and $W^2$ are independently selected from the group consisting of a carbonyl, a methylene, a sulfone and a sulfoxide;
$R^2$ and $R^5$ are independently selected from the group consisting of:

a) a $C_2$ to $C_{20}$ straight chain or branched chain alkyl, which is optionally substituted with an oxo, a hydroxyl or an alkoxy;
b) a $C_2$ to $C_{20}$ straight chain or branched chain alkenyl or dialkenyl, which is optionally substituted with an oxo, a hydroxyl or an alkoxy;
c) a $C_2$ to $C_{20}$ straight chain or branched chain alkoxy, which is optionally substituted with an oxo, a hydroxyl or an alkoxy;
d) NH-($C_2$ to $C_{20}$ straight chain or branched chain alkyl), in which said alkyl group is optionally substituted with an oxo, a hydroxyl or an alkoxy; and
e)

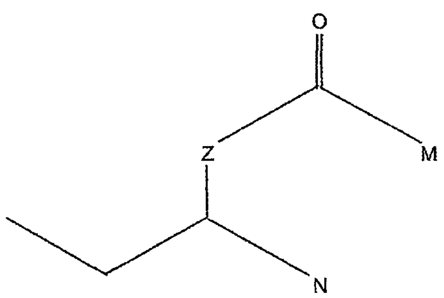

in which Z is selected from the group consisting of O and NH, and M and N are independently selected from the group consisting of a $C_2$-$C_{20}$ linear or branched chain alkyl, alkenyl, alkoxy, acyloxy, alkylamino and acylamino;
$R^3$ and $R^6$ are independently selected from the group consisting of a $C_2$ to $C_{20}$ straight chain or branched chain alkyl and alkenyl, optionally substituted with an oxo or a fluoro;
$R^4$ and $R^7$ are independently selected from the group consisting of C(O)-($C_2$ to $C_{20}$ straight chain or branched chain alkyl or alkenyl), a $C_2$ to $C_{20}$ straight chain or branched chain alkyl, a $C_2$ to $C_{20}$ straight chain or branched chain alkoxy, and a $C_2$ to $C_{20}$ straight chain or branched chain alkenyl; in which said alkyl, alkenyl or alkoxy groups can be independently and optionally substituted with a hydroxyl, a fluoro or a $C_1$-$C_5$ alkoxy;
$G_1$, $G_2$, $G_3$ and $G_4$ are independently selected from the group consisting of an oxygen, a methylene, an amino, a thiol, -C(O)NH-, -NHC(O)-, and -N(C(O) ($C_1$-$C_4$ alkyl))-;
or $G^2R^4$ or $G^4R^7$ can together be a hydrogen atom or a hydroxyl;

and in which, for formula III:

a' and b' are independently 2, 3, 4, 5, 6, 7 or 8, preferably 2;
$Z^1$ is selected from the group consisting of $-OP(O)(OH)_2$, $-P(O)(OH)_2$, $-OP(O)(OR^8)$ (OH) where $R^8$ is selected from the group consisting of a $C_1$-$C_4$ alkyl chain, $-OS(O)_2OH$, $-S(O)_2OH$, $-CO_2H$, -OB $(OH)_2$,-OH, $-CH_3$, $-NH_2$ and $-NR^9{}_3$ where $R^9$ is a $C_1$-$C_4$ alkyl chain;
$Z^2$ is selected from the group consisting of $-OP(O)(OH)_2$, $-P(O)(OH)_2$, -OP(O) $(OR^{10})$ (OH) where $R^{10}$ is selected from the group consisting of a $C_1$-$C_4$ alkyl chain, $-OS(O)_2OH$, $-S(O)_2OH$, $-CO_2H$, $-OB(OH)_2$, -OH, $-CH_3$, $-NH_2$ and $-NR^{11}$ where $R^{11}$ is a $C_1$-$C_4$ alkyl chain;

and in which, for formula IV:

R12 is H or a $C_1$-$C_4$ alkyl chain;

or a pharmaceutically acceptable salt of the compound of chemical formula I, II, III or IV;

ii. squalene,
iii. an aqueous solvent,
iv. a nonionic hydrophilic surfactant which is a polyoxyethylene alkyl ether,
v. a nonionic hydrophobic surfactant, and

which is thermoreversible.

2. Emulsion according to Claim 1, in which at least 90% of the population by volume of the oil droplets has a size ≤ 200 nm.

3. Emulsion according to Claim 1 or 2, in which at least 50% of the population by volume of the oil droplets has a size ≤ 110 nm.

4. Emulsion according to one of Claims 1 to 3, in which the phase inversion occurs at a temperature of between 45°C and 80°C, preferably of between 50°C and 65°C.

5. Emulsion according to one of Claims 1 to 4, further comprising at least one alditol.

6. Emulsion according to one of Claims 1 to 5, in which the aqueous phase is a buffered saline solution.

7. Emulsion according to one of Claims 1 to 6, in which the TLR4 agonist is a chemical compound of formula I:

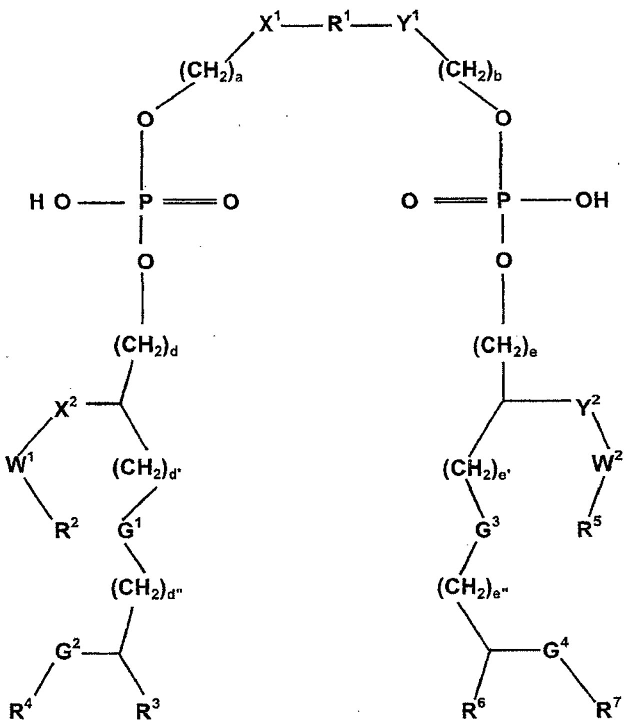

or a pharmaceutically acceptable salt of this compound.

**8.** Emulsion according to Claim 7, in which:

R1 is C(O) or C(O)-(CH2)$_n$-C(O), n being 1, 2, 3 or 4,
a, b, d, d', d", e, e' and e" are independently 1 or 2,
X1, X2, Y1 and Y2 are NH,
W1 and W2 are C(O),
R2 and R5 are independently selected from the group consisting of a $C_{10}$-$C_{15}$ straight chain alkyl optionally substituted with an oxo, an NH-($C_{10}$-$C_{15}$ straight chain alkyl), and

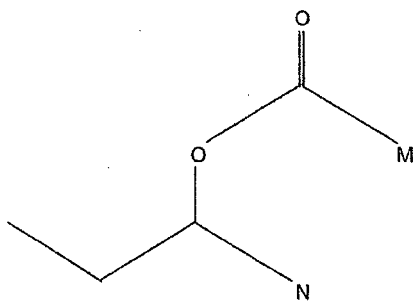

in which M and N are independently a $C_2$-$C_{20}$ straight chain alkyl or alkenyl,
R3 and R6 are $C_5$-$C_{10}$ straight chain alkyls,
R4 and R7 are selected from the group consisting of a hydrogen, C(O)-($C_8$-$C_{12}$ straight chain alkyl) and C(O)-($C_8$-$C_{12}$ straight chain alkenyl),
G1 and G3 are an oxygen or -NH(CO)-,
G2 and G4 are an oxygen.

**9.** Emulsion according to Claim 7 or 8, in which the chemical compound is chosen from the group of chemical compounds consisting of disodium salt of dodecanoic acid 1,1'-[(1R,6R,22R,27R)-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphoshaheptacosane-1,27-diyl] ester, dodecanoic acid 1,1'-[(1S, 6R,24R,29S)-1,29-diheptyl-9,21-dihdyroxy-9,21-dioxido-14,16-dioxo-6,24-bis[(1-oxotetra-decyl)amino]-4,8,10,20,22,26-hexaoxa-13,17-diaza-9,21-diphosphanonacosane-1,29-diyl] ester, the disodium salt of dodecanoic acid 1,1'-[(1R,6R,22S,27R)-1,27-diheptyl-9,19-dihydroxy-9,19-dioxido-14-oxo-6,22-bis[(1-oxotetra-decyl)amino]-4,8,10,18,20,24-hexaoxa-13,15-diaza-9,19-diphosphaheptacosane-1,27-diyl] ester, dodecanoic acid 1,1'-[(1R,6R,22S,27R)-1,27-diheptyl-9,19-dihydroxy-9,19-dioxido-14-oxo-6,22-bis[(1-oxotetradecyl)amino]-4,8,10,18,20,24-hexa-oxa-13,15-diaza-9,19-diphosphaheptacosane-1,27-diyl] ester, the disodium salt of dodecanoic acid 1,1'-[(1R,6S,22S,27R)-6,22-bis[(1,3-dioxotetra-decyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, the disodium salt of dodecanoic acid 1,1'-[(1R,6R,22R,27R)-6,22-bis[(1,3-dioxotetra-decyl)amino]-1,27-diheptyl-9,19-dihdyroxy-9,19-dioxido-14-oxo-4,8,10,18,20,24-hexaoxa-13,15-diaza-9,19-diphosphaheptacosane-1,27-diyl] ester, the disodium salt of 5-dodecenoic acid 1,1'-[(1R,6R,24R,29R)-6,24-bis[(1,3-dioxatetra-decyl)amino]-1,29-diheptyl-9,21-dihydroxy-9,21-dioxido-14,16-dioxo-4,8,10,20,22,26-hexaoxa-13,17-diaza-9,21-diphosphanonacosane-1,29-diyl] ester, the disodium salt of 5-dodecenoic acid 1,1'-[(1R,6S,24S,29R)-6,24-bis[(1,3-dioxotetra-decyl)amino]-1,29-diheptyl-9,21-dihdyroxy-9,21-dioxido-14,16-dioxo-4,8,10,20,22,26-hexaoxa-13,17-diaza-9,21-diphosphanonacosane-1,29-diyl] ester, the disodium salt of dodecanoic acid 1,1'-[1R,6R,22R,27R)-6,22-bis[[(dodecyl-amino)carbonyl]amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, dodecanoic acid 1,1'-[(1R,6R,24R,29R)-1,29-diheptyl-10,20-dihydroxy-10,20-dioxido-15-oxo-6,24-bis[(1-oxo-tetradecyl)amino]-4,9,11,19,21,26-hexaoxa-14,16-diaza-10,20-diphosphanonacosane-1,29-diyl] ester, the disodium salt of 5-dodecenoic acid 1,1'-[(1R,27R)-6,22-bis[(1,3-dioxotetra-decyl) amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, the disodium salt of 5-dodecenoic acid 1,1-[(1R,32R)-6,27-bis[(1,3-dioxotetradecyl)amino]-1,32-diheptyl-9,24-dihydroxy-9,24-dioxido-14,19-dioxo-4,8,10,23,25,29-hexaoxa-13,20-diaza-9,24-diphosphadotriacontane-1,32-diyl] ester, the disodium salt of 5-dodecenoic acid 1,1'-[(1R,28R)-1,28-diheptyl-8,21-dihydroxy-5,24-bis[[[(3R)-3-hydroxydecyl]oxy]methyl]-8,21-dioxido-3,13,16,26-tetraoxo-7,9,20,22-tetraoxa-4,12,17,25-tetraaza-8,21-diphosphaoctacosane-1,28-diyl] ester, the disodium salt of dodecanoic acid 1,1'-[(1R,30R)-6,25-bis[(1,3-dioxotetradecyl)amino]-1,30-diheptyl-9,22-dihydroxy-9,22-dioxido-14,17-dioxo-4,8,10,21,23,27-hexaoxa-13,18-diaza-9,22-diphosphatriacontane-1,30-diyl) ester, the disodium salt of dodecanoic acid 1,1'-[(1R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, the disodium salt of 5-dodecenoic acid 1,1'-[(1R,29R)-6,24-bis[(1,3-dioxotetradecyl)amino]-1,29-diheptyl-9,21-dihydroxy-9,21-dioxido-14,16-dioxo-4,8,10,20,22,26-hexaoxa-13,17-diaza-9,21-diphosphanonacosane-1,29-diyl] ester, the disodium salt of 5-dodecenoic acid 1,1'-[(1R,29R)-1,29-diheptyl-9,21-dihydroxy-9,21-dioxido-14,16-dioxo-6,24-bis[(1-oxotetradecyl) amino]-4,8,10,20,22,26-hexaoxa-13,17-diaza-9,21-diphosphanonacosane-1,29-diyl] ester, the disodium salt of dodecanoic acid 1,1'-[(1R,27R)-1,27-dipheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester, and the disodium salt of 5-dodecenoic acid 1,1'-[(1R,27R-1,27-diheptyl-9,19-dihydroxy-9,19-dioxido-14-oxo-6,22-bis[(1-oxotetradecyl)amino]-4,8,10,18,20,24-hexaoxa-13,15-diaza-9,19-diphosphaheptacosane-1,27-diyl] ester.

**10.** Emulsion according to one of Claims 1 to 9, wherein the ratio of the amount of TLR4 agonist to the total amount of nonionic hydrophilic and hydrophobic surfactants is between $0.01\times10^{-2}$ and $5\times10^{-2}$, preferably between $0.05\times10^{-2}$ and $2\times10^{-2}$.

**11.** Emulsion according to one of Claims 1 to 10, wherein the polyoxyethylene alkyl ether is chosen from the group consisting of polyoxyethylene (12) cetostearyl ether (ceteareth-12), polyoxyethylene (20) cetostearyl ether (ceteareth-20), polyoxyethylene (21) stearyl ether (steareth-21), polyoxyethylene (20) cetyl ether (ceteth-20), polyoxyethylene (10) cetyl ether (ceteth-10), polyoxyethylene (10) stearyl ether (steareth-10), polyoxyethylene (20) stearyl ether (steareth-20), polyoxyethylene (10) oleyl ether (oleth-10) and polyoxyethylene (20) oleyl ether (oleth-20).

**12.** Emulsion according to one of Claims 1 to 11, wherein the hydrophobic surfactant is a sorbitan ester or a mannide ester.

**13.** Emulsion according to one of Claims 1 to 12, wherein the amounts of hydrophilic and hydrophobic surfactants are such that the overall HLB of the surfactants is between 8.5 and 10.

**14.** Emulsion according to one of Claims 1 to 13, wherein the amount of squalene represents between 5% and 45% of the total weight of the emulsion.

**15.** Emulsion according to one of Claims 1 to 14, wherein the ratio of the amount of squalene to the amount of surfactants is between 2.0 and 4.0, preferably between 2.5 and 3.5.

**16.** Emulsion according to one of Claims 1 to 15, wherein the TLR4 agonist is the disodium salt of dodecanoic acid 1,1'-[(1R,6R,22R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester, the nonionic hydrophilic surfactant is polyoxyethylene (12) cetostearyl ether (ceteareth-12), the nonionic hydrophobic surfactant is sorbitan monooleate and the aqueous solvent is a phosphate buffer or a citrate buffer.

**17.** Emulsion according to Claim 16, wherein:

a. the amount of squalene represents between 5% and 45% of the total weight of the emulsion (weight/weight),
b. the ratio of the amount of squalene to the total amount of polyoxyethylene (12) cetostearyl ether (ceteareth-12) and of sorbitan monooleate is between 2.0 and 4.0,
c. the amounts of ceteareth-12 and of sorbitan monooleate are such that the HLB is between 8.5 and 10, and
d. the ratio of the amount of the disodium salt of dodecanoic acid 1,1'-[(1R,6R,22R,27R)-6,22-bis[(1,3-dioxotetradecyl)amino]-1,27-diheptyl-9,19-dihydroxy-9,19-dioxido-14-oxo-4,8,10,18,20,24-hexaoxa-13,15-diaza-9,19-diphosphaheptacosane-1,27-diyl] ester to the total amount of polyoxyethylene (12) cetostearyl ether (ceteareth-12) and of sorbitan monooleate is between $0.01\times10^{-2}$ and $2\times10^{-2}$.

**18.** Emulsion according to Claim 17, further comprising mannitol, the amount of which represents between 0.1 and 10% of the total weight of the emulsion.

**19.** Emulsion according to one of Claims 1 to 18, further comprising a lyophilization substrate.

**20.** Use of an emulsion according to one of Claims 1 to 19, for preparing a vaccine composition.

**21.** Use of an emulsion according to one of Claims 1 to 19, for preparing a vaccine composition intended for a population of individuals who present an imbalance in the CD4+ T cell response, in particular elderly individuals.

**22.** Use of an emulsion according to one of Claims 1 to 19, for preparing a vaccine composition comprising, as vaccine antigen, at least one flu virus hemagglutinin.

**23.** Use of an emulsion according to one of Claims 1 to 19, for preparing a vaccine composition comprising, as vaccine antigen, at least one cytomegalovirus (CMV) envelope antigen.

**24.** Use of an emulsion according to Claim 23, wherein the CMV envelope antigen is the gB protein of CMV or a derivative thereof which comprises at least one neutralizing epitope.

**25.** Use of an emulsion according to Claim 23 or 24, wherein the CMV envelope antigen is the gH protein or a derivative thereof.

**26.** Use of an emulsion according to Claim 24 or 25, wherein the CMV envelope antigen is the gB protein without the transmembrane domain and in which the cleavage site is ineffectual.

**27.** Method for preparing an O/W emulsion according to one of Claims 1 to 19, comprising a step in which an inverse water-in-oil (W/O) emulsion is obtained by raising the temperature and a step in which this inverse water-in-oil (W/O) emulsion is converted into an O/W emulsion by lowering the temperature.

**28.** Method according to Claim 27, in which the W/O emulsion is obtained by carrying out a first step in which an aqueous phase comprising an aqueous solvent, a polyoxyethylene alkyl ether and a TLR4 agonist is mixed with an oily phase comprising squalene and a nonionic hydrophobic surfactant so as to obtain an O/W emulsion, and a second step in which the O/W emulsion is heated to a temperature which is at least the phase inversion temperature of the emulsion.

**29.** Method according to Claim 27, in which the W/O emulsion is obtained by separately heating an aqueous phase comprising an aqueous solvent, a polyoxyethylene alkyl ether and a TLR4 agonist and an oily phase comprising squalene and a nonionic hydrophobic surfactant to a temperature which is at least the phase inversion temperature of the emulsion, and then by mixing the aqueous phase with the oily phase while maintaining the temperature of the mixture at a temperature which is at least the phase inversion temperature.

**30.** Method according to Claim 28 or 29, in which the TLR4 agonist is in the oily phase instead of being in the aqueous phase.

**31.** Method according to one of Claims 28 to 30, in which the aqueous phase also comprises an alditol.

**32.** Method according to one of Claims 28 to 31, in which the phase inversion temperature is between 45°C and 80°C, and preferably between 50°C and 65°C.

**33.** Method for preparing a vaccine composition, in which at least one vaccine antigen is mixed with an O/W emulsion comprising:

a) a TLR4 agonist, as defined in claim 1,
b) squalene,
c) an aqueous solvent,
d) a nonionic hydrophilic surfactant which is a polyoxyethylene alkyl ether, and
e) a nonionic hydrophobic surfactant, **characterized in that** the O/W emulsion is prepared according to a phase inversion method comprising a step in which an emulsion is obtained in the form of an inverse W/O emulsion by increasing the temperature and a step in which the W/O emulsion is converted into an O/W emulsion by lowering the temperature.

**34.** Method according to one of Claims 27 to 33, further comprising a lyophilization step.

**35.** Vaccine composition in the form of a thermoreversible O/W emulsion comprising a vaccine antigen, a TLR4 agonist as defined according to Claim 1, squalene, an aqueous solvent, a nonionic hydrophilic surfactant which is a polyoxyethylene alkyl ether, and a nonionic hydrophobic surfactant.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 20004060396 A
- US 20030153532 A
- US 20050164988 A
- WO 0238176 A
- US 20020102562 A
- US 5547834 A
- US 6100064 A
- US 6162620 A
- US 5866383 A
- US 5552143 A
- US 6183750 B
- US 5338683 A
- WO 9215672 A
- WO 9639491 A
- US 5474914 A
- US 6610295 B
- US 5314800 A
- EP 0759995 A
- US 5834307 A

### Littérature non-brevet citée dans la description

- *Journal of Biological Chemistry,* 2001, vol. 276 (3), 1873-1880
- **GARDNER et al.** *Science,* 1998, vol. 282, 1126-1132
- *Journal of Général Virology,* 1999, vol. 80, 2183-2191
- *Journal of Virology,* 2005, vol. 79, 4066-4079
- **URBAN M et al.** *J. Virol,* 1992, vol. 66 (3), 1303-1311
- *J. Virol,* 1992, vol. 66 (3), 1303-1311
- **OUYANG et al.** *Mechanisms of ageing and development,* 2000, vol. 121, 131-137
- **RASMUSSEN L et al.** *J. Virol.,* 1985, vol. 55, 274-280
- **GONCZOL E. et al.** *J. Virological Methods,* 1986, vol. 14, 37-41
- **POTTER et al.** *Vaccine,* 2003, vol. 21, 940-945